(19) Europäisches Patentamt
European Patent Office
Office européen des brevets

(11) EP 1 908 479 A2

(12) EUROPEAN PATENT APPLICATION

(43) Date of publication:
**09.04.2008 Bulletin 2008/15**

(51) Int Cl.:
***A61K 39/395*** *(2006.01)* ***A61P 35/00*** *(2006.01)*

(21) Application number: **07110330.3**

(22) Date of filing: **15.03.2000**

(84) Designated Contracting States:
**AT BE CH CY DE DK ES FI FR GB GR IE IT LI LU MC NL PT SE**

(30) Priority: **19.03.1999 AU PP932199**

(62) Document number(s) of the earlier application(s) in accordance with Art. 76 EPC:
**00908832.9 / 1 171 469**

(71) Applicant: **ST VINCENT'S HOSPITAL SYDNEY LIMITED**
**Darlinghurst, NSW 2010 (AU)**

(72) Inventors:
- **Ward, Robyn Lynne**
**Woollahra, New South Wales 2025 (AU)**
- **Coomber, David William John**
**Camperdown, New South Wales 2050 (AU)**

(74) Representative: **Smart, Peter John**
**Beck Greener**
**Fulwood House,**
**12 Fulwood Place,**
**London WC1V 6HR (GB)**

Remarks:
•This application was filed on 15 - 06 - 2007 as a divisional application to the application mentioned under INID code 62.
•Claims 11-29,31-51,53,54,56-89,91-98,100-131 are deemed to be abandoned due to non-payment of the claims fees (Rule 45(3) EPC).

(54) **Anti-p53 Antibodies**

(57) The present invention relates to nucleotide sequences which encode polypeptides of antibodies against the p53 protein in vertebrates, and to the polypeptides and antibodies (or fragments thereof) encoded by those nucleotide sequences. The invention also relates to nucleotide sequences and polypeptide sequences for use in the development of diagnostic and therapeutic compositions, and to methods of using those diagnostic and therapeutic compositions in the diagnosis and treatment of cancer, rheumatoid arthritis and other disease states which exhibit abnormalities of p53.

EP 1 908 479 A2

## Description

## TECHNICAL FIELD

The present invention relates to nucleotide sequences which encode polypeptides of antibodies against the p53 protein in vertebrates, and to the polypeptides and antibodies (or fragments thereof) encoded by those nucleotide sequences. The invention also relates to nucleotide sequences and polypeptide sequences for use in the development of diagnostic and therapeutic compositions, and to methods of using those diagnostic and therapeutic compositions in the diagnosis and treatment of cancer, rheumatoid arthritis and other disease states which exhibit abnormalities of p53.

## BACKGROUND OF THE INVENTION

The p53 gene is mutated in more than 50% of human tumours (1). Point mutations in the central DNA binding domain are the most frequently observed mutation (2), and result in loss of function due to conformational changes (3). The half life of the mutated protein is usually increased resulting in accumulation of p53 in tumour cells. This accumulation of mutant protein is implicated as a factor in the development of an immune response to the protein in some cancer patients (4). The conformation of wild-type p53 is not static, with changes between the wild-type and mutant phenotype or conformation being induced, for instance, *in vitro* by buffer conditions, monoclonal antibodies, kinases and enzymes, or *in vivo* by kinases, phosphatases, and other p53 regulatory proteins.

Anti-P53 serum antibodies have been detected in up to 30% of individuals with cancer, and a range of different tumours. Monoclonal antibodies (MAb) to p53 have been invaluable in investigating the function of p53 and its role in tumorigenesis.

Molecular approaches to the generation of Mab offer several advantages over traditional methods such as EBV transformation or hybridoma technology. In part, this is because in humans, these traditional methods often result in a bias towards certain B cell populations and the creation of cell lines which are unstable or producing only low levels of antibody (5). In contrast, molecular genetic approaches allow the use of genetic material from any source of available B lymphocytes to create random combinations of cloned heavy and light chain immunoglobulin genes.

Previous studies of the immune response against p53 in cancer patients have relied on serum analysis. These studies have yielded important information on the clinical significance, epitope dominance and the role of protein overexpression in the development of the anti-p53 immune response. However, several critical questions remain unanswered. To date no human anti-p53 Mabs have been isolated either by conventional cell immortalisation methods or molecular biological procedures. Hence no information is available on human anti-p53 antibody V gene usage, the degree of somatic mutation and structural features of the anti-p53 antibodies. Such information is critical to any meaningful understanding of the nature and significance of the humoral immune response to p53.

The present invention describes the isolation of anti-p53 antibodies. The nucleotide sequence and gene usage of these antibodies were examined. These antibodies are a rich resource for use in functional studies of the protein, diagnostic assessment of p53 in normal and disease states, as well as in the development of vaccines, including idiotypic vaccines.

## SUMMARY OF THE INVENTION

### 1. Nucleic Acid Encoding a Polypeptide of an Antibody or fragment thereof to p53.

According to a first embodiment of the invention, there is provided an isolated and purified nucleic acid sequence comprising a polynucleotide sequence encoding a polypeptide of an antibody (or fragment thereof), wherein said antibody (or fragment thereof) has binding affinity to a p53 protein or a portion thereof in vertebrates, and wherein said nucleic acid sequence is obtained from a vertebrate host expressing an immune response against a naturally-occurring disease.

Typically, the immune response is characterised by expression of at least one p53 antibody.

Typically, the nucleic acid molecule comprises a polynucleotide sequence encoding an $F_{ab}$ antibody fragment (or fragment thereof) having binding affinity to a p53 protein or a portion thereof in vertebrates.

According to a second embodiment of the invention, there is provided an isolated and purified nucleic acid molecule comprising a polynucleotide sequence selected from the group consisting of SEQ ID Nos 1-30.

The following features relate to the first and second embodiments of the invention.

Typically, the nucleic acid molecule corresponds to a DNA or RNA molecule.

Generally, the nucleic acid molecule comprises a polynucleotide sequence(s), or an analogue thereof, encoding an antibody fragment or other immunologically active fragments thereof, such as complementarity determining regions, wherein the antibody (or fragment thereof) has binding affinity to a p53 protein or a portion thereof in vertebrates.

Typically, the antibody fragment has functional antigen-binding domains. Even more typically, the antibody fragment may exist in a form selected from the group consisting of: Fv, $F_{ab}$, $F(ab)_2$, scFv (single chain Fv), dAb (single domain antibody), bi-specific antibodies, diabodies and triabodies.

Typically, the antibody (or fragment thereof) has binding affinity to a p53 protein or a portion thereof. More typically, the antibody (or fragment thereof) has binding affinity for residues of one or more of the N-terminus, the C-terminus or the central domain of a p53 protein or a portion thereof. Even more typically, the antibody (or fragment thereof) has binding affinity for residues of the N-terminus of a p53 protein or a portion thereof. Yet more typically, the antibody (or fragment thereof) has binding affinity for residues about 10 to about 55. Still more typically, the antibody (or fragment thereof) has binding affinity for residues about 10 to about 25, or about 40 to about 50, or about 27 to about 44 of the N-terminus of a p53 protein or portion thereof or about 40 to about 44 of the N-terminus of a p53 protein or portion thereof. Even more typically, the antibody (or fragment thereof) has binding affinity for residues about 27 to about 44 of the N-terminus of a p53 protein or a portion thereof. Still more typically, the antibody (or fragment thereof) has binding affinity for residues about 40 to about 44 of the N-terminus of a p53 protein or a portion thereof.

Even more typically, the antibody (or fragment thereof) has binding affinity to residues of the central domain of a p53 protein or a portion thereof.

Typically, the nucleic acid molecule comprises a polynucleotide sequence encoding a polypeptide of an antibody (or fragment thereof) having binding affinity to a p53 protein or a portion thereof in vertebrates, wherein said polynucleotide sequence encodes an immunoglobulin light chain variable region polypeptide or an immunoglobulin heavy chain variable region polypeptide.

More typically, the nucleic acid molecule comprises a polynucleotide sequence encoding a polypeptide of an antibody (or fragment thereof) having binding affinity to a p53 protein or a portion thereof in vertebrates, wherein said nucleic acid molecule comprises a first polynucleotide sequence encoding an immunoglobulin light chain variable region polypeptide, and a second polynucleotide sequence encoding an immunoglobulin heavy chain variable region polypeptide.

Typically, the polynucleotide sequence encoding the immunoglobulin light chain variable region comprises polynucleotide sequence(s) encoding immunoglobulin light chain variable (V region) and joining (J region) segments.

Typically, the polynucleotide sequence encoding the immunoglobulin heavy chain variable region polypeptide comprises polynucleotide sequence(s) encoding immunoglobulin heavy chain variable (V region), diversity (D region) and joining (J region) segments.

More typically, the nucleic acid molecule also comprises a polynucleotide sequence(s) encoding one or more immunoglobulin constant regions operably linked with the immunoglobulin heavy chain variable or immunoglobulin light chain region(s). Even more typically, at least one of the immunoglobulin constant regions may be derived from a different source than the source from which the immunoglobulin variable region was derived. Still more typically, the source from which the immunoglobulin constant region is derived is human.

Typically, the p53 protein or a portion thereof is encoded by a wild type or mutant p53 gene.

Typically, the vertebrate is selected from the group consisting of human, non-human primate, murine, bovine, ovine, equine, caprine, leporine, avian, feline and canine. More typically, the vertebrate is human, non-human primate or murine. Even more typically, the vertebrate is human.

Typically, the nucleic acid molecule also includes within its scope an analogue of the polynucleotide sequence defined in accordance with the first or second embodiments of the invention, wherein said analogue encodes a polypeptide having a biological activity which is functionally the same as the polypeptide(s) encoded by the polynucleotide sequence defined in accordance with the first or second embodiments of the invention, wherein said polynucleotide sequence can be located and isolated using standard techniques in molecular biology, without undue trial and experimentation.

Typically, the nucleic acid molecule also includes within its scope an analogue of the polynucleotide sequence defined in accordance with the first or second embodiments of the invention, which has at least 45% homology to the polynucleotide sequences so defined. More typically, the analogue of the polynucleotide sequences has at least 55% homology, still more typically the analogue has at least 60% homology, even more typically, the analogue has at least 75% homology, still more typically, the analogue has at least 85% homology, and yet still more typically, the analogue has at least 90% homology, and yet even still more typically, the analogue has at least 95-99% homology to the polynucleotide sequences so defined.

The degree of homology between two nucleic acid sequences may be determined by means of computer programs known in the art such as GAP provided in the GCG program package (Program Manual for the Wisconsin Package, Version 8, August 1996, Genetics Computer Group, 575 Science Drive, Madison, Wisconsin, USA 53711) (Needleman, S.B. and Wunsch, C.D., (1970), Journal of Molecular Biology, 48, 443-453). Using GAP with the following settings for DNA sequence comparison: GAP creation penalty of 5.0 and GAP extension penalty of 0.3.

Antibody sequences may be aligned to each other using the Pileup alignment software, available as part of the GCG program package, using, for instance, the default settings of gap creation penalty of 5 and gap width penalty of 0.3.

Typically, the nucleic acid molecule also includes within its scope an analogue of the polynucleotide sequence defined in accordance with the first or second embodiments of the invention, wherein said analogue is capable of hybridising to the polynucleotide sequences under conditions of low stringency. More typically, low stringency hybridisation conditions correspond to hybridisation performed at 50˚C in 6xSSC.

Suitable experimental conditions for determining whether a given nucleic acid molecule hybridises to a specified nucleic acid may involve presoaking of a filter containing a relevant sample of the nucleic acid to be examined in 5 x SSC for 10 min, and prehybridisation of the filter in a solution of 5 x SSC, 5 x Denhardt's solution, 0.5% SDS and 100 μg/ml of denatured sonicated salmon sperm DNA, followed by hybridisation in the same solution containing a concentration of 10 ng/ml of a $^{32}$P-dCTP-labeled probe for 12 hours at approximately 45˚C, in accordance with the hybridisation methods as described in Sambrook et al. (1989; Molecular Cloning, A Laboratory Manual, 2nd edition, Cold Spring Harbour, New York).

The filter is then washed twice for 30 minutes in 2 x SSC, 0.5% SDS at least 55˚C (low stringency), at least 60˚C (medium stringency), at least 65˚C (medium/high stringency), at least 70˚C (high stringency), or at least 75˚C (very high stringency). Hybridisation may be detected by exposure of the filter to an x-ray film.

Further, there are numerous conditions and factors, well known to those skilled in the art, which may be employed to alter the stringency of hybridisation. For instance, the length and nature (DNA, RNA, base composition) of the nucleic acid to be hybridised to a specified nucleic acid; concentration of salts and other components, such as the presence or absence of formamide, dextran sulfate, polyethylene glycol etc; and altering the temperature of the hybridisation and/or washing steps.

Further, it is also possible to theoretically predict whether or not two given nucleic acid sequences will hybridise under certain specified conditions. Accordingly, as an alternative to the empirical method described above, the determination as to whether an analogous nucleic acid sequence will hybridise to the nucleic acid molecule in accordance with the first or second embodiments of the invention, can be based on a theoretical calculation of the $T_m$ (melting temperature) at which two heterologous nucleic acid sequences with known sequences will hybridise under specified conditions, such as salt concentration and temperature.

In determining the melting temperature for heterologous nucleic acid sequences ($T_{m(hetero)}$) it is necessary first to determine the melting temperature ($T_{m(homo)}$) for homologous nucleic acid sequence. The melting temperature ($T_{m(homo)}$) between two fully complementary nucleic acid strands (homoduplex formation) may be determined in accordance with the following formula, as outlined in Current Protocols in Molecular Biology, John Wiley and Sons, 1995, as:

$$T_{m(homo)} = 81.5^{\circ}C + 16.6(\log M) + 0.41(\%GC) - 0.61\ (\%\ form) - 500/L$$

M = denotes the molarity of monovalent cations,
%GC = % guanine (G) and cytosine (C) of total number of bases in the sequence,
% form = % formamide in the hybridisation buffer, and
L = the length of the nucleic acid sequence.

$T_m$ determined by the above formula is the $T_m$ of a homoduplex formation ($T_{m(homo)}$) between two fully complementary nucleic acid sequences. In order to adapt the $T_m$ value to that of two heterologous nucleic acid sequences, it is assumed that a 1% difference in nucleotide sequence between two heterologous sequences equals a 1˚C decrease in $T_m$. Therefore, the $T_m(_{hetero})$ for the heteroduplex formation is obtained through subtracting the homology % difference between the analogous sequence in question and the nucleotide probe described above from the $T_{m(homo)}$.

Typically, the nucleic acid molecule also includes within its scope an analogue of the polynucleotide sequence defined in accordance with the first or second embodiments of the invention, which because of the degeneracy of the genetic code, does not hybridise with the polynucleotide sequence defined in accordance with the second embodiment of the invention, but which encodes a polypeptide of an antibody (or fragment thereof) having binding affinity to a p53 protein or a portion thereof in vertebrates.

Typically the nucleic acid molecule as defined in accordance with the first or second embodiments of the invention also includes within its scope a nucleic acid molecule which is an oligonucleotide fragment of the polynucleotide sequence defined in accordance with the first or second embodiments of the invention.

Typically, the oligonucleotide fragment is between about 10 to about 100 nucleotides in length. More typically, the oligonucleotide fragment is between about 10 to about 75 nucleotides in length. Even more typically, the oligonucleotide fragment is between about 15 to about 50 nucleotides in length. Even more typically still, the oligonucleotide fragment is between about 15 to about 30 nucleotides in length. Yet still more typically, the oligonucleotide fragment is between about 5 to about 25 nucleotides in length.

**2. Polypeptide of an Antibody or fragment thereof to p53 and/or Antibody or fragment thereof to p53.**

According to a third embodiment of the invention, there is provided a polypeptide of an antibody (or fragment thereof) having binding affinity to a p53 protein or a portion thereof in vertebrates, wherein said polypeptide is obtained from a vertebrate host expressing an immune response against a naturally-occurring disease.

Typically, the immune response is characterised by expression of at least one p53 antibody.

According to a fourth embodiment of the invention, there is provided a polypeptide, wherein said polypeptide is encoded by the nucleic acid molecule defined in accordance with the first or second embodiments of the invention.

According to a fifth embodiment of the invention, there is provided a polypeptide comprising an amino acid sequence selected from the group consisting of SEQ ID Nos 31-60.

The following features relate to the third, fourth and fifth embodiments of the invention.

Typically, the polypeptide comprises functional antigen-binding domains, that is, heavy and light chain variable domains. Even more typically, the polypeptide of the antibody (or fragment thereof) may exist in a form selected from the group consisting of: Fv, $F_{ab}$, $F(ab)_2$, scFv (single chain Fv), dAb (single domain antibody), bi-specific antibodies, diabodies and triabodies.

Typically, the polypeptide has binding affinity to a p53 protein or a portion thereof. More typically, the antibody has binding affinity for residues of one or more of the N-terminus, the C-terminus or the central domain of a p53 protein or a portion thereof. Even more typically, the antibody has binding affinity for residues of the N-terminus of a p53 protein or a portion thereof. Yet more typically, the antibody has binding affinity for residues about 10 to about 55. Still more typically, the antibody has binding affinity for residues about 10 to about 25, or about 40 to about 50, or about 27 to about 44 of the N-terminus of a p53 protein or portion thereof or about 40 to about 44 of the N-terminus of a p53 protein or portion thereof. Even more typically, the antibody has binding affinity for residues about 27 to about 44 of the N-terminus of a p53 protein or a portion thereof. Still more typically, the antibody has binding affinity for residues about 40 to about 44 of the N-terminus of a p53 protein or a portion thereof.

Even more typically, the antibody has binding affinity to residues of the central domain of a p53 protein or a portion thereof.

Typically, the polypeptide corresponds to an immunoglobulin light chain variable region polypeptide or an immunoglobulin heavy chain variable region polypeptide.

More typically, the polypeptide comprises a first polypeptide which corresponds to an immunoglobulin light chain variable region polypeptide, and a second polypeptide which corresponds to an immunoglobulin heavy chain variable region polypeptide.

Typically, the immunoglobulin light chain variable region polypeptide comprises immunoglobulin light chain variable (V region) and joining (J region) segments.

Typically, the immunoglobulin heavy chain variable region polypeptide comprises immunoglobulin heavy chain variable (V region), diversity (D region) and joining (J region) segments.

More typically, the polypeptide also comprises polypeptide(s) which correspond to an immunoglobulin constant region(s) operably linked with the immunoglobulin light or heavy chain variable region(s). Even more typically, at least one of the constant regions may be derived from a different source than the source from which the variable region was derived. Still more typically, the source from which the constant region is derived is human.

Typically, the polypeptide includes within its scope a peptide fragment of the polypeptide of the third, fourth or fifth embodiment, wherein said peptide fragment may or may not have binding affinity to a p53 protein or a portion thereof.

Typically, the peptide fragment of the polypeptide is between about 5 to about 50 contiguous amino acids. More typically, between about 5 to about 35 contiguous amino acids. Even more typically, between about 5 to about 30 contiguous amino acids. Still more typically, between about 5 to about 25 contiguous amino acids. Yet still more typically, between about 8 to about 20 contiguous amino acids.

Typically, the polypeptide also includes within its scope a homologous polypeptide of the polypeptide defined in accordance with the third, fourth and fifth embodiments of the invention, which has at least 35% homology to the polypeptide sequences so defined. More typically, the homologue of the polypeptide sequences has at least 45% homology, still more typically the homologue has at least 65% homology, even more typically, the homologue has at least 75% homology, still more typically, the homologue has at least 85% homology, and yet still more typically, the homologue has at least 90% homology, and yet even still more typically, the homologue has at least 95-99% homology to the polypeptide sequences so defined.

As applied to polypeptides, the degree of homology between two polypeptide sequences when optimally aligned, may be determined through the use of computer alignment programs known in the art such as, for example: BLAZE (Intelligenetics) GAP, BESTFIT, ALIGN, using default gap weights. One specific example is the GAP program as provided in the GCG program package (Program Manual for the Wisconsin Package, Version 8, August 1996, Genetics Computer Group, 575 Science Drive, Madison, Wisconsin, USA 53711) (Needleman, S.B. and Wunsch, C.D., (1970), Journal of Molecular Biology, 48, 443-453), using the following settings for sequence comparison: GAP creation penalty of 5.0 and GAP extension penalty of 0.3.

According to a sixth embodiment of the invention, there is provided an antibody (or fragment thereof), wherein said antibody (or fragment thereof) has binding affinity to a p53 protein or a portion thereof in vertebrates, and wherein said antibody is obtained from a vertebrate host expressing an immune response against a naturally-occurring disease.

Typically, the immune response is characterised by expression of at least one p53 antibody.

Typically, the antibody (or fragment thereof) having binding affinity to a p53 protein or a portion thereof in vertebrates, corresponds to the an antibody (or fragment thereof) encoded by the nucleic acid molecule as defined in accordance with the first or second embodiments of the invention.

According to a seventh embodiment of the invention, there is provided an antibody (or fragment thereof) having binding affinity to a p53 protein or a portion thereof in vertebrates, wherein said antibody (or fragment thereof) is comprised of the polypeptide as defined in accordance with the third, fourth or fifth embodiment of the invention.

Typically, the antibody (or fragment thereof) having binding affinity to a p53 protein or a portion thereof in vertebrates, corresponds to the polypeptide as defined in accordance with the third, fourth or fifth embodiment of the invention.

Typically, as described throughout the specification, the antibody may be a whole antibody, or an antibody fragment, or other immunologically active fragments thereof, such as complementarity determining regions. More typically, the antibody fragment has functional antigen-binding domains, that is, heavy and light chain variable domains. Even more typically, the antibody fragment may exist in a form selected from the group consisting of: Fv, $F_{ab}$, $F(ab)_2$, scFv (single chain Fv), dAb (single domain antibody), bi-specific antibodies, diabodies and triabodies.

The following features relate to the sixth and seventh embodiments of the invention.

Typically, the antibody (or fragment thereof) is a polyclonal or monoclonal antibody. More typically, the antibody (or fragment thereof) is a monoclonal antibody. Even more typically, the monoclonal antibody is generated using molecular genetic, hybridoma or EBV (Epstein-Barr virus) transformation technology. Even more typically, the monoclonal antibody may be generated using recombinant antibody techniques, through screening a combinatorial antibody library or phage display technology.

Typically, the antibody (or fragment thereof) has binding affinity to a p53 protein or a portion thereof. More typically, the antibody (or fragment thereof) has binding affinity for residues of one or more of the N-terminus, the C-terminus or the central domain of a p53 protein or a portion thereof. Even more typically, the antibody (or fragment thereof) has binding affinity for residues of the N-terminus of a p53 protein or a portion thereof. Yet more typically, the antibody (or fragment thereof) has binding affinity for residues about 10 to about 55. Still more typically, the antibody (or fragment thereof) has binding affinity for residues about 10 to about 25, or about 40 to about 50, or about 27 to about 44 of the N-terminus of a p53 protein or portion thereof or about 40 to about 44 of the N-terminus of a p53 protein or portion thereof. Even more typically, the antibody (or fragment thereof) has binding affinity for residues about 27 to about 44 of the N-terminus of a p53 protein or a portion thereof. Still more typically, the antibody (or fragment thereof) has binding affinity for residues about 40 to about 44 of the N-terminus of a p53 protein or a portion thereof.

Even more typically, the antibody has binding affinity to residues of the central domain of a p53 protein or a portion thereof.

The following features relate to the first through to seventh embodiments of the invention.

Typically, the disease is selected from the group consisting of: cancer, rheumatoid arthritis and coronary heart disease. More typically, the disease is cancer. Typically, the cancer is selected from the group consisting of: carcinogenic tumours; tumours of epithelial origin, such as colo-rectal cancer, breast cancer, lung cancer, head and neck tumours, hepatic cancer, pancreatic cancer, ovarian cancer, gastric cancer, brain cancer, bladder cancer, prostate cancer and urinary/genital tract cancer, oesophageal cancer; mesenchymal tumours, such as sarcoma; and haemopoietic tumours, such as B cell lymphoma.

According to an eighth embodiment of the invention, there is provided a vector comprising the nucleic acid molecule as defined in accordance with the first or second embodiments of the invention.

Typically, the vector is a shuttle or expression vector. More typically, the vector is selected from the group consisting of: viral, plasmid, bacteriophage, phagemid, cosmid, bacterial artificial chromosome, and yeast artificial chromosome.

Typically, the vector is a plasmid and may be selected from the group consisting of: pBR322, M13mp18, pUC18 and pUC19.

Typically, the vector is a bacteriophage and may be selected from λgt10 and λgt11 or phage display vectors. More typically, the phage display vector is selected from vectors derived from pCOMB vectors. Even more typically, the phage display vector is of the MCO group, which for example, may include MCO1, MCO3 and MCO6 vectors. Still more typically, the vector is MCO3.

Typically, the vector is a mammalian expression vector, such as pG1 D102-MCO or pKN100-MCO.

Typically, the vector includes expression control sequences, such as an origin of replication, a promoter, an enhancer, and necessary processing information sites, such as ribosome binding sites, RNA splice sites, polyadenylation sites, and transcriptional terminator sequences.

More typically, the MCO vector contains, amongst others, sequences selected from the group consisting of: polypeptide tag, amber codon, geneIII, heavy and light chain specific multicloning site, ompA and/or pelB leader sequences, subtilisin cleavage site, and/or 6 histidine tag.

Still more typically, the vector may include selection markers to permit detection of those cells transformed with the desired polynucleotide sequences.

Typically, the vector may include heterologous coding sequence or sequences to permit the expression of a fusion protein comprising the polypeptide of the third, fourth or fifth embodiments.

According to a ninth embodiment of the invention, there is provided a host cell transformed with the vector as defined in accordance with the eighth embodiment of the invention.

Typically, the host cells are procaryotic or eucaryotic in nature.

More typically, the procaryotic host cells include bacteria, and examples of such bacteria include: *E. coli, Bacillus, Streptomyces, Pseudomonas, Salmonella,* and *Serratia.*

More typically, the eucaryotic host cells may be selected from the group consisting of: yeast, fungi, plant, insect cells and mammalian cells, either *in vivo* or in tissue culture. Examples of mammalian cells include: CHO cell lines, COS cell lines, HeLa cells, L cells, murine 3T3 cells, c6 glioma cells and myeloma cell lines.

Still more typically, the eucaryotic host cells are CHO DG44 cells.

According to a tenth embodiment of the invention, there is provided a vertebrate comprising a host cell as defined in accordance with the ninth embodiment of the invention, wherein said vertebrate does not include humans.

### 3. Pharmaceutical/Therapeutic and Diagnostic Compositions

According to an eleventh embodiment of the invention, there is provided a pharmaceutical composition comprising the polypeptide, or peptide fragment, as defined in accordance with the third, fourth or fifth embodiments of the invention, or an antibody (or fragment thereof) as defined in accordance with the sixth or seventh embodiments of the invention, together with a pharmaceutically acceptable carrier, adjuvant and/or diluent.

Typically, the antibody present in the pharmaceutical composition may exist as a whole antibody, or be present as an antibody fragment or other immunologically active fragments thereof, such as complementarity determining regions. More typically, the antibody fragment has functional antigen-binding domains, that is, heavy and light chain variable domains. Even more typically, the antibody fragment may exist in a form selected from the group consisting of: Fv, $F_{ab}$, $F(ab)_2$, scFv (single chain Fv), dAb (single domain antibody), bi-specific antibodies, diabodies and triabodies.

Typically, the polypeptide, peptide fragment, or antibody or fragment thereof present in the pharmaceutical composition may also exist in a form selected from the group consisting of: polypeptide/chelate, polypeptide/drug, polypeptide/prodrug, polypeptide/toxin, polypeptide/imaging marker, antibody/chelate, antibody/drug, antibody/prodrug, antibody/toxin and antibody/imaging marker.

More typically, the chelate may be selected from the group consisting of: $^{90}Y$, $^{131}I$ and $^{188}Re$.

More typically, the drug may be a cytotoxic drug. Even more typically, the cytotoxic drug may be selected from the group consisting of: adriamycin, melphalan, cisplatin, taxol, fluorouricil, cyclophosphamide and others known to those of skill in the art such as those included in "The Chemotherapy Source Book", M.C.Perry Williams and Wilkins, 2nd Ed, 1996), the entire contents of which are incorporated herein by reference.

More typically, the prodrug may be antibody directed prodrug therapy or ADEPT.

More typically, the toxin may be selected from the group consisting of: ricin, abrin, *Diptheria* toxin and *Pseudomonas* endotoxin (PE 40).

Typically, the imaging marker includes substances which can be detected by a gamma scanner or hand held gamma probe, and substances which can be detected by nuclear magnetic resonance imaging using a nuclear magnetic resonance spectrometer.

More typically, the imaging marker which may be detected using a gamma scanner include imaging markers selected from the group consisting of $^{125}I$, $^{131}I$, $^{123}I$, $^{111}In$, $^{105}$ Rh, $^{153}Sm$, $^{67}Cu$, $^{67}Ga$, $^{166}Ho$, $^{177}Lu$, $^{186}Re$, Re, and $^{99m}Tc$.

Typically, the imaging marker which can be detected using a nuclear magnetic resonance spectrometer is gadolinium.

Typically, the pharmaceutical composition in accordance with the eleventh embodiment of the invention may also include cytokines, such as: G-CSF, GM-CSF, interleukins.

Typically, the pharmaceutical composition in accordance with the eleventh embodiment of the invention may also include an adjuvant, such as mannan.

According to a twelfth embodiment of the invention, there is provided a vaccine, wherein said vaccine comprises a nucleic acid molecule as defined in accordance with the first or second embodiments of the invention, or a fragment thereof, or a polypeptide, or peptide fragment, as defined in accordance with the third, fourth or fifth embodiments of the invention, or an antibody or fragment thereof as defined in accordance with the sixth or seventh embodiments of the invention, together with a pharmaceutically acceptable carrier, adjuvant and/or diluent.

Typically, the vaccine is an idiotypic vaccine.

Typically, the antibody (or a fragment thereof) of the present invention may be used as an idiotypic immunogen. When used in this manner, the antibody (or a fragment thereof) of the present invention may function as an immunogen and elicit a second antibody (Ab2) and T cell ($T_2$) response against idiotopes of the original antibody (Ab1). Ab2 antibodies can bind to epitopes on the original antibody including the antigen binding site (idiotype). The anti-idiotypic antibody, Ab2, can spontaneously induce anti-anti-idiotypic antibodies (Ab3) as well as T cells ($T_3$) which may recognise the same epitope as Ab1. Since the first antibody binds both the p53 epitope and Ab2, Ab2 mimics the structure of the antigenic epitope (on p53). A proportion of Ab3 antibodies bind to the same epitope as the original antibody (Ab1), and may augment and prolong the efficacy of the original antibody. Induction of this anti-idiotypic network results in protection from metastases partly through the induction of p53-specific CTLs.

Alternatively, a vaccine composition containing a peptide fragment of the polypeptide of the present invention may be prepared by synthesis of a peptide. For example, the peptide may comprise selected amino acid regions of the CDR and/or FR of the polypeptide of the invention. Typically, the peptide fragment of the polypeptide of the present invention may or may not have binding affinity for a p53 protein or a portion thereof in vertebrates.

Typically, the vaccine is formulated for administration via an oral, inhalation, topical or parenteral route. More typically, the route of administration is parenteral.

According to a thirteenth embodiment of the invention, there is provided a method for inducing an immune response against disease in a vertebrate, comprising administering to said vertebrate an immunologically effective amount of the polypeptide (or peptide fragment thereof) as defined in accordance with the third, fourth or fifth embodiments of the invention, or an antibody (or fragment thereof) as defined in accordance with the sixth or seventh embodiments of the invention, or a pharmaceutical composition as defined in accordance with the eleventh embodiment of the invention, or a vaccine as defined in accordance with the twelfth embodiment of the invention.

Typically, the polypeptide, or peptide fragment, or antibody (or fragment thereof) as administered in accordance with the thirteenth embodiment of the invention, is administered together with a pharmaceutically acceptable carrier, adjuvant and/or diluent.

Typically, the polypeptide, or peptide fragment, or antibody (or fragment thereof) as administered in accordance with the thirteenth embodiment of the invention, may also be simultaneously or sequentially administered with cytokines, such as: G-CSF, GM-CSF, interleukins.

Typically, the pharmaceutical composition in accordance with the thirteenth embodiment of the invention may also include an adjuvant, such as mannan.

According to a fourteenth embodiment of the invention, there is provided the polypeptide (or peptide fragment thereof) as defined in accordance with the third, fourth or fifth embodiments of the invention, or an antibody (or fragment thereof) as defined in accordance with the sixth or seventh embodiments of the invention, or a pharmaceutical composition as defined in accordance with the eleventh embodiment of the invention, or a vaccine as defined in accordance with the twelfth embodiment of the invention, when used in inducing an immune response against disease in a vertebrate.

According to a fifteenth embodiment of the invention, there is provided the use of the polypeptide (or peptide fragment thereof) as defined in accordance with the third, fourth or fifth embodiments of the invention, or an antibody (or fragment thereof) as defined in accordance with the sixth or seventh embodiments of the invention, in the preparation of a vaccine for inducing an immune response against disease in a vertebrate.

According to a sixteenth embodiment of the invention, there is provided a method for inducing an immune response against disease in a vertebrate, comprising administering to said vertebrate an immunologically effective amount of the vaccine as defined in accordance with the twelfth embodiment of the invention.

According to a seventeenth embodiment of the invention, there is provided a vaccine as defined in accordance with the twelfth embodiment of the invention when used in inducing an immune response against disease in a vertebrate.

According to an eighteenth embodiment of the invention, there is provided a method for the treatment and/or prophylaxis of disease in a vertebrate in need of said treatment and/or prophylaxis, wherein said method comprises administering a therapeutically effective amount of the polypeptide (or peptide fragment thereof) in accordance with the third, fourth or fifth embodiments of the invention, or an antibody (or fragment thereof) in accordance with the sixth or seventh embodiments of the invention, or a pharmaceutical composition as defined in accordance with the eleventh embodiment of the invention, or a vaccine as defined in accordance with the twelfth embodiment of the invention.

According to a nineteenth embodiment of the invention, there is provided the polypeptide (or peptide fragment thereof) in accordance with the third, fourth or fifth embodiments of the invention, or an antibody (or fragment thereof) in accordance with the sixth or seventh embodiments of the invention, or a pharmaceutical composition as defined in accordance with the eleventh embodiment of the invention, or a vaccine as defined in accordance with the twelfth embodiment of the invention, when used in the treatment and/or prophylaxis of disease in a vertebrate in need of said treatment and/or prophylaxis.

According to a twentieth embodiment of the invention, there is provided use of the polypeptide (or peptide fragment thereof) in accordance with the third, fourth or fifth embodiments of the invention, or an antibody (or fragment thereof) in accordance with the sixth or seventh embodiments of the invention, or a pharmaceutical composition as defined in accordance with the eleventh embodiment of the invention, or a vaccine as defined in accordance with the twelfth embodiment of the invention in the preparation of a medicament for the treatment and/or prophylaxis of disease in a vertebrate in need of said treatment and/or prophylaxis.
Typically, the disease is selected from the group consisting of: cancer, rheumatoid arthritis and coronary heart disease. More typically, the disease is cancer.
Typically, the cancer is selected from the group consisting of: carcinogenic tumours; tumours of epithelial origin, such as colo-rectal cancer, breast cancer, lung cancer, head and neck tumours, hepatic cancer, pancreatic cancer, ovarian cancer, gastric cancer, brain cancer, bladder cancer, prostate cancer and urinary/genital tract cancer, oesophageal cancer; mesenchymal tumours, such as sarcoma; and haemopoietic tumours, such as B cell lymphoma.

**4. An antibody/nucleic acid based method and kit for detecting p53**

According to a twenty-first embodiment of the invention, there is provided a diagnostic kit for the detection of polypeptides encoded by the p53 gene in vertebrates, comprising the antibody (or fragment thereof) as defined in accordance with the sixth or seventh embodiments of the invention, together with a diagnostically acceptable carrier and/or diluent.
Typically, the kit may comprise the following containers:

(a) a first container containing the antibody (or fragment thereof) as defined in accordance with the sixth or seventh embodiments of the invention, and;
(b) a second container containing a conjugate comprising a binding partner of the antibody (or fragment thereof), together with a detectable label.

More typically, the kit may further comprise one or more other containers, containing other components, such as wash reagents, and other reagents capable of detecting the presence of bound antibodies. Even more typically, the detection reagents may include: labelled (secondary) antibodies, or where the antibody (or fragment thereof) of the present invention is itself labelled, the compartments may comprise antibody binding reagents capable of reacting with the labelled antibody (or fragment thereof) of the present invention.
According to a twenty-second embodiment of the invention, there is provided a method for screening for a disease in a vertebrate comprising

(a) contacting a sample from a vertebrate with a nucleic acid probe, and
(b) detecting hybridisation between the nucleic acid sample and the polynucleotide sequence.

Typically, hybridisation as compared to non-hybridisation is indicative of disease. Typically, the disease is cancer.
Typically, the nucleic acid probe corresponds to a portion of the polynucleotide sequence as defined in accordance with the first or second embodiments of the invention which is capable of selectively hybridising to nucleic acid from a sample.
Typically, hybridisation may occur and be detected through techniques that are routine and standard amongst those skilled in the art, and include southern and northern hybridisation, polymerase chain reaction (PCR) and ligase chain reaction (LCR) amplification.
Various low, medium or high stringency hybridisation levels may be used, depending on the specificity and selectivity desired.
According to a twenty-third embodiment of the invention, there is provided a method for screening for a disease in a vertebrate comprising:

(a) contacting a sample from a vertebrate with the antibody (or fragment thereof) defined in accordance with the sixth or seventh embodiments of the invention, and
(b) detecting the presence of the antibody (or fragment thereof) bound to a p53 polypeptide.

Typically, altered levels of the p53 polypeptide in the sample as compared to normal levels indicate disease. Typically, the disease is cancer.

**5. Gene Therapy**

According to a twenty-fourth embodiment of the invention, there is provided a method of gene therapy, wherein said method comprises:

(a) inserting a nucleic acid molecule as defined in accordance with the first or second embodiments of the invention, or a vector as defined in accordance with the eighth embodiment of the invention, into a host cell;
(b) expressing the nucleic acid molecule in the transformed cell.

Typically, the nucleic acid molecule or vector is inserted using methods selected from the group consisting of: microinjection, $CaPO_4$ precipitation, electroporation, lipofection/liposome fusion, particle bombardment and coupling the nucleic acid to chemically modified proteins.

Typically the nucleic acid molecule or vector is inserted into the nucleus of a host cell.

Typically, an expression vector containing the nucleic acid molecule is inserted into cells, the cells are grown *in vitro* and then infused in large numbers into patients. More typically, expression vectors derived from viruses such as retroviruses, vaccinia virus, adenovirus, adeno-associated virus, herpes viruses, several RNA viruses, or bovine papilloma virus, may be used for delivery of the nucleic acid into the targeted cell population. More typically, the targeted cell population comprises tumour cells.

**6. Preparing antibody (or fragment thereof) having binding affinity to a p53 protein or a portion thereof in vertebrates**

According to a twenty-fifth embodiment of the invention, there is provided a process for preparing an antibody (or fragment thereof) having binding affinity to a p53 protein or a portion thereof in vertebrates, wherein said process comprises:

(a) isolating from a vertebrate a nucleic acid molecule as defined in accordance with the first or second embodiments of the invention;
(b) cloning said nucleic acid molecule into a vector;
(c) constructing an antibody fragment library; and
(d) screening said library for clones expressing the antibody of interest.

Typically, the antibody (or fragment thereof) as prepared by the process as defined in accordance with the twenty-fifth embodiment of the invention has binding affinity to a p53 protein or a portion thereof in vertebrates.

Typically, the nucleic acid sample is obtained from individuals suffering a disease associated with the expression of p53, who express antibodies reactive with p53. More typically, the disease is selected from the group consisting of: cancer, rheumatoid arthritis and coronary heart disease. Even more typically, the disease is cancer. Still more typically, the cancer is selected from the group consisting of: carcinogenic tumours; tumours of epithelial origin, such as colorectal cancer, breast cancer, lung cancer, head and neck tumours, hepatic cancer, pancreatic cancer, ovarian cancer, gastric cancer, brain cancer, bladder cancer, prostate cancer and urinary/genital tract cancer, oesophageal cancer; mesenchymal tumours, such as sarcoma; and haemopoietic tumours, such as B cell lymphoma.

Typically, the nucleic acid sample is taken from an organ suffering from, or a collection point for expression of, the disease. Even more typically, the organ is a lymph node.

Typically, the nucleic acid sample is comprised of polynucleotide sequences in accordance with the first or second embodiments of the invention.

Typically, the nucleic acid sample is mRNA. More typically, the clone is prepared through RT-PCR (reverse transcriptase- PCR) and cloned into a suitable vector.

Typically, the vector is a phage display vector. More typically, the vector is selected from the group consisting of: MCO1, MCO3 and MCO6. Even more typically, the vector is MCO1.

Typically, nucleic acid clones are packaged into the phage display library to produce a primary antibody library. More typically, the phage display library was amplified by panning against recombinant p53, and selected recombinant antibodies obtained.

Typically, the antibody library represents clones expressing an antibody fragment, wherein said antibody has binding affinity to a p53 protein or a portion thereof. More typically, the antibody fragment is an $F_{ab}$ fragment. Even more typically, the recombinant antibody fragment is purified. Still more typically, the antibody fragment has binding affinity to a p53 protein or a portion thereof.

Typically, the antibody (or fragment thereof) having binding affinity to a p53 protein or a portion thereof is as defined in accordance with the sixth or seventh embodiments of the invention.

According to a twenty-sixth embodiment of the invention, there is provided a method of locating a nucleotide sequence encoding a polypeptide of an antibody (or fragment thereof) having binding affinity to a p53 protein or portion thereof in vertebrates, using the nucleic acid molecule of the first or second embodiments of the invention.

Typically, the method comprises:

(a) contacting a biological sample with a nucleic acid molecule of the first or second embodiments of the invention (or oligonucleotide fragment thereof); and
(b) identifying nucleotide sequences in the biological sample which hybridise to said nucleic acid molecule.

Typically, step (a) is performed under conditions which promote hybridisation of homologous sequences, which conditions are well known to those of skill in the art.

Specifically contemplated in this embodiment of the invention is a method of locating a nucleotide sequence encoding a polypeptide of an antibody (or fragment thereof), wherein said antibody has binding affinity to a p53 protein or portion thereof in vertebrates.

Typically, the method is a method of locating a nucleotide sequence encoding a polypeptide of an antibody (or fragment thereof) having binding affinity to residues of one or more of the N-terminus, the C-terminus or the central domain of a p53 protein or a portion thereof.

More typically, the method is a method of locating a nucleotide sequence encoding a polypeptide of an antibody (or fragment thereof) having binding affinity to residues of the N-terminus of a p53 protein or a portion thereof.

Even more typically, the antibody (or fragment thereof) has binding affinity for residues of the N-terminus of a p53 protein or a portion thereof. Yet more typically, the antibody (or fragment thereof) has binding affinity for residues about 10 to about 55. Still more typically, the antibody (or fragment thereof) has binding affinity for residues about 10 to about 25, or about 40 to about 50, or about 27 to about 44 of the N-terminus of a p53 protein or portion thereof or about 40 to about 44 of the N-terminus of a p53 protein or portion thereof. Even more typically, the antibody (or fragment thereof) has binding affinity for residues about 27 to about 44 of the N-terminus of a p53 protein or a portion thereof. Still more typically, the antibody (or fragment thereof) has binding affinity for residues about 40 to about 44 of the N-terminus of a p53 protein or a portion thereof.

Even more typically, the method is a method of locating a nucleotide sequence encoding a polypeptide of an antibody (or fragment thereof) having binding affinity to residues of the central domain of a p53 protein or a portion thereof.

## Definitions

The term "antibody" means an immunoglobulin molecule able to bind to a specific epitope on an antigen. Antibodies can be comprised of a polyclonal mixture, or may be monoclonal in nature. Further, antibodies can be entire immunoglobulins derived from natural sources, or from recombinant sources. The antibodies of the present invention may exist in a variety of forms, including for example as a whole antibody, or as an antibody fragment, or other immunologically active fragment thereof, such as complementarity determining regions. Similarly, the antibody may exist as an antibody fragment having functional antigen-binding domains, that is, heavy and light chain variable domains. Also, the antibody fragment may exist in a form selected from the group consisting of: Fv, $F_{ab}$, $F(ab)_2$, scFv (single chain Fv), dAb (single domain antibody), bi-specific antibodies, diabodies and triabodies.

By "antigen-recognising portion" is meant one or more portions of a variable region of an antibody (or fragment thereof) which are responsible for binding and/or recognising the target antigen (or epitope or idiotype) of the antibody. For example, it includes the CDR regions or the whole variable region, or any combination of these two regions including any changes in coding regions that may be induced in the region, without altering the binding properties of the antibody.

The antibody (or fragment thereof) of the present invention has binding affinity to a p53 protein or a portion thereof in vertebrates. Preferably, the antibody (or fragment thereof) of the present invention has binding affinity or avidity greater than about $10^5$ $M^{-1}$ more preferably greater than about $10^6$ $M^{-1}$ more preferably still greater than about $10^7$ $M^{-1}$ and most preferably greater than about $10^8$ $M^{-1}$. The techniques for generating and reviewing binding affinity are reviewed in Scatchard (1949), Annals of the New York Academy of Sciences, 51, 660-672, and Munson (1983), Methods in Enzymology 92, 543-577, the contents of each of which are incorporated herein by reference.

As used herein "gene transfer" means the process of introducing a foreign nucleic acid molecule into a cell. Gene transfer is commonly performed to enable the expression of a particular product encoded by the gene. The product may include a protein, polypeptide, anti-sense DNA or RNA, or enzymatically active RNA. Gene transfer can be performed in cultured cells or by direct administration into animals. Generally gene transfer involves the process of nucleic acid contact with a target cell by non-specific or receptor mediated interactions, uptake of nucleic acid into the cell through the membrane or by endocytosis, and release of nucleic acid into the cytoplasm from the plasma membrane or endosome. Expression may require, in addition, movement of the nucleic acid into the nucleus of the cell and binding to appropriate nuclear factors for transcription.

As used herein "gene therapy" is a form of gene transfer and is included within the definition of gene transfer as used herein and specifically refers to gene transfer to express a therapeutic product from a cell *in vivo* or *in vitro*. Gene transfer can be performed ex vivo on cells which are then transplanted into a patient, or can be performed by direct administration of the nucleic acid or nucleic acid-protein complex into the patient, or can be performed by transfer of modified cells into a patient.

As used herein, the term "naturally-occurring disease" refers to a disease that has spontaneously arisen, not iatragenically induced.

The term "wild-type", in terms of a gene or a gene product, refers to that gene or a gene product which is characteristic of most of the members of a species occurring naturally, and is thus arbitrarily designated the "normal" or "wild-type" form of the gene or gene product.

The term "mutant", in terms of a gene or gene product, refers a change in the gene or gene product when compared to the wild-type gene or gene product.

The term "isolated and purified" means that the material in question has been removed from its host, and associated impurities reduced or eliminated. Essentially, it means an object species is the predominant species present (ie., on a molar basis it is more abundant than any other individual species in the composition), and preferably a substantially purified fraction is a composition wherein the object species comprises at least about 30 percent (on a molar basis) of all macromolecular species present. Generally, a substantially pure composition will comprise more than about 80 to 90 percent of all macromolecular species present in the composition. Most preferably, the object species is purified to essential homogeneity (contaminant species cannot be detected in the composition by conventional detection methods) wherein the composition consists essentially of a single macromolecular species.

The term "operably linked" refers to the situation wherein for example, a nucleic acid is "operably linked" when it is placed into a functional relationship with another nucleic acid sequence. For instance, a promoter or enhancer is operably linked to a coding sequence if it effects the transcription of the coding sequence.

Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Typically, conservative amino acids substitution groups are: valine-leucine-isoleucine, phenylalanine-tyrosine, lysine-arginine, alanine-valine, and asparagine-glutamine.

As used herein the term "polypeptide" means a polymer made up of amino acids linked together by peptide bonds.

The term "analogue" as used herein with reference to a nucleic acid sequence means a sequence which is a derivative of the nucleic acid sequences of the invention, which derivative comprises addition, deletion, substitution of one or more bases and wherein the encoded polypeptide retains substantially the same function as the polypeptide encoded by the nucleic acid sequences of SEQ ID NOS 1-30.

In the context of this specification, the term "comprising" means "including principally, but not necessarily solely". Further, variations of the word "comprising", such as "comprise" and "comprises" have correspondingly varied meanings.

## BRIEF DESCRIPTION OF THE DRAWINGS

Figure 1: The reactivity of Fab against varying concentrations of p53 in a direct ELISA. Fabs binding to p53 were detected with 9E10 followed by a goat anti-mouse specific HRP conjugated antibody. Anti-Tetanus Toxoid indicates the signal obtained when a Fab which reacted with Tetanus toxoid was used.

Figure 2: Cross reactivity of Fabs with other antigens as assessed by ELISA. Binding Fabs (from crude bacterial supernatant) were detected with 9E10 followed by a goat anti-mouse specific HRP conjugated antibody. The signal obtained for p53 was four times greater than observed with other antigens.

Figure 3:(A) Binding of Fab clones to recombinant p53 in bacterial lysates. The binding of DO7 was detected with an HRP-goat anti-mouse (lane 1). The human-anti-p53 Fabs (163.1,5,17,24; lanes 3-6) and human-anti-tetanus Fab (negative control; lane 2) were detected with 9E10 followed by HRP-goat anti-mouse antibodies.

(B) Immunoblot analysis of immunoprecipitates from the colorectal cancer cell line HT-29. Immunoprecipitation was performed using the DO7 positive control antibody (lane 2), human Fab antibody reactive with tetanus toxoid (lane 1), Fab from clones 163.1, 5, 17, 24 (lanes 3-6 respectively) and Protein A with lysate alone (lanes 7). Following immunoprecipitation and electroblotting the blots were incubated with a goat anti p53 antibody followed by a HRP-conjugated donkey anti-goat antibody.

Figure 4: Deduced amino acid sequence of heavy (4A) and light chain (4B) clones reactive with p53. Replacement (uppercase) and silent mutations (lowercase) are shown with respect to the most homologous germline sequence.
Figure 5: Map of the mammalian expression vector. The heavy and light chain genes of the selected Fab clones were cloned into the *Not*1 and *Spe*1 of pG1D105 and the *Sal*1 and *Xba*1 of pKN101 respectively. The heavy and light chain vectors contain the neomycin resistance and dihydrofolate reductase genes, which confer resistance to the antibiotics G418 and methotrexate respectively and allow selection of transformed cells.
Figure 6: A 10% PAGE showing the purification of the whole anti-p53 antibody from clone C4B4. The molecular weight markers are indicated on the left of the gel, the arrows on the right show the position of the heavy and light chain.
Figure 7: Cross-reactivity of C4B4 and DO7 with p53 and other antigens as assessed by ELISA. C4B4 was detected with a HRP conjugated goat anti-human specific antibody and DO7 with a HRP conjugated goat anti-mouse specific antibody. Reactivity with the HRP conjugated goat anti-human was used as a negative control.
Figure 8: Immunoblot analysis of immunoprecipitates from the colorectal cancer cell line HT-29, MCF-7 and MethA. Immunoprecipitation was performed using the purified C4B4 and the anti-p53 monoclonal antibody DO7. A immunoprecipitation with Protein A alone with lysate was used as a negative control. Following immunoprecipitation and electroblotting the blots were incubated with a goat anti-p53 antibody followed by a HRP conjugated donkey anti-goat-HRP. Immunoprecipitation with C4B4 resulted in a similar pattern of reactivity to that of the DO7. No significant reactivity was seen in the negative control.
Figure 9: Alignment of the sequence of the p53 gene fragment peptides that were isolated. Three phage clones with unique nucleotide sequence were compared to the sequence of p53. Fragment sizes ranged from 14-32 amino acids and corresponded to a region in the amino terminus of p53.
Figure 10: Immunohistochemical analysis of p53 staining in a colorectal tumour using the C4B4 antibody. Bound antibody was detected with HRP conjugated goat anti-human antibody. The larger frame shows positive nuclear staining in the colorectal epithelium (regions of brown staining), while a similar region of tissue with no C4B4 is shown in the smaller frame and does not demonstrate any nuclear staining of the tumour tissue (blue staining).
Figure 11: The reactivity of varying concentration of 1159.8 against whole p53, the central domain of p53. Fab binding to p53 were detected with 9E10 followed by alkaline phosphatase conjugated goat anti-mouse antibody. The reactivity of the antibody to BSA was used as a negative control.
Figure 12: Cross reactivity of Fabs with other antigens as assessed by ELISA. Fab binding to p53 were detected with 9E10 followed by alkaline phosphatase conjugated goat anti-mouse antibody.
Figure 13. Immunodetection of whole p53 and the central domain of p53 using the Fabs 1159.8 and 163.1. A 4-20% SDS/PAGE was electroblotted onto PVDV and probed with the Fabs 1159.8 and 163.1, and the positive control murine monoclonal antibodies, DO7 and Pab 240. Fabs were detected with alkaline phosphatase conjugated goat anti-human $Fab_2$ specific, while murine antibodies were detected with an with alkaline phosphatase conjugated goat anti-mouse antibody.

## DETAILED DESCRIPTION OF THE INVENTION

### 1. Nucleic Acid Encoding a Polypeptide of an Antibody or fragment thereof to p53.

Included within the scope of this invention are the functional equivalents of the herein-described isolated nucleic acid molecules. The degeneracy of the genetic code permits substitution of certain codons by other codons which specify the same amino acid and hence would give rise to the same protein. The nucleic acid sequence can vary substantially since, with the exception of methionine and tryptophan, the known amino acids can be coded for by more than one codon. Thus, portions or all of the polynucleotide sequences of the present invention could be synthesised to give a nucleic acid sequence significantly different from that described herein (Figure 4). However, the encoded amino acid sequence thereof would be preserved.

In addition, the nucleic acid sequence may comprise a nucleotide sequence which results from the addition, deletion or substitution of at least one nucleotide to the 5'-end and/or the 3'-end of the nucleic acid formula shown in Figure 4, or a derivative thereof. For example, the present invention is intended to include any nucleic acid sequence resulting from the addition of ATG as an initiation codon at the 5'-end of the inventive nucleic acid sequence or its derivative, or from the addition of TTA, TAG or TGA as a termination codon at the 3'-end of the inventive nucleotide sequence or its derivative. Moreover, the nucleic acid molecule of the present invention may, as necessary, have restriction endonuclease recognition sites added to its 5'-end and/or 3'-end.

Such functional alterations of a given nucleic acid sequence afford an opportunity to promote secretion and/or processing of heterologous proteins encoded by foreign nucleic acid sequences fused thereto. All variations of the nucleotide sequences of the present invention, and fragments thereof permitted by the genetic code are, therefore, included in this invention.

Further, it is possible to delete codons or to substitute one or more codons by codons other than degenerate codons to produce a structurally modified polypeptide, but one which has substantially the same utility or activity of the polypeptide produced by the unmodified nucleic acid molecule. As recognised in the art, the two polypeptides are functionally equivalent, as are the two nucleic acid molecules which give rise to their production, even though the differences between the nucleic acid molecules are not related to degeneracy of the genetic code.

The nucleic acid molecule in accordance with the first or second embodiments of the invention may also include an expression control sequence operably linked to the coding sequences, including naturally-associated or heterologous promoter regions. Preferably, the expression control sequences will be procaryotic in nature in vectors capable of transforming or transfecting procaryotic host cells Even more preferably, the polynucleotides encoding the antibodies of the present invention are cloned into a phage display vector.

However, the nucleic acid molecule in accordance with the first or second embodiments of the invention may also be cloned into a eucaryotic expression system, and may also include an expression control sequence operably linked to the coding sequences, including naturally-associated or heterologous promoter regions. Preferably, the expression control sequences will be eucaryotic promoter systems in vectors capable of transforming or transfecting eucaryotic host cells.

Once the vector has been incorporated into the appropriate host, the host is maintained under conditions suitable for high level expression of the nucleotide sequences, and the collection and purification of the expressed immunoglobulin.

Further, the host cells are chosen, such that upon insertion of the vector into the host, selective features of the vector enable the relevant expressed polypeptide to either be displayed on the surface of the host cell, or secreted/ expressed into the culture medium. Examples of such cells include XL1-Blue and HB2151 respectively. Effectively, soluble expression of Fab by any non-suppressor strain is envisaged, and these may include *E. coli* HB2151 or MC1061. Alternatively, a suppressor strain for the expression of Fab fused to the surface of a phage, such as *E. coli* XL1-blue or TG-1α.

These expression vectors are typically replicable in the host organisms either as episomes or as an integral part of the host chromosomal DNA. Commonly, expression vectors will contain selection markers. For example, typical selection markers include ampicillin-resistance or hygromycin-resistance, thereby permitting detection of those cells transformed with the desired DNA sequences.

In general, procaryotes can be used for cloning the DNA sequences of the present invention. *E. coli* is one procaryotic host particularly useful for cloning the DNA sequences of the present invention. Typically, *E. coli* produces antibody (or fragment thereof), such as Fab, by way of a phage particle which is itself produced in bacteria. Specific example strains include HB2151, which expresses a soluble antibody (or fragment thereof), and XL1-Blue which expresses the antibody (or fragment thereof) on the cell surface eg., phage display.

Eucaryotic organisms, such as yeast are also useful for expression. *Saccharomyces sp.* is a preferred yeast host, with suitable vectors having expression control sequences, an origin of replication, termination sequences and the like as desired. Typical promoters include 3-phosphoglycerate kinase and other glycolytic enzymes. Inducible yeast promoters include, among others, promoters from alcohol dehydrogenase 2, isocytochrome C, and enzymes responsible for maltose and galactose utilisation.

Mammalian cells are also typical hosts for expressing nucleotide segments encoding immunoglobulins or fragments thereof. A number of suitable host cell lines capable of secreting intact heterologous proteins have been developed in the art, and include CHO cell lines, various COS cell lines, HeLa cells, L cells and myeloma cell lines. Expression vectors for these cells can include expression control sequences, such as an origin of replication, a promoter, an enhancer, and necessary processing information sites, such as ribosome binding sites, RNA splice sites, polyadenylation sites, and transcriptional terminator sequences.

The vectors containing the DNA segments of interest can be transferred into the host cell by well-known methods, depending on the type of cellular host. For example, calcium chloride transfection and electroporation are commonly utilised for procaryotic cells, whereas calcium phosphate treatment, electroporation, lipofection, biolistics or viral-based transfection may be used for other cellular hosts. Other methods used to transform mammalian cells include the use of transfection, transformation, conjugation, protoplast fusion, polybrene, liposomes, electroporation, particle gun technology and microinjection (see, generally, Sambrook *et al.,* 1989).

After introduction of the vector, recipient host cells are generally grown in a selective medium, which inherently selects for the growth of those cells containing the introduced vector. A variety of incubation conditions can be used to form the polypeptides of the present invention, but the most preferred conditions are those which mimic physiological.

Alternatively, modification or inactivation of the nucleic acid sequences of the invention may be undertaken using well known anti-sense techniques, that is, involving a nucleotide sequence complementary to the polypeptide encoding sequence. More specifically, production of a polypeptide encoded by the nucleic acids of the invention, may be altered, that is, reduced or eliminated by introducing a polynucleotide sequence(s) complementary to these nucleic acid sequences encoding the polypeptide(s) which may be transcribed in a cell, and is capable of hybridising to the resulting mRNA produced in the cell. On the basis that the reaction occurs conditions allowing the complementary antisense nucleotide sequence to hybridise to the polypeptide mRNA, the amount of polypeptide translated is thus altered, that is, reduced or eliminated.

**2. Polypeptide of an Antibody or fragment thereof to p53 and/or Antibody or fragment thereof to p53.**

Antibodies or immunoglobulins are typically composed of four covalently bound peptide chains. For example, an IgG antibody has two light chains and two heavy chains. Each light chain is covalently bound to a heavy chain. In turn each heavy chain is covalently linked to the other to form a "Y" configuration, also known as an immunoglobulin conformation. Fragments of these molecules, or even heavy or light chains alone, may bind antigen.

A normal antibody heavy or light chain has an N-terminal ($NH_2$) variable (V) region, and a C-terminal (COOH) constant (C) region. The heavy chain variable region is referred to as $V_H$ (including, for example, $V_\gamma$), and the light chain variable region is referred to as $V_L$ (including $V_\kappa$ or $V_\lambda$). The variable region is the part of the molecule that binds to the antibody's cognate antigen, while the Fc region (the second and third domains of the C region) on the heavy chain determines the antibody's effector function (eg., complement fixation, opsonisation). Full-length immunoglobulin or antibody "light chains" are encoded by a variable region gene at the N-terminus and a κ (kappa) or λ (lambda) constant region gene at the COOH-terminus. Full-length immunoglobulin or antibody "heavy chains", are similarly encoded by a variable region gene and one of the constant region genes, eg., gamma. Typically, the "$V_L$ " will include the portion of the light chain encoded by the $V_L$ and $J_L$ (J or joining region) gene segments and the "$V_H$" will include the portion of the heavy chain encoded by the $V_H$, and $D_H$ (D or diversity region) and $J_H$ gene segments.

An immunoglobulin light or heavy chain variable region consists of a "framework" region interrupted by three hypervariable regions, also called complementarity-determining regions or CDRs. The sequences of the framework regions of different light or heavy chains are relatively conserved within a species. The framework region of an antibody, that is the combined framework regions of the constituent light and heavy chains, serves to position and align the CDRs in three dimensional space. The CDRs are primarily responsible for binding to an epitope of an antigen. The CDRs are typically referred to as CDR1, CDR2, and CDR3, numbered sequentially starting from the N-terminus.

The two types of light chains, κ (kappa) and λ (lambda), are referred to as isotypes. Isotypic determinants typically reside in the constant region of the light chain, also referred to as the $C_L$ in general, and $C_\kappa$ or $C_\lambda$ in particular Likewise, the constant region of the heavy chain molecule, also known as $C_H$, determines the isotype of the antibody. Antibodies are referred to as IgM, IgD, IgG, IgA, and IgE depending on the heavy chain isotype. The isotypes are encoded in the μ (mu), δ (delta), γ (gamma), α (alpha), and ε (epsilon) segments of the heavy chain constant region, respectively.

The heavy chain isotypes determine different effector functions of the antibody, such as opsonisation or complement fixation. In addition, the heavy chain isotype determines the secreted form of the antibody. Secreted IgG, IgD, and IgE isotypes are typically found in single unit or monomeric form. Secreted IgM isotype is found in pentameric form; secreted IgA can be found in both monomeric and dimeric form.

In a related aspect, the invention features a monoclonal antibody, or an Fab, $(Fab)_2$, scFv (single chain Fv), dAb (single domain antibody), bi-specific antibodies, diabodies and triabodies, or other immunologically active fragment thereof (eg., a CDR-region). Such fragments are useful as immunosuppressive agents. Alternatively, the antibody of the invention may have attached to it an effector or reporter molecule. For instance, an antibody or fragment thereof of the invention may have a macrocycle, for chelating a heavy metal atom, or a toxin, such as ricin, attached to it by a covalent bridging structure. In addition, the Fc fragment or $CH_3$ domain of a complete antibody molecule may be replaced or conjugated by an enzyme or toxin molecule, such as chelates, toxins, drugs or prodrugs, and a part of the immunoglobulin chain may be bonded with a polypeptide effector or reporter molecule, such as biotin, fluorochromes, phosphatases and peroxidases. Bispecific antibodies may also be produced in accordance with standard procedures well known to those skilled in the art.

The present invention further contemplates genetically modifying the antibody variable and/or constant regions to include effectively homologous variable and constant region amino acid sequences. Generally, changes in the variable region will be made to improve or otherwise modify antigen binding properties of the antibody or fragment thereof. Changes in the constant region will, in general, be made in order to improve or otherwise modify biological properties, such as complement fixation, interaction with membranes, and other effector functions.

In the present context, effectively homologous refers to the concept that differences in the primary structure of the variable region of the antibody (or fragment thereof) may not alter the binding characteristics of the antibody or fragment thereof. Changes of amino acids are permissible in effectively homologous sequences so long as the resultant antibody or fragment thereof retains its desired property.

Amino acid changes in the polypeptide or the antibody or fragment thereof may be effected by techniques well known persons skilled in the relevant art. For example, amino acid changes may be effected by nucleotide replacement techniques which include the addition, deletion or substitution of nucleotides, under the proviso that the proper reading frame is maintained. Exemplary techniques include random mutagenesis, site-directed mutagenesis, oligonucleotide-mediated or polynucleotide-mediated mutagenesis, deletion of selected region(s) through the use of existing or engineered restriction enzyme sites, and the polymerase chain reaction.

In a related aspect, the invention further contemplates peptide fragments of the polypeptides of SEQ ID NOs 31-60. For example, peptide fragments comprising between about 5 and about 50 contiguous amino acids, preferably between about 5 and about 35 amino acids, even more preferably between about 5 and about 30 amino acids, even more preferably still between about 5 and about 25 amino acids and yet more preferably still between about 8 and about 20 amino acids are contemplated in this aspect of the invention. It will be appreciated by those skilled in art that such peptide fragments may or may not have affinity for a p53 protein or a portion thereof.

For example, the peptide fragment may be selected from the VL chain and/or from the VH chain of the polypeptides of the present invention. Preferably, the peptide fragment may be selected from the complementarity determining region (CDR) and/or from the framework region (FR) of the VH and/or VL chain. More preferably still, the peptide fragment is selected from the VH and/or VL region of the CDR.

The peptide fragments of the present invention find industrial use, for example, in immunisation protocols to create an idiotypic response. As will be appreciated by those skilled in the art, the peptide fragments of the present invention may be used to immunise a patient in need of such immunisation by administration of an amount effective to induce immunity to p53, either wild-type or mutant. One skilled in the art would be able, by routine experimentation, to determine what an effective, non-toxic amount of peptide fragment would be for the present purpose. Generally, however, an effective dosage is expected to be in the range of about 5 milligrams to about 100 milligrams per dose, preferably about 5 milligrams to about 75 milligrams per dose, more preferably about 10 milligrams to about 50 milligrams per dose, even more preferably about 20 milligrams to about 40 milligrams per dose.

The polypeptide of the third, fourth and fifth embodiment and/or the antibody of the sixth and seventh embodiments of the present invention are also useful in functional studies of p53 protein in vertebrates. As will be apparent to those skilled in this art, example studies include assays for determining p53 expression in normal and disease states, the effect on cell growth and proliferation of antibody binding to p53. For example, functional studies may be performed *in vivo* or *in vitro*. More preferably, functional studies are performed *in vitro.*

### 3. Pharmaceutical/Therapeutic and Diagnostic Compositions

In another aspect, the invention features pharmaceutical compositions in which antibodies (or fragments thereof) of the present invention are provided for therapeutic, prophylactic or diagnostic uses. Such antibodies can also be provided as immunotoxins, that is, molecules which are characterised by two components and are particularly useful for killing selected cells *in vitro* or *in vivo*. One component is a cytotoxic agent which is usually fatal to a cell when attached or absorbed. The second component, known as the "delivery vehicle" provides a means for delivering the toxic agent to a particular cell type, such as carcinoma cells. The two components are commonly chemically bonded together by any of a variety of well-known chemical or genetic procedures. For example, when the cytotoxic agent is a protein and the second component is an intact immunoglobulin, the linkage may be by way of heterobifunctional crosslinkers, eg., carbodiimide, glutaraldehyde, and the like.

Once expressed, polypeptides of the present invention can be purified according to standard procedures of the art, including HPLC purification, size exclusion, ion-exchange and immuno-affinity (column) chromatography, gel electrophoresis and the like.

The antibodies of the present invention may be used as passive or active therapeutic agents against a number of human diseases, including cancer, wherein such cancer may include: tumours of epithelial origin, such as colo-rectal cancer, breast cancer, lung cancer, head and neck tumours, hepatic cancer, pancreatic cancer, brain cancer, ovarian cancer, gastric cancer, bladder cancer, prostate cancer and urinary/genital tract cancer, oesophageal cancer; mesenchymal tumours, such as sarcoma; and haemopoietic tumours, such as lymphoma.

The antibodies (or fragments thereof) of the present invention can be used either in their native form, or as part of an antibody/chelate, antibody/drug, antibody/prodrug, antibody/toxin or antibody/imaging marker complex. Additionally, whole antibodies or antibody fragments ($Fab_2$, Fab, Fv) may be used as imaging reagents or as potential vaccines or immunogens in active immunotherapy for the generation of anti-idiotypic responses.

Conjugates of the antibody (or fragment thereof) and imaging marker(s) may be administered in a pharmaceutically effective amount for the *in vivo* diagnostic assays of a number of human diseases, including cancer, wherein such cancer may include: tumours of epithelial origin, such as colo-rectal cancer, breast cancer, lung cancer, head and neck tumours, hepatic cancer, pancreatic cancer, brain cancer, ovarian cancer, gastric cancer, bladder cancer, prostate cancer and urinary/genital tract cancer, oesophageal cancer; mesenchymal tumours, such as sarcoma; and haemopoietic tumours, such as lymphoma, in a patient having a tumour that expresses p53 and then detecting the presence of the imaging marker by appropriate detection means.

Administration and detection of the antibody/imaging marker, as well as methods of conjugating the antibody/imaging marker, are accomplished by methods readily known or readily determined in the art. The dosage of such antibody/imaging marker will vary depending on the age and weight of the patient. Generally the dosage should be effective to visualise or detect tumour sites, distinct from normal tissues. Preferably a one-time dosage will be between about 0.1 mg to about 200mg. More preferably a one-time dosage will be between about 1 mg to about 150mg; even more preferably between about 5mg to about 100mg; even more preferably still a one-time dosage will be between about 10mg to about 50mg.

Example imaging markers include substances which can be detected by a gamma scanner or hand held gamma probe, and substances which can be detected by nuclear magnetic resonance imaging using a nuclear magnetic resonance spectrometer.

For example, the imaging marker which may be detected using a gamma scanner include imaging markers selected from the group consisting of $^{125}I$, $^{131}I$, $^{123}I$, $^{111}In$, $^{105}Rh$, $^{153}Sm$, $^{67}Cu$, $^{67}Ga$, $^{166}Ho$, $^{177}Lu$, $^{186}Re$, $^{188}Re$, and $^{99m}Tc$.

An example of an imaging marker which can be detected using a nuclear magnetic resonance spectrometer is gadolinium.

The amount of antibody (or fragment thereof) useful to produce a therapeutic effect can be determined by standard techniques well known to those of ordinary skill in the art. The antibodies will generally be provided by standard technique within a pharmaceutically acceptable buffer, and may be administered by any desired route. Because of the efficacy of the antibodies of the present invention, and their tolerance by humans it is possible to administer these antibodies repetitively in order to combat various diseases or disease states within a human.

One skilled in the art would be able, by routine experimentation, to determine what an effective, non-toxic amount of antibody (or fragment thereof) would be for the purpose of inducing immunosuppression. Generally, however, an effective dosage is expected to be in the range of about 0.05 to about 100 milligrams per kilogram body weight per day, preferably about 0.05 to about 50, more preferably about 0.5 to about 25, even more preferably about 0.5 to about 10 milligrams per kilogram body weight per day. Alternatively, an effective dosage may be up to 500mg/m$^2$. Generally, an effective dosage is expected to be in the range of about 50 to about 500mg/m$^2$, preferably about 50 to about 250mg/m$^2$, more preferably about 75 to about 250mg/m$^2$, even more preferably about 75 to about 150mg/m$^2$.

The antibodies (or fragments thereof) of this invention should also be useful for treating tumors in vertebrates More specifically, they should be useful for reducing tumor size, inhibiting tumor growth and/or prolonging the survival time of tumor-bearing vertebrates.

Accordingly, this invention also relates to a method of treating tumors in a human or other animal by administering to such human or animal an effective, non-toxic amount of an antibody or fragment thereof. One skilled in the art would be able, by routine experimentation, to determine what an effective, non-toxic amount of antibody or fragment thereof would be for the purpose of treating carcinogenic tumors. Generally, however, an effective dosage is expected to be in the range of about 0.05 to about 100 milligrams per kilogram body weight per day, preferably about 0.05 to about 50, more preferably about 0.5 to about 25, even more preferably about 0.5 about to about 10 milligrams per kilogram body weight per day. Alternatively, an effective dosage may be up to about 500mg/m$^2$. Generally, an effective dosage is expected to be in the range of about 50 to about 500mg/m$^2$, preferably about 50 to about 250mg/m$^2$, more preferably about 75 to about 250mg/m$^2$, even more preferably about 75 to about 150mg/m$^2$.

The antibodies of the invention may be administered to vertebrates, for example, humans or other animals in accordance with the above methods of treatment in an amount sufficient to produce a therapeutic or prophylactic effect.

The antibodies of the invention can be administered to such human or other animal in a conventional dosage form prepared by combining the antibody or fragment thereof of the invention with a conventional pharmaceutically acceptable carrier or diluent according to known techniques. It will be recognised by one of skill in the art that the form and character of the pharmaceutically acceptable carrier or diluent is dictated by the amount of active ingredient with which it is to be combined, the route of administration and other well-known variables.

The route of administration of the antibody (or fragment thereof) of the invention may be oral, parenteral, by inhalation or topical. The term parenteral as used herein includes intravenous, intradermal, intramuscular, subcutaneous, rectal, vaginal or intraperitoneal administration. The subcutaneous and intramuscular forms of parenteral administration are generally preferred.

The daily parenteral and oral dosage regimens for employing compounds of the invention to prophylactically or therapeutically induce immunosuppression, or to therapeutically treat carcinogenic tumors will generally be in the range of about 0.05 to about 100, preferably about 0.05 to about 50, more preferably about 0.5 to about 25, even more preferably about 0.5 to about 10 milligrams per kilogram body weight per day. Alternatively, an effective dosage may be up to about 500mg/m$^2$. Generally, an effective dosage is expected to be in the range of about 50 to about 500mg/m$^2$, preferably about 50 to about 250mg/m$^2$, more preferably about 75 to about 250mg/m$^2$, even more preferably about 75 to about 150mg/m$^2$.

The antibody or fragment thereof of the invention may also be administered by inhalation, that is, intranasal and/or oral inhalation administration. Appropriate dosage forms for such administration, such as an aerosol formulation or a metered dose inhaler, may be prepared by conventional techniques. The preferred dosage amount of a compound of the invention to be employed is generally within the range of about 0.05 to about 100, preferably about 0.05 to about 50, more preferably about 0.5 to about 25, even more preferably about 0.5 to about 10 milligrams per kilogram body weight per day. Alternatively, an effective dosage may be up to about 500mg/$m^2$. Generally, an effective dosage is expected to be in the range of about 50 to about 500mg/$m^2$, preferably about 50 to about 250mg/$m^2$, more preferably about 75 to about 250mg/$m^2$, even more preferably about 75 to about 150mg/$m^2$.

The antibody or fragment thereof of the invention may also be administered topically. By topical administration is meant non-systemic administration and includes the application of an antibody (or fragment thereof) compound of the invention externally to the epidermis, to the buccal cavity and instillation of such an antibody into the ear, eye and nose, and where it does not significantly enter the blood stream. By systemic administration is meant oral, intravenous, intraperitoneal and intramuscular administration. The amount of an antibody or fragment thereof required for therapeutic or prophylactic effect will, of course, vary with the antibody chosen, the nature and severity of the condition being treated and the animal undergoing treatment, and is ultimately at the discretion of the physician. A suitable topical dose of an antibody or fragment thereof of the invention will generally be within the range of about 1 to about 100 milligrams per kilogram body weight daily, preferably about 0.05 to about 50, more preferably about 0.5 to about 25, even more preferably about 0.5 to about 10 milligrams per kilogram body weight per day. Alternatively, an effective dosage may be up to about 500mg/$m^2$. Generally, an effective dosage is expected to be in the range of about 50 to about 500mg/$m^2$, preferably about 50 to about 250mg/$m^2$, more preferably about 75 to about 250mg/$m^2$, even more preferably about 75 to about 150mg/$m^2$.

In the administration of therapeutic formulations in accordance with the present invention and herein disclosed, there are preferred non-toxic pharmaceutical carriers, diluents, excipients and/or adjuvants. For administration of the above formulations the polypeptides to be used are admixed with these non-toxic carriers, diluents, excipients and/or adjuvants and may be in the form of capsules, aqueous or oily suspensions, emulsions, syrups, elixirs or injectable solutions.

Examples of pharmaceutically and veterinarily acceptable carriers or diluents are demineralised or distilled water; saline solution; vegetable based oils such as peanut oil, safflower oil, olive oil, cottonseed oil, maize oil, sesame oils such as peanut oil, safflower oil, olive oil, cottonseed oil, maize oil, sesame oil, arachis oil or coconut oil; silicone oils, including polysiloxanes, such as methyl polysiloxane, phenyl polysiloxane and methylphenyl polysolpoxane; volatile silicones; mineral oils such as liquid paraffin, soft paraffin or squalane; cellulose derivatives such as methyl cellulose, ethyl cellulose, carboxymethylcellulose, sodium carboxymethylcellulose or hydroxypropylmethylcellulose; lower alkanols, for example ethanol or iso-propanol; lower aralkanols; lower polyalkylene glycols or lower alkylene glycols, for example polyethylene glycol, polypropylene glycol, ethylene glycol, propylene glycol, 1,3-butylene glycol or glycerin; fatty acid esters such as isopropyl palmitate, isopropyl myristate or ethyl oleate; polyvinylpyrridone; agar; carrageenan; gum tragacanth or gum acacia, and petroleum jelly. Typically, the carrier or carriers will form from 10% to 99.9% by weight of the compositions.

Some examples of suitable carriers, diluents, excipients and adjuvants for oral use include peanut oil, liquid paraffin, sodium carboxymethylcellulose, methylcellulose, sodium alginate, gum acacia, gum tragacanth, dextrose, sucrose, sorbitol, mannitol, gelatine and lecithin. In addition these oral formulations may contain suitable flavouring and colourings agents. When used in capsule form the capsules may be coated with compounds such as glyceryl monostearate or glyceryl distearate which delay disintegration.

Adjuvants typically include emollients, emulsifiers, thickening agents, preservatives, bactericides and buffering agents.

Solid forms for oral administration may contain binders acceptable in human and veterinary pharmaceutical practice, sweeteners, disintegrating agents, diluents, flavourings, coating agents, preservatives, lubricants and/or time delay agents. Suitable binders include gum acacia, gelatine, corn starch, gum tragacanth, sodium alginate, carboxymethylcellulose or polyethylene glycol. Suitable sweeteners include sucrose, lactose, glucose, aspartame or saccharine. Suitable disintegrating agents include corn starch, methylcellulose, polyvinylpyrrolidone, guar gum, xanthan gum, bentonite, alginic acid or agar. Suitable diluents include lactose, sorbitol, mannitol, dextrose, kaolin, cellulose, calcium carbonate, calcium silicate or dicalcium phosphate. Suitable flavouring agents include peppermint oil, oil of wintergreen, cherry, orange or raspberry flavouring. Suitable coating agents include polymers or copolymers of acrylic acid and/or methacrylic acid and/or their esters, waxes, fatty alcohols, zein, shellac or gluten. Suitable preservatives include sodium benzoate, vitamin E, alpha-tocopherol, ascorbic acid, methyl paraben, propyl paraben or sodium bisulphite. Suitable lubricants include magnesium stearate, stearic acid, sodium oleate, sodium chloride or talc. Suitable time delay agents include glyceryl monostearate or glyceryl distearate.

Liquid forms for oral administration may contain, in addition to the above agents, a liquid carrier. Suitable liquid carriers include water, oils such as olive oil, peanut oil, sesame oil, sunflower oil, safflower oil, arachis oil, coconut oil, liquid paraffin, ethylene glycol, propylene glycol, polyethylene glycol, ethanol, propanol, isopropanol, glycerol, fatty alcohols, triglycerides or mixtures thereof.

Suspensions for oral administration may further comprise dispersing agents and/or suspending agents. Suitable suspending agents include sodium carboxymethylcellulose, methylcellulose, hydroxypropylmethyl-cellulose, poly-vinylpyrrolidone, sodium alginate or acetyl alcohol. Suitable dispersing agents include lecithin, polyoxyethylene esters of fatty acids such as stearic acid, polyoxyethylene sorbitol mono- or di-oleate, -stearate or -laurate, polyoxyethylene sorbitan mono- or di-oleate, -stearate or -laurate and the like.

The emulsions for oral administration may further comprise one or more emulsifying agents. Suitable emulsifying agents include dispersing agents as exemplified above or natural gums such as guar gum, gum acacia or gum tragacanth.

For administration as an injectable solution or suspension, non-toxic parenterally acceptable diluents or carriers can include, Ringer's solution, isotonic saline, phosphate buffered saline, ethanol and 1,2 propylene glycol.

Further, a vaccine composition containing the recombinant polypeptide may be prepared for use by standard methods, well known to those of ordinary skill in the art. In one embodiment, the immunogenic peptide may be produced in a recombinant system by expression of the polynucleotide sequence (or a fragment thereof) in accordance with the present invention, and subsequently isolated. For example, microbial cells containing the exogenous gene of interest may be cultured in large volume bioreactors, then collected by centrifugation and subsequently ruptured, for instance by high pressure homogenisation. The resulting cell lysate may be resuspended in appropriate diluent such as those described herein, and filtered to obtain an aqueous suspension of the immunogen. The recombinant protein can be administered in crude form, for example, by diluting in a 0.1 M phosphate buffer (pH 7.4) to 50-500 μg/ml concentration, and then passing through a sterile 0.22 micron filter.

Alternatively, a vaccine composition containing the recombinant polypeptide may be prepared in a mammalian expression system, utilising host cells such as Chinese Hamster Ovary (CHO) cells. The antibody (or fragment thereof) having binding affinity to p53 or a portion thereof may be manufactured using batch fermentation with serum free medium. After fermentation the antibody may be purified via a multistep procedure incorporating chromatography and viral inactivation/removal steps. For instance, the antibody may be first separated by Protein A affinity chromatography and then treated with solvent/detergent to inactivate any lipid enveloped viruses. Further purification, typically by anion and cation exchange chromatography may be used to remove residual proteins, solvents/detergents and nucleic acids. The purified antibody may be further purified and formulated into 0.9% saline using gel filtration columns. The formulated bulk preparation may then be sterilised and viral filtered and dispensed.

Alternatively, the antibody (or a fragment thereof) of the present invention may be used as an idiotypic immunogen. As is known to those of skill in the art, when used in this manner, the antibody (or a fragment thereof) of the present invention may function as an immunogen and elicit a second antibody (Ab2) and T cell ($T_2$) response against idiotopes of the original antibody (Ab1). Ab2 antibodies can bind to epitopes on the original antibody including the antigen binding site (idiotype). The anti-idiotypic antibody, Ab2, can spontaneously induce anti-anti-idiotypic antibodies (Ab3) as well as T cells ($T_3$) which may recognise the same epitope as Ab1. Since the first antibody binds both the p53 epitope and Ab2, Ab2 mimics the structure of the antigenic epitope (on p53). A proportion of Ab3 antibodies bind to the same epitope as the original antibody (Ab1), and may augment and prolong the efficacy of the original antibody. Induction of this anti-idiotypic network results in protection from metastases partly through the induction of p53-specific CTLs.

Alternatively, a vaccine composition containing a peptide fragment of the polypeptide of the present invention may be prepared by synthesis of a peptide, using standard methods known to those in the art, such as by automated synthesis on, for instance, an Applied Biosystems model 430A. For example, the peptide may comprise selected amino acid regions of the CDR and/or FR of the polypeptide of the invention. The synthetic peptide can be administered, for example, after diluting in a 0.1 M phosphate buffer (pH 7.4) to 50-500 μg/ml concentration, and passing through a sterile 0.22 micron filter.

Alternatively, the vaccine may be a DNA based vaccine. In one aspect, the DNA based vaccine may comprise naked DNA comprising a nucleic acid molecule as defined in the first or second embodiments of the invention, or a fragment thereof.

In another aspect, the DNA based vaccine may comprise a nucleic acid molecule as defined in the first or second embodiments of the invention, or a fragment thereof, cloned into an expression vector. Typically, the expression vector is a eucaryotic expression vector and may include expression control sequences, such as an origin of replication, a promoter, an enhancer, and necessary processing information sites, such as ribosome binding sites, RNA splice sites, polyadenylation sites, and transcriptional terminator sequences.

A typical vaccination regime is to deliver the vaccine in multiple doses generally one, two or three equal doses.

In general to induce the production of antibodies to the vaccines of the invention, they can be oleogenous or aqueous suspensions formulated in accordance with known methods in the art using suitable dispersing, suspension and/or wetting agents. Examples of suitable dispersing, suspension and wetting agents include Freund's complete/incomplete adjuvant, Montenide Marcol adjuvant and phosphate buffered saline, and mannan.

It will be appreciated that the examples referred to above are illustrative only and other suitable carriers, diluents, excipients and adjuvants known to the art may be employed without departing from the spirit of the invention.

**4. An antibody/nucleic acid based method and kit for detecting p53**

The present invention also encompasses a method of detecting a p53 polypeptide in a sample, wherein the method comprises:

(a) contacting a sample with the antibody (or fragment thereof) as defined in accordance with the sixth or seventh embodiments of the invention, and
(b) detecting the presence of the antibody (or fragment thereof) bound to a p53 polypeptide.

Typically, altered levels of p53 polypeptide may indicate the presence or onset of disease, wherein an example of such a disease is cancer.

Conditions for incubating an antibody (or fragment thereof) with a test sample vary widely, depending on the format of detection used in the assay, the detection method, and the type and nature of the antibody used. A person of ordinary skill in the art would readily appreciate that any one of the commonly available immunological assays could be used in performing the method of detection. For example, these assays include: radioimmunoassays, enzyme-linked immunosorbent assays, and/or immunoflourescent assays.

Further, the test sample used in the assay may consist of tissue, cells, protein or membrane extracts of cells, and biological fluids, such as blood, serum, plasma or urine.

A kit for performing the above method of the invention contains all the necessary reagents to carry out the above methods of detection. For example, the kit may comprise the following containers:

(a) a first container containing the antibody (or fragment thereof) of the present invention;
(b) a second container containing a conjugate comprising a binding partner of the antibody (or fragment thereof), together with a detectable label.

Typically, the kit may further comprise one or more other containers, containing other components, such as wash reagents, and other reagents capable of detecting the presence of bound antibodies. More typically, the detection reagents may include: labelled (secondary) antibodies, or where the antibody (or fragment thereof) of the present invention is itself labelled, the compartments may comprise antibody binding reagents capable of reacting with the labelled antibody (or fragment thereof) of the present invention.

Further, the kit of the present invention, as described above in relation to antibodies, can be readily incorporated, without the expenditure of inventive ingenuity, into a kit for nucleic acid probes. One skilled in the art would select the nucleic acid probe from the polynucleotides of the present invention, according to techniques known in the art as described above. Samples to be tested include but should not be limited to RNA samples of human tissue.

Such a kit comprises at least one container means having disposed therein the above-described nucleic acid probe. The kit may further comprise other containers comprising one or more of the following: wash reagents and reagents capable of detecting the presence of bound nucleic acid probe. Examples of detection reagents include, but are not limited to radiolabelled probes, enzymatic labelled probes (horseradish peroxidase, alkaline phosphatase), and affinity labelled probes (biotin, avidin, or steptavidin).

In detail, a compartmentalised kit includes any kit in which reagents are contained in separate containers. Such containers include small glass containers, plastic containers or strips of plastic or paper. Such containers allow the efficient transfer of reagents from one compartment to another compartment such that the samples and reagents are not cross-contaminated and the agents or solutions of each container can be added in a quantitative fashion from one compartment to another. Such containers will include a container which will accept the test sample, a container which contains the probe or primers used in the assay, containers which contain wash reagents (such as phosphate buffered saline, Tris-buffers, and like), and containers which contain the reagent detect the hybridised probe, bound antibody, amide product, or the like.

Furthermore, one skilled in the art would readily recognise that the nucleic acid probes in the present invention can readily be incorporated into one of the established kit formats which are known in the art.

**5. Gene Therapy**

In its simplest form, gene transfer can be performed by simply injecting minute amounts of DNA into the nucleus of a cell, through a process of microinjection. Once recombinant genes are introduced into a cell, they can be recognised by the cells normal mechanisms for transcription and translation, and a gene produce will be expressed.

Other methods have also been attempted for introducing DNA into larger numbers of cells. These methods include: transfection, wherein DNA is precipitated with $CaPO_4$ and taken into cells by pinocytosis; electroporation, wherein cells are exposed to large voltage pulses to introduce holes into the membrane; lipofection/liposome fusion, wherein DNA is packaged into lipophilic vesicles which fuse with a target cell; and particle bombardment using DNA bound to small projectiles. Another method for introducing DNA into cells is to couple the DNA to chemically modified proteins.

In one embodiment, an expression vector containing the polynucleotide sequence according to the present invention is inserted into cells, the cells are grown *in vitro* and then infused in large numbers into patients. Expression vectors derived from viruses such as retroviruses, vaccinia virus, adenovirus, adeno-associated virus, herpes viruses, several RNA viruses, or bovine papilloma virus, may be used for delivery of the polynucleotide sequences of the invention into the targeted cell population (eg., tumour cells). Methods which are well known to those skilled in the art can be used to construct recombinant viral vectors containing coding sequences. Alternatively, recombinant nucleic acid molecules encoding protein sequences can be used as naked DNA or in reconstituted system, for example, liposomes or other lipid systems for delivery to target cells.

It has also been shown that adenovirus proteins are capable of destabilising endosomes and enhancing the uptake of DNA into cells. The admixture of adenovirus to solutions containing DNA complexes, or the binding of DNA to polylysine covalently attached to adenovirus using protein crosslinking agents substantially improves the uptake and expression of the recombinant gene.

The invention will now be described in greater detail by reference to specific Examples, which should not be construed as in any way limiting on the scope thereof.

## EXAMPLES

### Example 1

### Isolation and characterisation of anti-p53 human antibodies

#### Materials & Methods

#### Patient data

After obtaining informed consent, blood and tissue samples were collected from 100 individuals seen at St Vincent's Hospital from 1993-1997 who were undergoing resection of colorectal cancer.

Clotted blood was centrifuged at 2000 g for 10 min and serum stored in aliquots at -70˚C prior to use. Samples from 50 healthy individuals were obtained and used as controls in all ELISA and immunoprecipitation experiments. A fresh pericolic lymph node in the region of the tumour was harvested from colectomy tissue and frozen in liquid nitrogen prior to RNA extraction (6).

#### Immunohistochemical detection of p53

Sections of paraffin embedded tumour tissue from each individual were subjected to immunohistochemical analysis of p53 as previously described (7). Tumour tissue was considered to have accumulated mutant p53 when the average of ten high powered fields showed greater than 5% of tumor cells with nuclear staining, in the absence of staining in the stromal cells and normal epithelium.

#### Production of recombinant p53

Recombinant p53 was expressed and purified. Briefly, a cDNA clone of wild type p53 in the expression vector pET19b was transfected into *E.coli* strain BL-21(DE3 ) (Novagene Inc. Madison, WI). Protein was purified from crude bacterial lysates using $Ni_2^+$ resin. p53 purity was assessed by polyacrylamide gel electrophoresis (PAGE) and then immunoblotting. The protein concentration was determined using the Biochonincic acid method with reference to a standard curve generated with bovine serum albumin (BSA).

#### Detection of anti-p53 serum antibodies

Wells of a microtitre plate (Polysorb, Nunc, Denmark) were coated with purified recombinant p53 (5 μg/ml in Phosphate Buffered Saline; PBS) overnight at 4˚C. Coated wells were washed three times each with 200 μl of PBS and then blocked with PBS/2% BSA for 1 hour at room temperature (RT). Patient serum samples (n=100) were diluted 1 in 100 in PBS and then applied in duplicate to the p53 and incubated for 1 hour at RT. Binding antibodies were detected with an alkaline phosphatase conjugated goat anti-human IgG Fc-specific antibody (0.5 μg/ml in PBS/2% BSA: Jackson Immuno Research Lab Inc, PA, USA) The reactivity of each patient to p53 was expressed as a value relative to a standard curve generated from control serum known to contain anti-p53 antibodies, as described previously (7). Serum activity was compared to a healthy group of volunteers (n=50) and considered positive for anti-p53 antibodies when the anti-p53 score was > 2 standard deviations above the mean of the normal group.

The isotype of antibodies in reactive sera was assessed using the above protocol, except, that the anti-human IgG Fc-specific antibody was replaced with mouse anti-human IgG (IgG1, IgG2, IgG3, and IgG4: DAKO Corp CA, USA) isotype specific antibody (1 μg/ml) and detected with an alkaline phosphatase (AP) conjugated goat anti-mouse antibody (0.5 μg/ml in PBS/2% BSA: Jackson Immuno Research Lab Inc).

The anti-p53 serum titre was defined as the lowest dilution of serum that generated a signal of 3 times above background.

**Library construction and biopanning**

Pericolic lymph nodes were ground to a fine powder in liquid nitrogen, and total RNA extracted using standard procedures (8). IgG1 kappa chain Fab libraries were constructed in the MCO1 vector as described previously (9). Briefly, immunoglobulin genes were amplified by RT-PCR using primers specific for human kappa and IgG1 immunoglobulin genes followed by digestion with Sac1/Xba1 or Spe1/Xho1 respectively. The products were then cloned sequentially (light chain then heavy chain) into the phage display phagemid vector, MCO1, and the combinatorial libraries electroporated into XL1-blue cells and packaged with helper phage to give the primary antibody phage library.

The size of the library was calculated from a proportion of clones taken after electroporation (n= 20 for each library) of the final heavy and light chain construct. A diagnostic PCR amplifying the variable region of the heavy and light chain and BstN1 finger printing (see below) were used to calculate the number of clones with unique heavy and light chain inserts. On this basis the total library size was estimated.

Wells of a microtitre plate were coated with recombinant p53 as described above, and then washed with PBS and blocked with BSA (2% v/v) /PBS. Aliquots of the phage antibody libraries ($10^{12}$ cfu in 100μl) were applied to each well and incubated at room temperature for two hours. Excess phage were washed from the plate with six washes with PBS/Tween, followed by two washes in PBS. Adherent phage were then eluted with 100 μl of 0.1 M glycine pH 3.0 for 10 min at room temperature, and neutralised with 1 M Tris pH 8. Eluted phage were reamplified for the next round of panning as described previously (6). The panning procedure was carried out five times. An aliquot was taken from the eluted output from each round of panning and used to infect the *E.coli* non suppressor strain HB2151 for the production of soluble Fab. Infected bacteria were plated onto Luria Broth agar with 50μg/ml of carbenicillin and single colonies were picked for soluble Fab production

**Analysis of soluble Fab reactivity by ELISA**

Cultures were grown overnight from a single colony at 37˚C in 2YT broth with 2% glucose (v/v) and 50μg/ml of carbenicillin (2YT/glu/carb). These were then diluted 1 in 100 in 2YT/glu/carb and grown at 37˚C until an OD of 0.8. The cultures were then centrifuged and resuspended in 2YT containing 1M IPTG and 50 μg/ml of carbenicillin and grown overnight at 30˚C. Following centrifugation, the supernatant from the overnight cultures was assessed for anti-p53 Fabs by ELISA.

Culture supernatant was applied in duplicate to ELISA plates coated with p53, and incubated for 2 hours at RT. After washing with PBS, 100 μl of the anti-myc monoclonal antibody, 9E10 was added to each well (detecting the myc tag on the C terminus of the heavy chain, 0.5 μg/ml in PBS/0.5% BSA), and incubated at room temperature for 1 hour. The wells were again washed and HRP conjugated goat anti-mouse (0.5 μg/ml in PBS/2% BSA: Jackson Immuno Research Lab Inc) antibody was added. After further washing , colour was developed with 100 μl of TMB substrate (Kirkegaard and Perry Laboratories, Gaithersburg MD) and the reaction was stopped with 50 μl of 1 M $H_2SO_4$- Clones were considered positive where the OD was more than three times the signal seen in wells not coated with p53. In each ELISA, a negative control without 9E10 was used to detect cross reactivity of Fab, secondary antibodies and p53.

Reactive anti-p53 Fabs were reanalysed using p53 coated at concentrations from 10-0.015 μg/ml, or by incubating Fab with 50 μg/ml of soluble p53 for 1 hr prior to application on the p53 coated ELISA plate (1μg/ml). To confirm that the light chain was involved in binding to p53 the ELISA was repeated using a biotinylated goat anti-human kappa specific antibody (0.2 μg/ml in PBS/2% BSA: Rockland, Gilbertsville, PA) followed by HRP conjugated streptavidin (0.05 μg/ml in PBS/2% BSA: DAKO Corp).

The cross reactivity of Fabs with other antigens was assessed by ELISA using a similar method to that described for p53. The following antigens and concentrations were used; insulin (5μg/ml), ErbB2 extracellular domain (5μg/ml; gift from Ruth Lyons, Garvan Institute, Sydney , Australia), Muc1 (5μg/ml; gift Dr Ian McKenzie, Austin Research Institute, Melbourne, Australia), and CEA (5μg/ml; extracted from tissue as described by Matsuoka et al 1991), tetanus toxoid

(1μg/ml; CSL, Melbourne, Australia), BSA (1μg/ml; Sigma-Aldrich, Castle Hill, Australia) and keyhole limpet haemocyanin (1μg/ml; Sigma-Aldrich).

**Analysis of Fab reactivity by immunoprecipitation**

The colorectal cancer cell line HT29, which contains mutant p53, was used to assess the reactivity of Fabs with human p53 from eucaryotic cells. Approximately $10^7$ cells were lysed in TNES buffer (50 mM Tris pH 7.5, 2 mM EDTA, 100 mM NaCl, 1% NP-40, protease inhibitor cocktail [Boehringer Mannheim, Castle Hill, Australia] and 1 mM PMSF) and then cell debris removed by centrifugation at 10000g for 10 min. Approximately 250 μg of the lysate was used in each immunoprecipitation. Either the mouse anti DO7 (0.5 μg; DAKO Corp) or the bacterially expressed Fab was added to the lysate and incubated for 1 hour at 4˚C. The anti-myc 9E10 antibody (1 μg) was then added to the mixture containing Fab and incubated for 1 hour at 4˚C. At this point 20 μl of (packed volume) protein A-sepharose (Zymed Laboratories Inc. San Francisco, CA) was added to all tubes and incubated for a further 1 hour at 4˚C. The protein A sepharose was washed four times with PBS, and subject to 10% PAGE under denaturing and reduced conditions. Proteins were transferred to PVDF membrane by electroblotting, blocked with 10% skim milk powder and probed with a goat anti-p53 antibody specific for the N terminal region of the protein (Santa Cruz Biotech., Santa Cruz, CA). This was followed by a donkey anti-goat-HRP antibody (Jackson Immuno Research Lab Inc), and then the blots were developed using chemiluminescent substrate (DuPont NEN, North Sydney, Australia). A negative Fab control (Fab specific for tetanus toxoid), and a Protein A sepharose and extract only control, were included in each experiment as negative controls.

**Epitope mapping**

A set of deletion mutants derived from human p53 were used in epitope mapping. The deletion mutants used were Hup53, 3M (residues 1-393), 3R (1-223), 4U (1-106), 11 (27-393) and 18 (44-393) as described by (10). Briefly cultures of *E.coli* (Bl21 DE3λ) containing the constructs were grow to OD 0.8. The cells were lysed in bacterial lysis buffer (50 mM Tris pH 7.5, 10 mM EDTA, 50 mM NaCl, 1% NP-40 and 1 mM PMSF) and 50μl of the lysate were subject to PAGE and electroblotting as described above. Bacterial expressed Fab was incubated with the membrane for 1 hour at RT and then washed with PBS. Bound Fab was detected with 9E10 and HRP-conjugated goat anti-mouse. Negative controls were as described above.

**Sequence analysis**

The variable region of selected clones was sequenced using a cycle sequencing kit according to the manufacturers specifications (Promega, Madison, WI). Miniprep DNA was prepared by alkaline lysis and both strands of DNA sequenced using primers outside the variable region. The primers used for sequencing the light chain were 5'-AA GAC AGC TAT CGC GAT T (OmpA leader sequence) and 5'-ATG AAG ACA GAT GGT GCA GC (5' end of the kappa constant region) and the heavy chain 5'-CTA CGG CAG CCG CTG GAT TG (PelB leader sequence) and 5'-GGA AGT AGT CCT TGA CCA G (5' end of the IgG CH1 region).

The heavy and light chain variable region for Fab clones was matched to available V genes, D genes and J genes using the DNA plot alignment package and V base sequence data base.

Using the method of Chang and Casali (11), the frequency of replacement mutations (R) in the CDR and framework (FR), for each of the p53 antibodies, was calculated with respect to its closest germ line gene. The probability that replacement mutations were occurring at a frequency above or below the expected random frequency was calculated in a binomial distribution model, using the expected number of R mutations in the germline gene, the actual number of observed R mutations in the Fab sequences, and the probability of R mutations localising to the CDR or FRs (11). Amino acids from 1-94 of the heavy chain and 1-95 for the light chain were used for the analysis of R mutations. Amino acid residues occurring as a result of primer sequence in the FR1 region were excluded from the analysis. A p value of less than 0.05 indicated that the R mutations had occurred in a non-random fashion.

**Fab purification**

Soluble Fab was precipitated with ammonium sulphate (35% (w/v) final), resuspended in 5 ml of PBS and then purified by IMAC affinity chromatography. Eluted fractions containing Fab were pooled and then fractionated by size exclusion chromatography (Superdex 200 Pharmacia) in HBS buffer (0.01 M HEPES pH 7.4, 0.15 M NaCl, 3 mM EDTA and 0.005% NP 40). Purity was assessed by PAGE and silver staining.

**BIAcore analysis of selected Fabs**

Recombinant p53 was coupled to a CM5 chip using standard amine immobilisation protocols. The chip was activated using 50 mM N-hydroxysuccinimide and 200 mM N-(dimethylaminopropyl)-N'-ethylcarbodimide. Recombinant p53 at 100 μg/ml in PBS diluted 1 in 10 in sodium acetate (1 M, pH 4.8) was injected at a flow rate of 10 μl/min. No greater than 400 RU were coupled to the chip for affinity analysis.

All measurements were carried out in HBS buffer. For the analysis of affinity, concentrations of Fab ranging from 10-200 nM were injected for 90 sec at a flow rate of 30 μl/min over 2 flow cells, one with coupled P53 and the other without. Dissociation was measured over 90 sec by the injection of HBS buffer. The chip was regenerated with 20 μl of 1 M glycine pH 2 at 30 μl/min flow rate. The RU of the blank flow cell was subtracted from the p53 coupled cell and the affinity constants calculated using the BIAevaluation 3 software package for a global fit.

**Results**

**Patient serum analysis and antibody library construction**

Of the 100 patients with colorectal cancer screened for antibodies against p53, 17 were found to have anti-p53 antibodies. From the patients found to have p53 reactive serum six were selected for further study, including one patient with no detectable anti-p53 antibodies as a negative control. In addition, each of the patients was assessed for the predominant IgG isotype reactive with p53. It was found that all the individuals selected had predominantly IgG1 reactive anti-p53 antibodies. IgG1k antibody libraries were therefore constructed from the pericolic lymph node tissue taken from these six colorectal cancer patients. The size of the antibody libraries from each of the constructed individuals, together with clinical data, serum and reactivity against full length p53 is shown in Table 1.

**Anti-p53 Fab selection**

Each antibody library was subjected to five rounds of panning against recombinant p53. A 20-100 fold increase in the number of eluted phage were observed in rounds 4 and 5.

No Fabs with reactivity against p53 were identified from 32 phage clones isolated from each library after each of the first three rounds of panning (total number of clones analysed = 960) The library from patient 163 was found to have 1/32 p53 reactive clones from round 4 and 42/128 p53 reactive clones from round five. No positive clones from rounds four or five (96 clones analysed from output phage) were identified from patient antibody libraries 100,107,149, 357 or 790 (192 phage clones analysed from each library).

The 43 p53 reactive clones isolated from library 163 were analysed by restriction enzyme digestion and five clones were eliminated from further analysis on the basis of lacking a heavy chain of the correct size. All clones had light chain inserts of the expected size. The remaining 38 clones were DNA fingerprinted from variable region PCR products using the frequent cutting restriction enzyme BstN1. This allowed the identification of four unique heavy chain BstN1 profiles which paired with five unique light chain profiles, giving a total of 14 clones with unique heavy and light chain combinations (results not shown). Four clones with unique heavy chain were epitope mapped and analysed for reactivity against recombinant p53, cell line derived p53, as well as for cross reactivity with other antigens (clones 163.1, 163.5, 163.17, 163.24). The nucleotide sequence of the 14 clones with unique heavy and light chain combinations was determined, the deduced amino acid sequence generated and the mutation pattern analysed.

**Conformation of anti-p53 Fab reactivity**

The reactivity of clones 163.1,5,17 and 24 with varying concentrations of p53 is shown in Figure 1. The reactivity of the Fabs against p53 was also demonstrated using the sheep anti-human kappa antibody (results not shown) involvement of both heavy and light chain in p53 binding. When pre-incubated with excess p53 prior to ELISA analysis the signal was reduced to between 11-27% of the levels observed in the standard protocol (results not shown). Furthermore, the four clones showed no reactivity against other antigens, including, CEA, erbB2, MUC-1, insulin, tetanus toxoid, KLH and BSA (Figure 2).

The ability of the Fabs to detect p53 in bacterial lysates was assessed by Western analysis (Figure 3a). The Fabs were able to detect p53 in the lysate but didn't appear to react with other proteins. In addition it was found that each of the Fabs were able to immunoprecipitate mutant p53 from the human colorectal cancer cell line HT-29 (Figure 3b).

**Epitope Mapping**

Epitope mapping of the Fab clones 163.1,5,17 and 24 showed that all were reactive with full length Hup53, and deletion constructs 3M, 3R, 4U and 11. None of the clones were reactive with the 18 construct (residues 44-393), indicating that the Fabs were reactive with an epitope between residues 27 and 44 (inclusive) of human p53.

**Affinity analysis**

The dissociation constants for the antibodies 163.1, 5, 17 and 24 were $1.19*10^{-8}$, $1.5*10^{-8}$, $1.57*10^{-8}$ and $1.38*10^{-8}$ respectively. The $\chi^2$ value were all less than 1 when using the model for 1:1 interaction with a drifting baseline.

**Sequence analysis**

For each of the 14 clones the closest germ line gene match and the percent nucleotide difference from this gene is shown in Table II. A comparison of the variable region of the 14 Fab clones showed that all the clones had greater than 95% homology with each other and appeared to have the same V gene D gene and J gene combination (Table II). The V region of these clones consisted of the V gene DP-7 (VH1-46) from the VH1 gene family, and the J gene, JH 4b. No D segment gene could be assigned to these clones with confidence due to the lack of homology with known D gene sequences, although all clones had a similar D regions. All the heavy chains of these 14 clones had extensive mutations throughout the V gene region. The percentage difference between the heavy chain V gene and the matched germ line V gene ranged from 14.6-18.5%. The mutations were frequent, not only in the CDR regions but also throughout FR1 and FR3 regions. There was relatively few mutations in the FR2 region. The light chain partners of these 14 clones had greater homology with the matched V gene than the heavy chain, with the percentage of mutations ranging from 0-5.9%. The light chain partners of these clones used the same light chain V gene DPK-24 in combination with either the JK2 or JK4 gene.

**Mutation analysis**

The deduced amino acid sequences from the 14 clones was used to deduce the replacement and silent mutations within FR and CDR regions (Figure 4), and these values used to calculate the probability that replacement mutations in FR or CDR were not random. Random mutations, either replacement or silent, occur evenly throughout a given sequence, while antigen driven responses are often localised and result in a higher or lower proportion of replacement mutations depending on the selection pressures defined by antigen selection (11). The probability that the mutations in the FR and CDR regions arose as a result of antigen driven selection is shown in Table III.

All the heavy chains derived from the DP-7 V gene, had p values less than 0.05 as a result of a lower proportion of replacement mutations in the FR than was expected. This suggests antigen-driven B cell selection, with suppression of replacement mutation in the FR. In contrast, the R mutations in the CDR region of the same clones was no greater than may be expected at random. Analysis of the matched light chain sequences showed that only clones 163.16 and 163.23 had significantly different number of R mutations. In these instances the pattern mirrored the heavy chain with negative selection occurring in the FR but no clear evidence of antigen driven selection in the CDR1 and CDR2 regions.

**Discussion**

In this study, combinatorial antibody gene libraries and phage display have been used to isolate high affinity human Fab fragments with specificity for the p53 tumour suppressor gene product. The isolated Fabs bound to the amino terminal region of p53 between residues 27 and 44, and were reactive with both recombinant p53, and with mutant p53 immunoprecipitated from colorectal cancer cells. This study represents the first report of the isolation of anti-p53 antibodies from an individual with a demonstrable antibody response to p53. As such, it provides important information on the gene usage and epitope specificity of the immune response to p53 seen in humans with malignancy. It also provides a reproducible strategy for the exploitation of these as useful immunotherapeutic agents.

Antibody phage display techniques are being increasingly used to examine the nature and specificity of the humoral immune response to a range of infectious and autoimmune diseases. The occurrence of anti-p53 antibodies in the serum of some individuals with colorectal cancer provided an opportunity to more closely examine the specificity of this response to an important tumour suppressor gene product.

In this study, libraries were constructed from pericolic lymph nodes draining a colorectal tumour, since it was considered that this tissue was more likely to represent an enriched source of anti-p53 antibodies. In order to further increase the likelihood of isolating specific Fabs, we selected individuals with a demonstrable IgG1 response to p53 protein or a portion thereof. In this regard, it is of note that all those Fabs with high affinity for p53 were derived from the individual with the highest serum antibody titre against p53, and that no antibodies were isolated from the one individual without a demonstrable serum response.

This study has, for the first time, provided an opportunity to examine the genetic structure of naturally occurring p53 antibodies, and to draw inferences from that structure regarding the nature of the immune response that produced them.

Nucleotide sequencing showed that the V genes of the p53 Fabs had undergone extensive mutation (14-18.5%), a finding that was highly unlikely to be explained on the basis of Tth (polymerase) induced errors (12). In fact, this frequency of V gene mutations is higher than that reported for class switched germinal centre and memory B cells (up to 4%), and strongly supports that the isolated antibodies reflect the occurrence of a specific antigen-driven humoral immune response in these individuals. The particularly high mutation frequency may reflect the chronic nature of antigen exposure in individuals with malignancy. While the mechanism of p53 presentation to the immune system remains uncertain, it is clear that the process can develop early in the process of tumor development. For instance, serum p53 antibodies have been reported in smokers several years prior to the detection of the malignancy (13). This suggests that antigenic p53 may be presented to the immune system throughout the course of the disease, and that this continual exposure may be responsible for the extensive somatic mutation rate in the V genes.

Statistical analysis of the frequency of replacement mutations in the V genes provides further evidence to support the contention that the isolated Fabs arose as a result of antigen-driven selection. Negative selection for the replacement mutations was seen in the framework regions of VH1 family antibodies, and their positive selection in CDR1 and 2 of clone 163.17, are typical features of affinity matured antibodies.

The structural features of the Fabs, and the inferences drawn from them, are supported by affinity analysis using surface plasmon resonance. The isolated Fabs all showed relatively high affinity for denatured p53, again suggesting that they represent the product of a specific antigen-driven immune response.

The successful isolation of stable and clonal Fabs has also allowed a closer examination of the epitope specificity of naturally occurring p53 antibodies. Fabs isolated in this study bound to residues 27-44 of p53, a region which is predominantly specific to human p53 (14). This region is particularly important as a site for interaction with transcription machinery, as well as viral proteins (15). To date, most polyclonal serum antibodies and murine monoclonals against p53 have been shown to bind to a narrow range of immunodominant epitopes that span residues in the N-terminal region (10-25, 40-50), the central region (120-130, 205-215, 285-295) and the C-terminal region (345-393). This study demonstrates that lymphocytes from individuals with cancer represent a unique and valuable source of such antibodies, and outlines strategies for the successful exploitation of this important resource.

**Example 2**

**Isolation and characterisation of anti-p53 antibodies to the central domain of p53**

**Materials & Methods**

**Purification of p53 central and carboxy terminal domain**

The central carboxy terminal domains of whole p53 (central domain, residues 95-298; carboxy terminal domain, 283-393) was amplified from wild type p53 sequence by PCR using p53 specific primers (central forward, 5'-ggccccatatgtcttctgtcccttcccag; central reverse, 5'-agtcatatgtcacagctcgtggtcaggctc; carboxy forward, 5'-gagaccatatgacagaggaacagaatctc; carboxy reverse, 5'-agtcatatgtcagtctgagtcaggccc). The PCR products were digested with Nde1 and cloned into the Nde1 site of the bacterial expression vector pET19b (Novagene). The central and carboxy terminal domains were expressed and purified as described for whole p53.

Purified central domain and carboxy terminal domain protein was used for the identification of colorectal cancer patients with central or carboxy terminal domain specific serum antibodies, the biopanning of Fab antibody, and the detection of soluble central domain reactive Fabs. The protocols to do this are essentially the same as those described in Example 1, except the central domain or carboxy terminal domain was coated onto the wells of microtitre plates at 2μg/ml in PBS.

The antigens used in the cross reactivity ELISA were coated at the same concentrations as described above (Example 1) except for the carboxy terminal domain of the p53 which was coated at 2 μg/ml in carconate buffer and early pregnancy factor (EPF) which was coated at 2 μg/ml in PBS.

**Immunoblot analysis**

Purified whole p53 and central domain run on SDS/PAGE gels were electroblotted onto PVDF membranes (0.22 μM, NEN/Dupont) for one hour at 100 V (Towbin *et al.,* 1979) and blocked for one hour in 10% skim milk powder/PBS. The primary antibodies DO7 (0.25 μg/ml in 10% skim milk powder/PBS), Pab240 (DO7 (0.25 μg/ml in 10% skim milk powder/PBS), 163.1 Fab bacterial supernatant or 1159.8 bacterial supernatant were then incubated for 1 hour at room temperature. Following this, the membrane was washed three times in PBS (10 minutes each wash), incubated for one hour with alkaline phosphatase goat anti-mouse or alkaline phosphatase goat anti human, Fab specific (0.1 μg/ml in 10% skim milk powder/PBS) and then washed three time with PBS. The membrane was equilibrated with carbonate buffer for five minutes prior to the addition of an alkaline phosphatase substrate solution (NBT [0.3 μg/ml], BCIP [0.15 μg/ml] in carbonate buffer pH 9.6).

**Results**

**Isolation of central domain reactive clones**

Five rounds of biopanning were performed against pure p53 central domain protein. The soluble Fab from clones taken from the final round of panning (n=88) were assessed for reactivity to the protein by ELISA. It was found that a single clone (1159.8) was reactive to the central domain and to whole p53 (Figure 11). Further analysis revealed that 1159.8 did not react with a range of other antigens including tumour antigens, self proteins, foreign proteins and haptens and confirms this clones specificity to central domain of p53 (Figure 12).

The ability of the clone to bind to whole p53 and the central domain was also assessed by immunoblot (Figure 13). It was shown that 1159.8 bound to whole p53 and central domain in a similar manner to the murine monoclonal antibodies DO7 and Pab 240. These murine monoclonal antibodies are specific for the amino terminal and central domains of p53 respectively. No central domain reactivity was observed with the amino terminal reactive Fab clone, 163.1, confirming the specificity of 1159.8 to this domain. The nucleotide sequence and the deduced amino acid sequence of clone 1159.8 can be found in SEQ ID No 29 and 30 for 1159.8 light chain variable sequence and 1159.8 heavy chain variable sequence, respectively.

**Example 3 - Mammalian Expression Vector**

**Construction of MCO1**

Equal concentrations (1μg) of two synthetic oligonucleotides, 99mer (sense: CT AGT GGC CAG GCC GGC CAG GAA CAA AAA CTC ATC TCA GAA GAG GAT CTG AAT GGG GCC GCA TAG TTC CCC GGG GCT GCT CAC TAT ACG CGC CAG GAG G ) and 91mer (antisense: CTG GCG CGT ATA GTG AGC AGC CCC GGG GAA CTA TGC GGC CCC ATT CAG ATC CTC TTC TGA GAT GAG TTT TTG TTC CTG GCC GGC CTG GCC A) were annealed in Sequanase reaction buffer (USB) by heating at 75˚C for 2 minutes followed by cooling to 35˚C over 1.5h. The double-stranded oligonucleotide (30 pmole) was then phosphorylated by incubating in 10mM ATP, 1x polynucleotide kinase buffer and 10U polynucleotide kinase (Boehringer Mannheim) at 27˚C for 60min. The kinase was inactivated and the DNA was phenol extracted, ethanol precipitated and resuspended in 20μl water. The double-stranded oligonucleotide was then ligated into phosphatase-treated, SpeI/BstXI digested NPC3, and the construct was transformed into electrocompetent XL1-Blue *E. coli* cells (Stratagene). Clones containing the synthetic oligonucleotide cassette were then identified by restriction enzyme analysis (MCO1 contains a *Sma*I site which is not present in NPC3), and by nucleotide sequencing (Sanger).

**Example 4**

**Construction of whole antibody**

**Materials & Methods**

The strategy for cloning and production of whole antibody is shown in Figure 5. The heavy and light chain variable regions from selected Fab clones were amplified by PCR using a touch down protocol. A typical reaction included template DNA (100 ng), 50 pmol each PCR primer (heavy chain primers, p53Vhfor- 5'-ata gtt gcg gcc gct gtg cag ctg ctc gag and p53Vhrev 5'-agt ttc act agt tga gga gac ggc; light chain primers, p53Vkfor 5'-tta cat gtc gac gcg gcc gag ctc acc and p53Vkrev:5'-ccc tgg ttc gac ctt tag ttt aga tct act gat), 1.5 mM $MgCl_2$, 200 μM dNTP's, 0.12 U Pfu polymerase in a volume of 50 μl. The conditions for the PCR were 94˚C for 4 minutes and then 2 cycles of 94˚C (denaturing) for 30s, 65˚C for 1 minute (primer annealing), 72˚C for 90 seconds (primer extension). An additional 20 cycles were then carried out under the same conditions except that the annealing step was reduced by 0.5˚C with each successive round. A final 10 PCR cycles were then performed with an annealing temperature of 55˚C.

The heavy and light PCR products were electrophoresed on 0.8% (w/v) Nusieve agarose, purified using Qiaquick DNA purification columns (Qiagen) and digested with appropriate restriction endonucleases (heavy chain, *Not* 1 and Spe 1; light chain, *Sal* 1 and *Xba* 1). The purified heavy chain was then cloned into the heavy chain expression vector pG1D105, and the light chain into the light chain expression vector pKN101 (Figure 5). The sequence of individual heavy and light chain clones was determined by cycle sequencing according to the manufacturer's specifications (Promega). Selected heavy and light chain clones were grown in XL1-blue *E.coli*, the plasmid extracted by alkaline lysis and then double purified by CsCl gradient centrifugation.

**Transfection and selection of an antibody expressing CHO cell line**

The parent cell line, CHO DG44, was seeded into 10 cm dishes at $6x10^5$ cells/dish and grown in CHO-S-SFM II (Gibco) supplemented with HT (10mM sodium hypoxanthine, 1.6 mM thymidine). The vectors containing the heavy chain (pG1D102) and light chain (pKN100) were transfected into the CHO DG44 cells using the Fugene transfection reagent (Boehringer Mannheim) according to the manufacturers specifications. In brief, equal amounts of heavy and light chain vector (1-5 μg of DNA total), were added to Fugene at a DNA:Fugene ratio of 1:3, and allowed to mix for 10 minutes at room temperature. The Fugene/DNA mix was added dropwise to the media and cells, and then incubated overnight.

Transfected cells were passaged into CHO-S-SFM-II containing methotrexate (5 nM) and G418 (500 μg/ml) and pools of transfectants which expressed antibody were subsequently plated at a cell concentration of 0.5 cells/well. The concentration of methotrexate was increased with each passage (5-50 nM) and the level of G418 was gradually removed from the culture media over the same period of time. Specific clones were expanded and then subjected to another round of cloning at 0.5 cells/well to ensure that a clonal population of cells was generated. The clones selected for the production of whole anti-p53 antibody (A1, B4) were maintained in media without G418 or methotrexate.

**Whole antibody production and purification**

Selected cell lines were cultured in cell culture and spinner flasks ($1x10^5$-$2x10^6$ cells/ml) over 5 to 10 days, the culture media and cells were then centrifuged, firstly at 1200 g to remove most cells and then again for 15 minutes at 4000 g to remove other cellular debris.

Antibody was purified from the culture supernatant using Protein A affinity chromatography. In brief, a column containing 1 ml of Protein A (BioRad AffiPrep Protein A resin) was equilibrated with 50 ml of PBS and the supernatant run over the column under gravity. The resin was then washed with 10 ml PBS under gravity. The antibody was eluted with 10 ml of 0.1 M glycine/0.2 M NaCl pH 3 and collected into 0.5 ml fractions, each of which was adjusted to pH 7 with 50 μl of 1M Tris HCl, pH 8. Purified antibody was used for all antibody characterisation.

Purified whole antibody was detected and quantified using an anti-human antibody capture ELISA. Wells of microtitre plate were coated with 100 μl of a goat anti-human IgG Fc specific antibody (1.8 μg/ml in 0.1M $NaHCO_3$/0.1% Bronidox) overnight at 4˚C. The wells were then washed with 200 μl of PBS/0.05% Tween/0.1% Bronidox three times, and blocked with PBS/1% skim milk/0.5% BSA/0.1% Bronidox for 1 hour at 37˚C. Serial dilutions of human antibody (1: 2 to 1:2000) were applied to the wells and incubated for 1 hour 37˚C. The wells were then washed three times with PBS/ 0.05% Tween/0.1% Bronidox and the wells incubated with 100 μl of a biotin conjugated anti-human Kappa (0.05 μg/ml in PBS/1% skim milk/0.5% BSA/0.1% Bronidox; Rockland, MA, USA) for 30 minutes at 37˚C. Wells were washed three times and 100 μl of alkaline phosphatase conjugated streptavidin (0.04 mg/ml in PBS/1% skim milk/0.5% BSA/0.1% Bronidox; Jackson Immuno Research Lab Inc) added to each well for 30 minutes at 37˚C. After further washing, p-nitrophenyl alkaline phosphatase substrate (1 mg/ml in carbonate buffer; Sigma) was added, the colour allowed to develop for approximately 20 minutes and the absorbance read at 410 nm. The concentration of antibody was calculated by comparing the absorbance value of the unknown to a standard curve that was generated using a positive control IgG1 antibody (The Binding Site, UK) diluted at concentrations ranging from 65-0.4 ng/ml.

**Characterisation of whole antibody**

**ELISA analysis of anti-p53 antibodies**

Purified p53 (100 μl at 2 μg/ml in PBS), the central domain of p53 (100 μl at 2μg/ml in PBS) and C-terminal domain (100μl at 2μg/ml in carbonate buffer) were coated onto wells of a microtitre plate overnight at 4˚C. Wells were washed three times with 200 μl of PBS and then 100 μl of purified antibody was applied in duplicate and incubated for 2 hours at room temperature. After washing with PBS, 100 μl of HRP conjugated goat anti-human (0.5 μg/ml in PBS/ 2% BSA: Jackson Immuno Research Lab Inc) antibody was added. After further washing, colour was developed with 100 μl of TMB substrate (Kirkegaard and Perry Laboratories, Gaithersburg MD) and the reaction was stopped with 50 μl of 1 M $H_2SO_4$. The monoclonal antibody DO7 (0.1 μg/ml) was used as a positive control under similar conditions except this antibody was detect with a HRP conjugated goat anti-mouse (0.5 μg/ml in PBS/2% BSA: Jackson Immuno Research Lab Inc).

The reactivity of p53 antibodies with other antigens was assessed by ELISA using a similar method to that described above for p53. The following antigens and concentrations were used in coating of wells; insulin (5μg/ml), erbB2 extracellular domain (2μg/ml), CEA (1 μg/ml), BSA (20 μg/ml; Sigma-Aldrich, Castlehill, Australia), keyhole limpet haemocyanin (1μg/ml ; Sigma-Aldrich), BSM (10 μg/ml;Sigma-Aldrich, Castlehill, Australia), hen egg white lysozyme (10 μg/ml;Sigma-Aldrich, Castlehill, Australia) and early pregnancy factor (10 μg/ml).

**Epitope analysis**

**Construction of p53 gene-fragment libraries**

The p53 gene was PCR amplified from the pET19b/p53 construct (7), run on a 0.8 % Nusieve gel and purified using Qiaquick DNA purification columns. The gene fragment was digested with DNase I (0.5 U/ml) and p53 gene fragments between 100-300 base pairs were gel-purified and end-repaired using $T_4$ DNA polymerase. Phosphorylated linkers FUSP (16) were blunt-end ligated to the p53 gene fragments and the ligated products were PCR amplified in a 75 μl mixture containing 12.5 μl 10 x PCR buffer (Fischer Biotech, Perth, Australia), 12.5 μl 25 mM $MgCl_2$, 20 μl 1.25 mM dNTP, 2.5 U Tth polymerase (Fischer Biotech, Perth, Australia) and 250 pmol of each of the primers: MCO234 (5'-atatccagacgctggcggtg) and MCO235 (5'-atatccagcagctggcggtg). The PCR reaction was performed using a Perkin Elmer GeneAmp PCR System 9600 starting with 94˚C for 5 minutes, followed by 35 cycles of amplification at 94˚C for 1 minutes, 60˚C for 30 s, 72˚C for 30 s and then a final incubation at 72˚C for 10 minutes. The PCR amplified fragments were gel purified, digested with *Pfl* MI (5u/μg of DNA) and were ligated to *Sfi* I (5u/μg DNA) digested fUSE5 vector DNA (17). The ligation mixture was electrotransformed into MC1061 using Bio-Rad Gene Pulser. To determine the library size, aliquots of 1 and 10 μl of the culture were plated on Luria broth agar plate containing tetracycline (20μg/ml) and streptomycin (50 μg/ml). The remaining culture was grown overnight in 400 ml 2YT supplemented with tetracycline (20μg/ml) and streptomycin (50 μg/ml). Phage were purified and titred as described by Smith (18).

**Affinity selection of p53 gene fragment libraries**

Wells of a microtitre plate were coated overnight at 4˚C with 100 μl of anti-p53 antibody (10 μg/ml) in PBS. The solution was removed, the well washed 3 times with 0.05 % Tween/PBS, and then blocked with PBS containing 0.05 % Tween and 2 % skim milk at 37˚C for 1 hour. p53 gene fragment libraries diluted to $10^{11}$ TU/100 μl in PBS containing 0.05% Tween and 2 % skim milk were added and the plate was incubated at 37˚C for 1 hour. Unbound phage were removed by washing five times in 0.05 % Tween/PBS and five times in PBS/0.5 % Tween. Bound phage were eluted for 10 minutes with 100 μl of 0.1 M glycine, pH 3.0 at room temperature and the eluate was immediately neutralised with 10 μl of 1M Tris-HCl, pH 9.5. The neutralised phage solution was used to infect 1 ml of mid-log K91 Kan culture for 15 minutes at 37˚C. The culture was incubated for another 30 minutes at 37˚C in 10 ml 2YT containing tetracycline (0.2 μg/ml). Aliquots of 100, 1 and 0.01 μl of culture were plated onto Luria broth agar plate containing tetracycline (20μg/ml) and kanamycin (100μg/ml) to determine the colony forming units of each aliquot and the after infection titre. The remaining infected culture was added to 100 ml of 2YT containing tetracycline (20μg/ml) and kanamycin (100μg/ml) and then incubated overnight with shaking to amplify the library. Phage were precipitated and were used for the second round of panning. Three rounds of panning were performed.

**Screening of positive clones by phage ELISA**

Single colonies from the outputs of each round of panning were grown overnight and phage isolated. Wells of a microtitre plate were coated with the anti-p53 antibody under the same conditions used for panning. Freshly prepared phage ($10^{10}$-$10^{11}$ cfu/ml) were incubated for 1 hour on coated and non-coated wells and then washed with 0.05 % Tween/PBS. After washing with PBS, 100 μl HRP conjugated mouse anti-M13 (0.5 μg/ml in PBS/2% BSA: Pharmacia) antibody was added and incubated for 1 hour at room temperature. After further washing, colour was developed with 100 μl of TMB substrate (Kirkegaard and Perry Laboratories, Gaithersburg MD) and the reaction was stopped with 50 μl of 1 M $H_2SO_4$.

**Immunoprecipitation**

The following cell lines were used for immunoprecipitation: HT29 (human colorectal cancer cell line with mutant p53), MCF-7 (breast cancer cell line with wild type p53) and MethA (mouse tumour cell line with mutant p53). Approximately $10^7$ cells were lysed in TNES buffer (50 mM Tris pH 7.5, 2 mM EDTA, 100 mM NaCl, 1% Nonidet P40, protease inhibitor cocktail (Boehringer Mannheim) and 1 mM PMSF), and then cell debris was removed by centrifugation at 10000 g for 10 minutes. Approximately 250 μg of total protein from the cell lysate was used in each immunoprecipitation. Either the monoclonal antibody DO7 (0.5 μg) or the whole anti-p53 antibody (approximately 1μg/ml) was added to the lysate and incubated for one hour at 4˚C. At this point 20 μl (packed volume) of protein A-sepharose (Zymed Laboratories Inc.) was added to all tubes and incubated for a further one hour at 4˚C. The protein A sepharose was washed four times with PBS, and subject to 10% SDS PAGE under denaturing and reduced conditions. Proteins were transferred to PVDF membrane by electroblotting, blocked with 10% skim milk powder and probed with a goat anti-human p53 antibody specific for the N-terminal region of the protein (0.2 μg/ml). This was followed by four washes with PBS, one hour incubation with a HRP conjugated donkey anti-goat- antibody (0.2 μg/ml) and four more washes with PBS. The blots were developed using chemiluminescence substrate (DuPont NEN). Protein A sepharose alone, and extract alone were each included in all experiments as negative controls.

**Immunohistochemistry**

4 μm paraffin sections of colorectal tumour tissue were dewaxed and rehydrated, treated with 3% (v/v) hydrogen peroxide for 5 minutes, and then microwaved in 0.01 M citrate buffer pH 6.0 for 10 minutes. The sections were then blocked with normal rabbit serum (1:5 in 2% BSA/TBS) for 20 minutes before incubating for one hour with either the whole anti-p53 antibody (C4B4) or the monoclonal antibody DO7 (both diluted to 2.5 μg/ml in 2% BSA/TBS). The bound antibody was then detected by incubating the slide for 30 minutes with horseradish peroxidase conjugated rabbit anti human or horseradish peroxidase conjugated rabbit anti-mouse (1:100 in 2% BSA/TBS), each for 30 minutes. Each incubation was followed by extensive washing in TBS. Colour was developed with 3-3'diaminebenzidine tetra-hydrochloride (0.03% in 0.003% $H_2O_2$), and sections were counterstained with haematoxylin before mounting.

**Results**

**Construction and characterisation of the whole antibody**

The variable region genes cloned into the expression vectors pKN100 and pG1D102 were sequenced and shown to have the same sequence as the parent Fab clones. CHO cells were successfully transfected with the vectors and two clones, C4B4 and C4A1 from the pools of transfectants. When analysed by ELISA, the antibodies from both clones were shown to react with whole p53 but not the central or C-terminal domains of the protein, indicating that they were reactive with the amino terminal region alone. The antibody from clone C4B4 was purified using protein A chromatography and analysed by SDS PAGE under reduced and nonreduced conditions and shown to produce a whole antibody with heavy and light chains of the correct size (Figure 6). Purified antibody was assessed by ELISA for cross reactivity with a range of antigens including haptens, self antigens and tumour antigens. It was found that the antibody C4B4 bound to whole p53 but not to the other antigens used in the assay (Figure 7). The antibody production from C4B4 was higher than that obtained from C4A1, therefore this clone was subjected to a final round of cloning to produce the clone referred to as C4B4G4.

**Immunoprecipitation**

The human cell lines HT29 and MCF-7, and the mouse cell line MethA were used to assess the reactivity of the whole antibody to p53 derived from eukaryotic cells. It was shown that C4B4 was able to immunoprecipitate wild and mutant human p53 and mutant murine p53 in the same manner as the p53 monoclonal antibody DO7 (Figure 8).

**Epitope Analysis**

The p53 gene fragment library was subjected to 3 rounds of pannning against purified C4B4 antibody. Analysis of phage clones from rounds two and three for reactivity to the C4B4 showed that 15/22 clones in round two and 19/22 from round three bound to the antibody. Sequence analysis of the reactive clones showed that they all contained the fragment of p53 containing amino acid residues from 40-54 (Figure 9). When compared to the epitope defined in experiments using deletion mutants and the parent Fab antibody 163.5, the epitope may be defined to include those residues from 40-44.

**Immunohistochemistry**

Purified C4B4G4 antibody was used to detect p53 in 4 μm sections of tumour tissue and matched normal tissue from individuals with colorectal cancer. It was demonstrated that the antibody was reactive specifically with tumour cells and that it had a nuclear staining pattern similar to the positive control antibody DO7 (Figure 10).

**Example 5 - p53 Detection Systems**

The antibody or a fragment thereof of the present invention may be used for the detection of polypeptides encoded by the p53 gene in vertebrates, in normal and in disease states. For example, the antibody may be used to capture p53 protein from patient sample in the following manner.

The anti-p53 antibody or fragment thereof, such as anti-p53 Fab, is coated onto an appropriate surface (eg., ELISA plate, Polysorb-immuno plate (NUNC, Denmark) using a solution of about 2μg/ml. This is then blocked with bovine serum albumin (BSA) at a concentration of about 2% (w/v) before the surface is washed with phosphate buffered saline (PBS). The patient sample is added and incubated for an appropriate length of time before being removed and the surface again washed with PBS. A p53 specific polyclonal antibody conjugated to a reporter molecule (eg., alkaline phosphatase, horse radish peroxidase, FITC) is then added before the surface is again washed with PBS or other buffer. A substrate (eg., 5-bromo-4-chloro-3-indolyl phosphate (BCIP) with nitro blue tetrazolium (NBT), 3,3',5,5'-tetramethylbenzidine dichloride (TMB)) appropriate for the reporter molecule is then added in order to visualise and, if necessary, quantitate bound p53 protein.

**Example 6 - Pharmaceutical Formulations**

While it is of course possible for an antibody or fragment thereof of the present invention to be administered alone, it is preferable that it be administered as a pharmaceutical formulation. The active ingredient may comprise, for topical administration, from 0.001 % to 10% by weight, eg., from 1% to 5% by weight of the formulation, although it may comprise as much as 10% by weight but preferably not in excess of 5% by weight, and more preferably from 0.1% to 1% by weight of the formulation.

The topical formulations of the present invention, comprise an active ingredient together with one or more acceptable carriers, and optionally any other therapeutic ingredients. The carriers must be "acceptable" in terms of being compatible with the other ingredients of the formulation, and not deleterious to the recipient thereof.

Formulations suitable for topical administration include liquid or semi-liquid preparations suitable for penetration through the skin to the site of where treatment is required, such as liniments, lotions, creams, ointments or pastes, and drops suitable for administration to the eye, ear or nose.

Drops according to the present invention may comprise sterile aqueous or oily solutions or suspensions. These may be prepared by dissolving the active ingredient in a aqueous solution of a bactericidal and/or fungicidal agent and/or any other suitable preservative, and optionally including a surface active agent. The resulting solution may then be clarified by filtration, transferred to a suitable container and sterilised. Sterilisation may be achieved by: autoclaving or maintaining at 90˚C-100˚C for half an hour, or by filtration, followed by transfer to a container by an aseptic technique. Examples of bactericidal and fungicidal agents suitable for inclusion in the drops are phenylmercuric nitrate or acetate (0.002%), benzalkonium chloride (0.01%) and chlorhexidine acetate (0.01%). Suitable solvents for the preparation of an oily solution include glycerol, diluted alcohol and propylene glycol.

Lotions according to the present invention include those suitable for application to the skin or eye. An eye lotion may comprise a sterile aqueous solution optionally containing a bactericide and may be prepared by methods similar to those described above in relation to the preparation of drops. Lotions or liniments for application to the skin may also include an agent to hasten drying and to cool the skin, such as an alcohol or acetone, and/or a moisturiser such as glycerol, or oil such as castor oil or arachis oil.

Creams, ointments or pastes according to the present invention are semisolid formulations of the active ingredient for external application. They may be made by mixing the active ingredient in finely-divided or powdered form, alone or in solution or suspension in an aqueous or non-aqueous fluid, with a greasy or non-greasy basis. The basis may comprise hydrocarbons such as hard, soft or liquid paraffin, glycerol, beeswax, a metallic soap; a mucilage; an oil of natural origin such as almond, corn, arachis, castor or olive oil; wool fat or its derivatives, or a fatty acid such as stearic or oleic acid together with an alcohol such as propylene glycol or macrogols.

The formulation may incorporate any suitable surface active agent such as an anionic, cationic or non-ionic surface active such as sorbitan esters or polyoxyethylene derivatives thereof. Suspending agents such as natural gums, cellulose derivatives or inorganic materials such as silicaceous silicas, and other ingredients such as lanolin, may also be included.

Further, it will be apparent to one of ordinary skill in the art that the optimal quantity and spacing of individual dosages of an antibody (or fragment thereof) of the present invention will be determined by the nature and extent ofthe condition being treated, the form, route and site of administration, and the nature of the particular vertebrate being treated. Also, such optimum conditions can be determined by conventional techniques.

It will also be apparent to one of ordinary skill in the art that the optimal course of treatment, such as, the number of doses of the antibodies (or fragments thereof) of the present invention given per day for a defined number of days, can be ascertained by those skilled in the art using conventional course of treatment determination tests.

The following are to be construed as merely illustrative examples of formulations and not as a limitation of the scope of the present invention in any way.

**Example 6(a) - Capsule Composition**

A pharmaceutical composition containing the antibody(s) (or fragments thereof) of the present invention in the form of a capsule is prepared by filling a standard two-piece hard gelatin capsule with 50 mg of an antibody (or fragment thereof) of the invention, in powdered form, 100 mg of lactose, 35 mg of talc and 10 mg of magnesium stearate.

**Example 6(b) - Injectable Parenteral Composition**

A pharmaceutical composition of this invention in a form suitable for administration by injection may be prepared by stirring 2% by weight of an antibody (or fragment thereof) of the present invention in 10% by volume propylene glycol and water. The solution is sterilised by filtration.

**Example 6(c) - Ointment Composition**

A typical composition for delivery as an ointment includes 1.0g of the antibody (or fragment thereof) of the invention, together with white soft paraffin to 100.0 g, is dispersed to produce a smooth, homogeneous product. Collapsible metal tubes are then filled with the dispersion.

**Example 6(d) - Topical Cream Composition**

A typical composition for delivery as a topical cream is outlined below:

Antibody (or fragment thereof) 1.0 g
Polawax GP 200 25.0 g
Lanolin Anhydrous 3.0 g
White Beeswax 4.5 g
Methyl hydroxybenzoate 0.1 g
Deionised & sterilised Water to 100.0 g

The polawax, beeswax and lanolin are heated together at 60˚C, a solution of methyl hydroxybenzoate is added and homogenisation is achieved using high speed stirring. The temperature is then allowed to fall to 50˚C. The antibody (or fragment thereof) of the present invention is then added and dispersed throughout, and the composition is allowed to cool with slow speed stirring.

**Example 6(e) - Topical Lotion Composition**

A typical composition for delivery as a topical lotion is outlined below:

Antibody (or fragment thereof) 1.2 g
Sorbitan Monolaurate 0.8 g
Polysorbate 20 0.7 g
Cetostearyl Alcohol 1.5 g
Glycerin 7.0 g
Methyl Hydroxybenzoate 0.4 g
Sterilised Water about to 100.00 ml

The methyl hydroxybenzoate and glycerin are dissolved in 70 ml of the water at 75˚C. The sorbitan monolaurate, polysorbate 20 and cetostearyl alcohol are melted together at 75˚C and added to the aqueous solution. The resulting emulsion is homogenised, allowed to cool with continuous stirring and the antibody (or fragment thereof) of the present invention is added as a suspension in the remaining water. The whole suspension is stirred until homogenised.

**Example 6(f) - Eye Drop Composition**

A typical composition for delivery as an eye drop is outlined below:

Antibody (or fragment thereof) 0.3 g
Methyl Hydroxybenzoate 0.005 g
Propyl Hydroxybenzoate 0.06 g
Purified Water about to 100.00 ml.

The methyl and propyl hydroxybenzoates are dissolved in 70 ml purified water at 75°C, and the resulting solution is allowed to cool. The antibody (or fragment thereof) of the invention is then added, and the solution sterilised by filtration through a membrane filter (0.022 μm pore size), and aseptically packed into sterile containers.

**Example 6(g) - Composition for Inhalation Administration (I)**

For an aerosol container with a capacity of 20-30 ml: a mixture of 10 mg of an antibody (or fragment thereof) of the present invention with 0.5-0.8% by weight of a lubricating agent, such as polysorbate 85 or oleic acid, and mixture was dispersed in a propellant, such as freon, and put into an appropriate aerosol container for either intranasal or oral inhalation administration.

**Example 6(h) - Composition for Inhalation Administration (II)**

For an aerosol container with a capacity of 20-30 ml: a mixture of 10 mg of an antibody (or fragment thereof) of the present invention in ethanol (8-10 ml), 0.1-0.2% of a lubricating agent, such as polysorbate 85 or oleic acid was added, and the mixture dispersed in a propellant, such as freon, and put into an appropriate aerosol container for either intranasal or oral inhalation administration.

**Example 6(i) - Composition for Parenteral Administration**

The antibodies (or fragments thereof) and pharmaceutical compositions of the present invention are also useful for parenteral administration, that is, subcutaneously, intramuscularly or intravenously.

The compositions for parenteral administration will commonly comprise a solution of an antibody (or fragment thereof) of the present invention or a cocktail thereof dissolved in an acceptable carrier, such as: water, buffered water, 0.4% saline, and 0.3% glycine etc, wherein such solutions are sterile and relatively free of particulate matter. These solutions are then subsequently sterilised.

The compositions may contain further pharmaceutically acceptable substances as required to approximate physiological conditions such as pH adjusting and buffering agents, etc. The concentration of the antibody (or fragment thereof) of the present invention in such a composition can vary, and will be primarily based on fluid volumes, viscosities, etc., according to the particular mode of administration selected.

Thus, a pharmaceutical composition of the present invention for intramuscular injection could be prepared to contain 1 mL sterile buffered water, and 50 mg of an antibody (or fragment thereof) of the present invention.

Similarly, a pharmaceutical composition for intravenous infusion may comprise 250 ml of sterile Ringer's solution, and 150 mg of an antibody (or fragment thereof) of the present invention. Methods for preparing parenterally administrable compositions are apparent to those skilled in the art, and are described in more detail in, for example, Remington's Pharmaceutical Science, 15th ed., Mack Publishing Company, Easton, Pa., hereby incorporated by reference herein.

Also, the antibodies (or fragments thereof) of the present invention can be lyophilised for storage and reconstituted prior to use.

Depending on the intended result, the pharmaceutical composition of the present invention can be administered for prophylactic and/or therapeutic treatments. In a therapeutic application, compositions are administered to a patient already suffering from a disease, in an amount sufficient to cure or at least partially arrest the disease and its complications. In prophylactic applications, compositions containing the antibodies (or fragments thereof) or a cocktail thereof are administered to a patient not already in a disease state to enhance the patient's resistance.

Single or multiple administrations of the pharmaceutical compositions can be carried out with dose levels and pattern being selected by the treating physician. Regardless, the pharmaceutical composition of the present invention should provide a quantity of the altered antibodies (or fragments thereof) sufficient to effectively treat the patient.

It should also be noted that the antibodies of this invention may be used for the design and synthesis of either peptide or non-peptide compounds (mimetics) which would be useful in the same therapy as the antibody (or fragment thereof).

**Table I: Clinical details, anti-p53 serum titre, and antibody library sizes of patients selected for the study**

| Patient ID | Sex | Site of the tumour | Dukes Stage | Degree of differentiation | Detection of over-expressed p53 | P53 mutation | Anti-P53 titre (IgG) | Predominant IgG isotype | Library size |
|---|---|---|---|---|---|---|---|---|---|
| 100 | M | sigmoid colon | B | Poor | yes | | 0 | NA | $1.3*10^6$ |
| 107 | F | sigmoid colon | B | Poor | yes | | 512 | IgG1 | $1.7*10^6$ |
| 149 | M | rectum | C | Moderate | yes | | 1024 | IgG1 | $1.6*10^7$ |
| 163 | M | sigmoid colon | B | Poor | yes | | 8192 | IgG1 | $4.5*10^7$ |
| 357 | F | rectum | C | Moderate | no | | 512 | IgG1 | $2.4*10^7$ |
| 790 | M | sigmoid colon | C | Moderate | yes | | 16384 | IgG1 | $3.0*10^7$ |

**Table II: The most homologous germline sequence is shown together with the number of nucleotide mutations.**

| Clone number | VH gene | D gene | J gene | Nucleotide mutations in the V region * | VK gene family | J gene | Nucleotide mutations in the V region* |
|---|---|---|---|---|---|---|---|
| 163.16 | DP-7 | ND | JH4b | 43/294 (14.6) | DPK24 | JK2 | 10/305 (3.2) |
| 163.23 | DP-7 | ND | JH4b | 43/294 (14.6) | DPK24 | JK2 | 10/305 (3.2) |
| 163.22 | DP-7 | ND | JH4b | 44/294 (15) | DPK24 | JK2 | 11/305 (3.6) |
| 163.1 | DP-7 | ND | JH4b | 44/294 (15) | DPK24 | JK2 | 11/305 (3.6) |
| 163.15 | DP-7 | ND | JH4b | 45/294 (15.3) | DPK24 | JK4 | 3/305 (1) |
| 163.20 | DP-7 | ND | JH4b | 52/294 (17.7) | DPK24 | JK2 | 14/305 (4.6) |
| 163.5 | DP-7 | ND | JH4b | 52/294 (17.7) | DPK24 | JK2 | 18/305 (5.9) |
| 163.7 | DP-7 | ND | JH4b | 51/294 (17.3) | DPK24 | JK4 | 7/305 (2.3) |
| 163.6 | DP-7 | ND | JH4b | 51/294 (17.3) | DPK24 | JK4 | 6/305 (2) |
| 163.9 | DP-7 | ND | JH4b | 50/294 (17) | DPK24 | JK2 | 14/305 (4.6) |
| 163.2 | DP-7 | ND | JH4b | 52/294 (17.7) | DPK24 | JK4 | 4/305 (1.3) |
| 163.14 | DP-7 | ND | JH4b | 52/294 (17.7) | DPK24 | JK2 | 14/305 (4.6) |
| 163.24 | DP-7 | ND | JH4b | 54/294 (18.5) | DPK24 | JK2 | 0/305 (0) |
| 163.17 | DP-7 | ND | JH4b | 54/294 (18.5) | DPK24 | JK4 | 2/294 (0.6) |
| * The number of nucleotide mutations in the V region / total number of nucleotides (%) | | | | | | | |

**Table IIIA: Variable gene mutational analysis: The total number of replacement (R) and silent (S) mutations in the FR and CDR regions 1 and 2 of each heavy chain genes.**

| Clone number | Total number of R mutations | FR R mutations (expected) | CDR R mutations (expected) | FR R:S ratio | CDR R:S ratio | p(FR) * | p(CDR)* |
|---|---|---|---|---|---|---|---|
| 163.16 | 35 | 15 (23.12) | 8 (7.13) | 15:8 | 8:2 | **0.02** | 0.13 |
| 163.23 | 35 | 15 (21.34) | 8 (6.40) | 15:8 | 8:2 | **0.02** | 0.13 |
| 163.22 | 36 | 15 (21.34) | 8 (6.58) | 15:8 | 8:3 | **0.01** | 0.13 |
| 163.1 | 36 | 15 (21.34) | 8 (6.58) | 15:8 | 8:3 | **0.01** | 0.13 |
| 163.15 | 37 | 15 (21.94) | 8 (6.76) | 15:9 | 8:3 | **0.01** | 0.14 |
| 163.20 | 38 | 16 (21.34) | 7 (6.95) | 16:9 | 7:6 | **0.01** | 0.16 |
| 163.5 | 38 | 16 (22.53) | 7 (6.9) | 16:9 | 7:6 | **0.01** | 0.16 |
| 163.17 | 39 | 16 (23.12) | 7 (7.13) | 16:10 | 7:6 | **0.01** | 0.16 |
| 163.6 | 36 | 16 (21.34) | 7 (6.58) | 16:9 | 7:6 | **0.03** | 0.16 |
| 163.9 | 37 | 16 (21.94) | 7 (6.77) | 16:8 | 7:6 | **0.02** | 0.16 |
| 163.2 | 36 | 16 (22.53) | 7 (6.58) | 16:9 | 7:6 | **0.03** | 0.16 |
| 163.14 | 36 | 16 (21.34) | 7 (6.58) | 16:9 | 7:6 | **0.03** | 0.16 |
| 163.24 | 42 | 19 (24.90) | 8 (7.68) | 19:8 | 8:5 | **0.02** | 0.15 |
| 163.17 | 39 | 16 (23.14) | 5 (7.13) | 16:9 | 7:6 | **0.01** | 0.16 |

* Shaded areas indicate clones with a non-random distribution of R mutations.

**Table IIIB: Variable gene mutational analysis: The total number of replacement (R) and silent (S) mutations in the FR and CDR regions 1 and 2 of each light chain genes.**

| Clone number | Total number of R and S mutations | FR R mutations (expected) | CDR R mutations (expected) | FR R:S ratio | CDR R:S ratio | p(FR)* | p(CDR)* |
|---|---|---|---|---|---|---|---|
| 163.16 | 6 | 0 (1.97) | 4 (1.05) | 0:2 | 4:0 | **0.04** | 0.09 |
| 163.23 | 6 | 0 (2.55) | 4 (2.05) | 0:2 | 4:0 | **0.04** | 0.09 |
| 163.22 | 7 | 1 (2.97) | 5 (2.38) | 2:0 | 5:1 | 0.07 | 0.07 |
| 163.1 | 7 | 1 (2.98) | 5 (2.38) | 1:1 | 5:1 | 0.07 | 0.07 |
| 163.15 | 2 | 0 (0.85) | 1 (0.68) | 0:0 | 1:1 | 0.33 | 0.44 |
| 163.20 | 10 | 3 (4.25) | 7 (3.41) | 3:3 | 2:2 | 0.19 | 0.19 |
| 163.5 | 13 | 6 (5.53) | 2 (4.43) | 6:3 | 2:2 | 0.21 | 0.09 |
| 163.17 | 5 | 3 (1.70) | 1 (1.36) | 1:0 | 2:2 | 0.32 | 0.30 |
| 163.6 | 4 | 1 (1.70) | 1 (1.36) | 1:0 | 1:2 | 0.32 | 0.30 |
| 163.9 | 11 | 5 (4.68) | 2 (3.75) | 5:4 | 2:0 | 0.23 | 0.15 |
| 163.2 | 2 | 0 (0.85) | 1 (0.68) | 0:0 | 1:1 | 0.33 | 0.45 |
| 163.14 | 11 | 5 (4.68) | 2 (3.75) | 5:4 | 2:0 | 0.23 | 0.15 |
| 163.24 | 0 | 0 (0) | 0 (0) | 0:0 | 0:0 | 1 | 1 |
| 163.17 | 2 | 0 (0.85) | 1 (0.68) | 1:1 | 0:0 | 0.33 | 0.44 |

* Shaded areas indicate clones with a non-random distribution of R mutations.

**Table IVA: Primers**

| Primer | Sequence | Annealing site |
|---|---|---|
| VH1a | 5'-CAG GTG CAG **CTC GAG** CAG TCT GGG-3' | 5' V FR1 of VH1 family |
| VH3a | 5'-GAG GTG CAG **CTC GAG** GAG TCT GGG-3' | 5' V FR1 of VH3 family |
| VH1f | 5'-CAG GTG CAG CTG **CTC GAG** TCT GGG-3' | 5' V FR1 of VH1 family |
| VH2f | 5'-CAG GTG CAG CTA **CTC GAG** TCG GG-3' | 5' V FR1 of VH2 family |

(continued)

| Primer | Sequence | Annealing site |
|---|---|---|
| VH3f | 5'-GAG GTG CAG CTG **CTC GAG** TCT GGG-3' | 5' V FR1 of VH3 family |
| VH4f | 5'-CAG GTG CAG CTG **CTC GAG** TCG GG-3' | 5' V FR1 of VH4 family |
| VH5f | 5'-GAG GTG CAG **CTC GAG** CAG TCT GGA-3' | 5' V FR1 of VH5 family |
| VH6f | 5'-CAG GTA CAG CTG **CTC GAG** TCA GGT CCA-3' | 5' V FR1 of VH6 family |
| CG1Z | 5'-GCA TGT **ACT AGT** TTT GTC ACA AGA TTT GGG -3' | 3' primer, γ1 hinge region (reverse transcription primer) |
| CG2Z | 5'-CGG TGG ACT AGT GAC ACA ACA TTT GCG | 3' primer, γ2 hinge region (reverse transcription primer) |
| CG3Z | 5'-TGG GCA ACT AGT GCA TGT GTG AGT TGT G | 3' primer, γ3 hinge region (reverse transcription primer) |
| CG4Z | 5'-TGG GCA ACT AGT GCA TGG GGG ACC ATA TTT GGA | 3' primer, γ4 hinge region (reverse transcription primer) |
| Primers used for the reverse transcription and amplification of human heavy chain immunoglobulin genes. Nucleic acid residues that are in bold represent restriction enzyme sites. | | |

**Table IVB: Primers**

| Primer | Sequence | Annealing site |
|---|---|---|
| VK1a | 5'-GAC ATC **GAG CTC** ACC CAG TCT CCA-3' | 5' V FR1 of Vκ1 family |
| VK2a | 5'-GAT ATT **GAG CTC** ACT CAG TCT CCA-3' | 5' V FR1 of Vκ2 family |
| VK3a | 5'-GAA ATT **GAG CTC** ACG CAG TCT CCA-3' | 5' V FR1 of Vκ3 family |
| CK1Z | 5'-GCG CCG **TCT AGA** ATT AAC ACT CTC CCC TGT TGA AGC TCT TTG TGA CGG GCG AAC TCA G-3' | 3' primer, 3' end of κ light chain (reverse transcription primer) |

(continued)

| Primer | Sequence | Annealing site |
|---|---|---|
| CL2 | 5'-cgc cgt cta gaa cta tga aca ttc tgt agg | 3' primer, 3' CL region of human lambda light chain (reverse transcription primer) |
| Vl1-2 | 5'-CAG TCT GAG CTC ACT CAG CCR CCC | FR1 of human lambda light chain VL1 and VL2 families |
| VL3 | 5'-TCC TAT GAG CTC ACT CAG | FR1 of human lambda light chain VL3 family |
| VL4-5-9 | 5'-CAG CCT GAG CTC ACT CAG | FR1 of human lambda light chain VL4, VL5 and VL9 families |
| VL6 | 5'-AAT TTT GAG CTC ACT CAG CCC | FR1 of human lambda light chain VL6 family |
| VL7 | 5'-CAG GCT GAG CTC ACT CAG GAG | FR1 of human lambda light chain VL7 family |
| VL8 | 5'-CAG ACT GAG CTC ACC CAG GAG | FR1 of human lambda light chain VL8 family |
| VL10 | 5'-CAG GCA GAG CTC ACT CAG CCA | FR1 of human lambda light chain VL10 family |

Primers used for the reverse transcription and amplification of the human k light chain immunoglobulin genes. Nucleic acid residues in bold represent restriction enzyme sites.

**Table V: Identification of individual sequences**

| SEQ ID No | Clone | Type | Light or Heavy Chain |
|---|---|---|---|
| 1 | 163.1 | DNA | Light |
| 2 | 163.1 | DNA | Heavy |
| 3 | 163.2 | DNA | Light |
| 4 | 163.2 | DNA | Heavy |
| 5 | 163.5 | DNA | Light |
| 6 | 163.5 | DNA | Heavy |
| 7 | 163.6 | DNA | Light |
| 8 | 163.6 | DNA | Heavy |
| 9 | 163.7 | DNA | Light |
| 10 | 163.7 | DNA | Heavy |
| 11 | 163.9 | DNA | Light |
| 12 | 163.9 | DNA | Heavy |
| 13 | 163.14 | DNA | Light |
| 14 | 163.14 | DNA | Heavy |
| 15 | 163.15 | DNA | Light |
| 16 | 163.15 | DNA | Heavy |
| 17 | 163.16 | DNA | Light |
| 18 | 163.16 | DNA | Heavy |
| 19 | 163.17 | DNA | Light |
| 20 | 163.17 | DNA | Heavy |
| 21 | 163.20 | DNA | Light |
| 22 | 163.20 | DNA | Heavy |
| 23 | 163.22 | DNA | Light |

(continued)

| SEQ ID No | Clone | Type | Light or Heavy Chain |
|---|---|---|---|
| 24 | 163.22 | DNA | Heavy |
| 25 | 163.23 | DNA | Light |
| 26 | 163.23 | DNA | Heavy |
| 27 | 163.24 | DNA | Light |
| 28 | 163.24 | DNA | Heavy |
| 29 | 1159.8 | DNA | Light |
| 30 | 1159.8 | DNA | Heavy |
| 31 | 163.1 | Amino acid | Light |
| 32 | 163.1 | Amino acid | Heavy |
| 33 | 163.2 | Amino acid | Light |
| 34 | 163.2 | Amino acid | Heavy |
| 35 | 163.5 | Amino acid | Light |
| 36 | 163.5 | Amino acid | Heavy |
| 37 | 163.6 | Amino acid | Light |
| 38 | 163.6 | Amino acid | Heavy |
| 39 | 163.7 | Amino acid | Light |
| 40 | 163.7 | Amino acid | Heavy |
| 41 | 163.9 | Amino acid | Light |
| 42 | 163.9 | Amino acid | Heavy |
| 43 | 163.14 | Amino acid | Light |
| 44 | 163.14 | Amino acid | Heavy |
| 45 | 163.15 | Amino acid | Light |
| 46 | 163.15 | Amino acid | Heavy |
| 47 | 163.16 | Amino acid | Light |
| 48 | 163.16 | Amino acid | Heavy |
| 49 | 163.17 | Amino acid | Light |
| 50 | 163.17 | Amino acid | Heavy |
| 51 | 163.20 | Amino acid | Light |
| 52 | 163.20 | Amino acid | Heavy |
| 53 | 163.22 | Amino acid | Light |
| 54 | 163.22 | Amino acid | Heavy |
| 55 | 163.23 | Amino acid | Light |
| 56 | 163.23 | Amino acid | Heavy |
| 57 | 163.24 | Amino acid | Light |
| 58 | 163.24 | Amino acid | Heavy |
| 59 | 1159.8 | Amino acid | Light |
| 60 | 1159.8 | Amino acid | Heavy |

**References**

1. Hollstein, M., D. Sidransky, B. Vogelstein, C. C. Harris. 1991. p53 mutations in human cancers. Science. 253: 49 .
2. Pavletich, N. P., K. A. Chambers, C. O. Pabo. 1993. The DNA-binding domain of p53 contains the four conserved regions and the major mutation hot spots. Genes & Development. 7: 2556.
3. Vogelstein, B., K. W. Kinzler. 1992. p53 function and dysfunction. Cell. 70: 523 .
4. Winter, S. F., J. D. Minna, B. E. Johnson, T. Takahashi, A. F. Gazdar, D. P. Carbone. 1992. Development of antibodies against p53 in lung cancer patients appears to be dependent on the type of p53 mutation. Cancer Research. 52: 4168.
5. Abrams, P. G., J. L. Rossio, H. C. Stevenson, K. A. Foon. 1986. Optimal strategies for developing human-human monoclonal antibodies. Methods in Enzymology. 121: 107.
6. Clark, M. A., N. J. Hawkins, A. Papaioannou, R. J. Fiddes, R. L. Ward. 1997. Isolation of Human Anti-C-Erbb-2 Fabs From a Lymph Node-Derived Phage Display Library. Clinical & Experimental Immunology. 109: 166 .
7. Coomber, D., N. J. Hawkins, M. Clark, A. Meagher, R. L. Ward. 1996. Characterisation and Clinicopathological Correlates of Serum Anti-P53 Antibodies in Breast and Colon Cancer. Journal of Cancer Research & Clinical Oncology. 122: 757.
8. Chomczynski, P., N. Sacchi. 1987. Single-step method of RNA isolation by acid guanidinium thiocyanate-phenol-chloroform extraction. Analytical Biochemistry. 162: 156 .
9. Ward, R. L., M. A. Clark, J. Lees, N. J. Hawkins. 1996. Retrieval of Human Antibodies From Phage-Display Libraries Using Enzymatic Cleavage. Journal of Immunological Methods. 189: 73.
10. Nissim, A., H. R. Hoogenboom, I. M. Tomlinson, G. Flynn, C. Midgley, D. Lane, G. Winter. 1994. Antibody fragments from a 'single pot' phage display library as immunochemical reagents. Embo Journal. 13: 692 .
11. Chang, B., P. Casali. 1994. The CDR1 sequences of a major proportion of human germline Ig VH genes are inherently susceptible to amino acid replacement. Immunol Today. 15: 367.
12. Hengen, P. N. 1995. Fidelity of DNA polymerases for PCR. Trends in Biochemical Sciences. 20: 324 .
13. Lubin, R., B. Schlichtholz, D. Bengoufa, G. Zalcman, J. Tredaniel, A. Hirsch, C. C. de Fromentel, C. Preudhomme, P. Fenaux, G. Fournier, et al. 1993. Analysis of p53 antibodies in patients with various cancers define B-cell epitopes of human p53: distribution on primary structure and exposure on protein surface. Cancer Research. 53: 5872.
14. Soussi, T., P. May. 1996. Structural aspects of the p53 protein in relation to gene evolution: a second look. J Mol Biol. 260: 623 .
15. Ko, L. J., C. Prives. 1996. p53: puzzle and paradigm. Genes Dev. 10: 1054.
16. Nagesha H. S., M. Yu and L. F. Wang, 1996, Application of linker-ligation-PCR for construction of phage display epitope libraries, Journal of Biological Methods, 60, 147-54
17. Petersen G., D. Song, B. Hugle-Dorr, I. Oldenburg and E. K. Bautz, 1995, Mapping of linear epitopes recognized by monoclonal antibodies with gene-fragment phage display libraries, Mol Gen Genet, 249, 425-31
18. Smith G. P., 1985, Filamentous fusion phage: novel expression vectors that display cloned antigens on the virion surface, Science, 228, 1315-7

## Claims

1. An isolated and purified nucleic acid sequence comprising a polynucleotide sequence encoding a polypeptide of an antibody (or fragment thereof), wherein said antibody (or fragment thereof) has binding affinity to a p53 protein or a portion thereof in vertebrates, and wherein said nucleic acid sequence is obtained from a vertebrate host expressing an immune response against a naturally-occurring disease.

2. A nucleic acid sequence according to claim 1, wherein said immune response is **characterised by** expression of at least one p53 antibody.

3. A nucleic acid sequence according to claim 1 or claim 2 comprising a polynucleotide sequence encoding an $F_{ab}$ antibody fragment (or fragment thereof) having binding affinity to a p53 protein or a portion thereof in vertebrates.

4. An isolated and purified nucleic acid sequence comprising a polynucleotide sequence selected from the group consisting of SEQ ID Nos 1-30.

5. A nucleic acid sequence according to any one of claims 1 to 4, wherein the nucleic acid sequence is DNA.

6. A nucleic acid sequence according to any one of claims 1 to 4, wherein the nucleic acid sequence is RNA.

**7.** A nucleic acid sequence according to any one of claims 1 to 6, wherein the nucleic acid sequence comprises a polynucleotide sequence(s), or an analogue thereof, encoding an antibody fragment or other immunologically active fragment thereof, wherein the antibody (or fragment thereof) has binding affinity to a p53 protein or a portion thereof in vertebrates.

**8.** A nucleic acid sequence according to claim 7, wherein the antibody fragment or other immunologically active fragment comprises at least one complementarity determining region.

**9.** A nucleic acid sequence according to claim 7 or claim 8, wherein the antibody fragment comprises at least one functional antigen-binding domain.

**10.** A nucleic acid sequence according to any one of claims 7 to 9, wherein the antibody fragment is selected from the group consisting of: Fv, $F_{ab}$, $F(ab)_2$, scFv (single chain Fv), dAb (single domain antibody), bi-specific antibodies, diabodies and triabodies.

**11.** A nucleic acid sequence according to any one of claims 1 to 10, wherein the antibody (or fragment thereof) has binding affinity for residues of one or more of the N-terminus, the C-terminus or the central domain of a p53 protein or a portion thereof.

**12.** A nucleic acid sequence according to any one of claims 1 to 11, wherein the antibody (or fragment thereof) has binding affinity for residues of the N-terminus of a p53 protein or a portion thereof.

**13.** A nucleic acid sequence according to any one of claims 1 to 12, wherein the antibody (or fragment thereof) has binding affinity for residues about 10 to about 55 of the N-terminus of a p53 protein or portion thereof.

**14.** A nucleic acid sequence according to any one of claims 1 to 12, wherein the antibody (or fragment thereof) has binding affinity for residues about 10 to about 25 of the N-terminus of a p53 protein or portion thereof.

**15.** A nucleic acid sequence according to any one of claims 1 to 12, wherein the antibody (or fragment thereof)has binding affinity for residues about 40 to about 50 of the N-terminus of a p53 protein or portion thereof.

**16.** A nucleic acid sequence according to any one of claims 1 to 12, wherein the antibody (or fragment thereof) has binding affinity for residues about 27 to about 44 of the N-terminus of a p53 protein or portion thereof.

**17.** A nucleic acid sequence according to any one of claims 1 to 12, wherein the antibody (or fragment thereof) has binding affinity for residues about 40 to about 44 of the N-terminus of a p53 protein or portion thereof.

**18.** A nucleic acid sequence according to any one of claims 1 to 11, wherein the antibody (or fragment thereof) has binding affinity for residues of the central domain of a p53 protein or a portion thereof.

**19.** A nucleic acid sequence according to any one of claims 1 to 18, wherein said sequence comprises a polynucleotide sequence encoding a polypeptide of an antibody (or fragment thereof) having binding affinity to a p53 protein or a portion thereof in vertebrates, wherein said polynucleotide sequence encodes an immunoglobulin light chain variable region polypeptide or an immunoglobulin heavy chain variable region polypeptide.

**20.** A nucleic acid sequence according to any one of claims 1 to 19, wherein said sequence comprises a polynucleotide sequence encoding a polypeptide of an antibody (or fragment thereof) having binding affinity to a p53 protein or a portion thereof in vertebrates, wherein said nucleic acid sequence comprises a first polynucleotide sequence encoding an immunoglobulin light chain variable region polypeptide, and a second polynucleotide sequence encoding an immunoglobulin heavy chain variable region polypeptide.

**21.** A nucleic acid sequence according to any one of claims 1 to 20, wherein the vertebrate is selected from the group consisting of human, non-human primate, murine, bovine, ovine, equine, caprine, leporine, avian, feline and canine.

**22.** A nucleic acid sequence according to any one of claims 1 to 21, wherein the vertebrate is human.

**23.** An isolated and purified nucleic acid sequence comprising an analogue of the nucleic acid sequence according to any one of claims 1 to 22, wherein said analogue encodes a polypeptide having a biological activity which is functionally the same as the polypeptide(s) encoded by said polynucleotide sequence.

**24.** A nucleic acid sequence according to any one of claims 1 to 23, wherein the disease is selected from the group consisting of cancer, rheumatoid arthritis and coronary heart disease.

**25.** A nucleic acid sequence according to claim 24, wherein the disease is cancer.

**26.** A nucleic acid sequence according to claim 25, wherein the cancer is selected from the group consisting of carcinogenic tumours; tumours of epithelial origin, such as colo-rectal cancer, breast cancer, lung cancer, head and neck tumours, hepatic cancer, pancreatic cancer, ovarian cancer, gastric cancer, brain cancer, bladder cancer, prostate cancer and urinary/genital tract cancer, oesophageal cancer; mesenchymal tumours, such as sarcoma; and haemopoietic tumours, such as B cell lymphoma.

**27.** A polypeptide of an antibody (or fragment thereof) having binding affinity to a p53 protein or a portion thereof in vertebrates, wherein said polypeptide is obtained from a vertebrate host expressing an immune response against a naturally-occurring disease.

**28.** A polypeptide according to claim 27, wherein said immune response is **characterised by** expression of at least one p53 antibody.

**29.** An isolated and purified polypeptide, wherein said polypeptide is encoded by the nucleic acid sequence according to any one of claims 1 to 26.

**30.** An isolated and purified polypeptide comprising an amino acid sequence selected from the group consisting of SEQ ID Nos 31-60.

**31.** A polypeptide according to any one of claims 27 to 30, wherein said polypeptide is selected from the group consisting of: Fv, $F_{ab}$, $F(ab)_2$, scFv (single chain Fv), dAb (single domain antibody), bi-specific antibodies, diabodies and triabodies.

**32.** A polypeptide according to any one of claims 27 to 31, wherein said polypeptide has binding affinity to a p53 protein or a portion thereof.

**33.** A polypeptide according to any one of claims 27 to 32, wherein said polypeptide has binding affinity for residues of one or more of the N-terminus, the C-terminus or the central domain of a p53 protein or a portion thereof.

**34.** A polypeptide according to any one of claims 27 to 33, wherein said polypeptide has binding affinity for residues of the N-terminus of a p53 protein or a portion thereof.

**35.** A polypeptide according to any one of claims 27 to 34, wherein said polypeptide has binding affinity for residues about 10 to about 55 of the N-terminus of a p53 protein or portion thereof.

**36.** A polypeptide according to any one of claims 27 to 34, wherein said polypeptide has binding affinity for residues about 10 to about 25 of the N-terminus of a p53 protein or portion thereof.

**37.** A polypeptide according to any one of claims 27 to 34, wherein said polypeptide has binding affinity for residues about 40 to about 50 of the N-terminus of a p53 protein or portion thereof.

**38.** A polypeptide according to any one of claims 27 to 34, wherein said polypeptide has binding affinity for residues about 27 to about 44 of the N-terminus of a p53 protein or portion thereof.

**39.** A polypeptide according to any one of claims 27 to 34, wherein said polypeptide has binding affinity for residues about 40 to about 44 of the N-terminus of a p53 protein or portion thereof.

**40.** A polypeptide according to any one of claims 27 to 33, wherein said polypeptide has binding affinity for residues of the central domain of a p53 protein or a portion thereof.

**41.** An isolated and purified polypeptide, wherein said polypeptide is a homologous polypeptide of the polypeptide according to any one of claims 27 to 40.

**42.** A polypeptide according to claim 41, wherein said polypeptide is at least 45% homologous to the polypeptide according to any one of claims 27 to 40.

**43.** A polypeptide according to claim 41, wherein said polypeptide is at least 75% homologous to the polypeptide according to any one of claims 27 to 40.

**44.** A polypeptide according to claim 41, wherein said polypeptide is at least 95-99% homologous to the polypeptide according to any one of claims 27 to 40.

**45.** A polypeptide according to any one of claims 27 to 44, wherein the disease is selected from the group consisting of cancer, rheumatoid arthritis and coronary heart disease.

**46.** A polypeptide according to claim 45, wherein the disease is cancer.

**47.** A polypeptide according to claim 46, wherein the cancer is selected from the group consisting of carcinogenic tumours; tumours of epithelial origin, such as colo-rectal cancer, breast cancer, lung cancer, head and neck tumours, hepatic cancer, pancreatic cancer, ovarian cancer, gastric cancer, brain cancer, bladder cancer, prostate cancer and urinary/genital tract cancer, oesophageal cancer; mesenchymal tumours, such as sarcoma; and haemopoietic tumours, such as B cell lymphoma.

**48.** A peptide fragment of the polypeptide of any one of SEQ ID Nos 31-60, wherein said peptide fragment induces an immune response when administered to a vertebrate.

**49.** A peptide fragment according to claim 48, wherein said peptide fragment comprises between about 5 and about 50 contiguous amino acids of any one of SEQ ID Nos 31-60.

**50.** A peptide fragment according to any one of claims 48 to 49, wherein said peptide fragment comprises between about 5 and about 30 contiguous amino acids of any one of SEQ ID Nos 31-60.

**51.** A peptide fragment according to any one of claims 48 to 50, wherein said peptide fragment comprises between about 8 and about 20 contiguous amino acids of any one of SEQ ID Nos 31-60.

**52.** A peptide fragment according to claim 48, wherein said peptide fragment is derived from the complementarity determining region.

**53.** A peptide fragment according to any one of claims 48 to 52, wherein said immune response is an idiotypic response.

**54.** A peptide fragment according to any one of claims 48 to 53, wherein the vertebrate is human.

**55.** An antibody or fragment thereof, wherein said antibody or fragment thereof has binding affinity to a p53 protein or a portion thereof in vertebrates, and wherein said antibody is obtained from a vertebrate host expressing an immune response against a naturally-occurring disease.

**56.** An antibody or fragment thereof according to claim 55, wherein said immune response is **characterised by** expression of a p53 antibody.

**57.** An antibody, or fragment thereof, having binding affinity to a p53 protein or a portion thereof in vertebrates, wherein said antibody or fragment thereof is comprised of the polypeptide according to any one of claims 27 to 47.

**58.** An antibody, or fragment thereof, having binding affinity to a p53 protein or a portion thereof in vertebrates, wherein said antibody or fragment thereof is encoded by the nucleic acid sequence according to any one of claims 1 to 26.

**59.** An antibody fragment according to any one of claims 55 to 58, wherein said fragment is an immunologically active fragment.

**60.** An antibody fragment according to any one of claims 55 to 59, wherein said fragment comprises at least one complementarity determining region.

61. An antibody fragment according to any one of claims 55 to 60, wherein said fragment is selected from the group consisting of: Fv, $F_{ab}$, $F(ab)_2$, scFv (single chain Fv), dAb (single domain antibody), bi-specific antibodies, diabodies and triabodies.

62. An antibody, or fragment thereof, according to any one of claims 55 to 61, which is a polyclonal antibody.

63. An antibody, or fragment thereof, according to any one of claims 55 to 61, which is a monoclonal antibody.

64. An antibody or fragment thereof according to any one of claims 57 to 63, wherein said antibody or fragment thereof has binding affinity for residues of one or more of the N-terminus, the C-terminus or the central domain of a p53 protein or a portion thereof.

65. An antibody or fragment thereof according to any one of claims 57 to 64, wherein said antibody or fragment thereof has binding affinity for residues of the N-terminus of a p53 protein or a portion thereof.

66. An antibody or fragment thereof according to any one of claims 57 to 65, wherein said antibody or fragment thereof has binding affinity for residues about 10 to about 55 of the N-terminus of a p53 protein or portion thereof.

67. An antibody or fragment thereof according to any one of claims 57 to 65, wherein said antibody or fragment thereof has binding affinity for residues about 10 to about 25 of the N-terminus of a p53 protein or portion thereof.

68. An antibody or fragment thereof according to any one of claims 57 to 65, wherein said antibody or fragment thereof has binding affinity for residues about 40 to about 50 of the N-terminus of a p53 protein or portion thereof.

69. An antibody or fragment thereof according to any one of claims 57 to 65, wherein said antibody or fragment thereof has binding affinity for residues about 27 to about 44 of the N-terminus of a p53 protein or portion thereof.

70. An antibody or fragment thereof according to any one of claims 57 to 65, wherein said antibody or fragment thereof has binding affinity for residues about 40 to about 44 of the N-terminus of a p53 protein or portion thereof.

71. An antibody or fragment thereof according to any one of claims 57 to 64, wherein said antibody or fragment thereof has binding affinity for residues of the central domain of a p53 protein or a portion thereof.

72. An antibody or fragment thereof according to any one of claims 55 to 71, wherein the disease is selected from the group consisting of cancer, rheumatoid arthritis and coronary heart disease.

73. An antibody or fragment thereof according to claim 72, wherein the disease is cancer.

74. An antibody or fragment thereof according to claim 73, wherein the cancer is selected from the group consisting of carcinogenic tumours; tumours of epithelial origin, such as colo-rectal cancer, breast cancer, lung cancer, head and neck tumours, hepatic cancer, pancreatic cancer, ovarian cancer, gastric cancer, brain cancer, bladder cancer, prostate cancer and urinary/genital tract cancer, oesophageal cancer; mesenchymal tumours, such as sarcoma; and haemopoietic tumours, such as B cell lymphoma.

75. A vector comprising the nucleic acid sequence according to any one of claims 1 to 26.

76. A vector according to claim 75, wherein said vector is selected from the group consisting of viral, plasmid, bacteriophage, phagemid, cosmid, bacterial artificial chromosome, and yeast artificial chromosome.

77. A vector according to claim 76, wherein said bacteriophage is selected from the group consisting of λgt10 and λgt11 and phage display vectors.

78. A vector according to claim 77, wherein said phage display vector is selected from vectors derived from pCOMB vectors.

79. A vector according to claim 76 or 77, wherein said phage display vector is of the MCO group.

**80.** A vector according to any one of claims 77 to 79, wherein said phage display vector is selected from the group consisting of MCO1, MCO3 and MCO6 vectors.

**81.** A vector according to any one of claims 77 to 80, wherein said phage display vector is MCO3.

**82.** A vector according to claim 75, wherein said vector is a mammalian expression vector.

**83.** A vector according to claim 82, wherein said mammalian expression vector is pG1D102-MCO or pKN100-MCO.

**84.** A host cell transformed with the vector according to any one of claims 75 to 83.

**85.** A host cell according to claim 84, wherein said host cell is selected from the group consisting of *E. coli, Bacillus, Streptomyces, Pseudomonas, Salmonella,* and *Serratia.*

**86.** A host cell according to claim 84, wherein said host cell is selected from the group consisting of yeast, fungi, plant, insect cells and mammalian cells.

**87.** A host cell according to claim 86, wherein said mammalian cells are selected from the group consisting of CHO cell lines, COS cell lines, HeLa cells, L cells, murine 3T3 cells, c6 glioma cells and myeloma cell lines.

**88.** A host cell according to claim 86 or claim 87, wherein said mammalian cells are CHO DG44 cells.

**89.** A non-human vertebrate comprising a host cell according to any one of claims 84 to 88.

**90.** A pharmaceutical composition comprising the polypeptide according to any one of claims 27 to 47, or a peptide fragment according to any one of claims 48 to 54, or an antibody or fragment thereof according to any one of claims 55 to 74, together with a pharmaceutically acceptable carrier, adjuvant and/or diluent.

**91.** A pharmaceutical composition according to claim 90, wherein said polypeptide is in a form selected from the group consisting of polypeptide/chelate, polypeptide/drug, polypeptide/prodrug, polypeptide/toxin, polypeptide/imaging marker, antibody/chelate, antibody/drug, antibody/prodrug, antibody/toxin and antibody/imaging marker.

**92.** A pharmaceutical composition according to claim 91, wherein said chelate is selected from the group consisting of: $^{90}$Y, $^{131}$I and $^{188}$Re.

**93.** A pharmaceutical composition according to claim 91, wherein said drug is a cytotoxic drug.

**94.** A pharmaceutical composition according to claim 93, wherein said cytotoxic drug is selected from the group consisting of adriamycin, melphalan, cisplatin, taxol, fluorouricil, cyclophosphamide

**95.** A pharmaceutical composition according to claim 91, wherein said prodrug is an antibody directed prodrug therapy or ADEPT.

**96.** A pharmaceutical composition according to claim 91, wherein said toxin is selected from the group consisting of ricin, abrin, *Diptheria* toxin and *Pseudomonas* endotoxin (PE 40).

**97.** A pharmaceutical composition according to claim 91, wherein said imaging marker is selected from the group consisting of $^{125}$I $^{131}$I $^{123}$I, $^{111}$In, Rh, $^{153}$Sm, $^{67}$Cu, $^{67}$Ga, $^{166}$Ho, $^{177}$Lu, $^{186}$Re, $^{188}$Re, and $^{99m}$Tc.

**98.** A pharmaceutical composition according to claim 91, wherein said imaging marker is gadolinium.

**99.** A vaccine comprising a nucleic acid sequence according to any one of claims 1 to 26, or a fragment thereof, or a polypeptide according to any one of claims 27 to 47, or a peptide fragment according to any one of claims 48 to 54, or an antibody or fragment thereof according to any one of claims 55 to 74, together with a pharmaceutically acceptable carrier, adjuvant and/or diluent.

**100.** A vaccine according to claim 99, wherein said vaccine is an idiotypic vaccine.

**101.** A vaccine according to claim 99 or claim 100, wherein said vaccine is formulated for administration via an oral, inhalation, topical or parenteral route.

**102.** A method for inducing an immune response against disease in a vertebrate, comprising administering to said vertebrate an immunologically effective amount of the polypeptide (or peptide fragment thereof) according to any one of claims 27 to 47, or a peptide fragment according to any one of claims 48 to 54, or an antibody (or fragment thereof) according to any one of claims 55 to 74, or a pharmaceutical composition according to any one of claims 90 to 98, or a vaccine according to any one of claims 99 to 101.

**103.** The method according to claim 102, wherein the polypeptide, peptide fragment, or antibody (or fragment thereof) is administered together with a pharmaceutically acceptable carrier, adjuvant and/or diluent.

**104.** A method for the treatment and/or prophylaxis of disease in a vertebrate in need of said treatment and/or prophylaxis, wherein said method comprises administering to said vertebrate a therapeutically effective amount of the polypeptide (or peptide fragment thereof) according to any one of claims 27 to 47, or the peptide fragment according to any one of claims 48 to 54, or an antibody (or fragment thereof) according to any one of claims 55 to 74, or a pharmaceutical composition according to any one of claims 90 to 98, or a vaccine according to any one of claims 99 to 101.

**105.** The method according to any one of claims 102 to 104, wherein the disease is selected from the group consisting of cancer, rheumatoid arthritis and coronary heart disease.

**106.** The method according to any one of claims 102 to 105, wherein the disease is cancer.

**107.** The method according to claim 106, wherein the cancer is selected from the group consisting of carcinogenic tumours; tumours of epithelial origin, such as colo-rectal cancer, breast cancer, lung cancer, head and neck tumours, hepatic cancer, pancreatic cancer, ovarian cancer, gastric cancer, brain cancer, bladder cancer, prostate cancer and urinary/genital tract cancer, oesophageal cancer; mesenchymal tumours, such as sarcoma; and haemopoietic tumours, such as B cell lymphoma.

**108.** A diagnostic kit for the detection of polypeptides encoded by the p53 gene in vertebrates, said kit comprising the antibody (or fragment thereof) according to any one of claims 55 to 74, together with a diagnostically acceptable carrier and/or diluent.

**109.** A diagnostic kit according to claim 108, wherein said kit comprises:

(a) a first container containing the antibody (or fragment thereof) according to any one of claims 55 to 74, and;
(b) a second container containing a conjugate comprising a binding partner of the antibody (or fragment thereof), together with a detectable label.

**110.** A method for screening for a disease in a vertebrate comprising:

(a) contacting a sample from a vertebrate with a nucleic acid probe comprising a nucleic acid sequence according to any one of claims 1 to 26, or an oligonucleotide fragment thereof, and
(b) detecting hybridisation between the nucleic acid sample and the polynucleotide sequence.

**111.** A method according to claim 110, wherein the oligonucleotide fragment is between about 10 to about 100 nucleotides in length.

**112.** A method according to claim 110 or claim 111, wherein the oligonucleotide fragment is between about 15 to about 30 nucleotides in length.

**113.** The method according to any one of claims 110 to 112, wherein hybridisation as compared to non-hybridisation is indicative of disease.

**114.** The method according to any one of claims 110 to 113, wherein said disease is cancer.

**115.** The method according to any one of claims 110 to 114, wherein hybridisation is conducted under low, moderate, or high stringency.

**116.** The method according to any one of claims 110 to 115, wherein hybridisation is conducted under high stringency.

**117.** A method for screening for a disease in a vertebrate comprising:

(a) contacting a sample from a vertebrate with the antibody (or fragment thereof) according to any one of claims 55 to 74, and
(b) detecting the presence of the antibody (or fragment thereof) bound to a p53 polypeptide.

**118.** A method according to claim 117, wherein said disease is cancer.

**119.** A method of gene therapy, wherein said method comprises:

(a) inserting a nucleic acid sequence according to any one of claims 1 to 26, or a vector according to any one of claims 75 to 83, into a host cell;
(b) expressing the nucleic acid sequence in the transformed cell.

**120.** The method according to claim 119, wherein said vector is an expression vector.

**121.** A process for preparing an antibody (or fragment thereof) having binding affinity to a p53 protein or a portion thereof in vertebrates, wherein said process comprises:

(a) isolating from a vertebrate a nucleic acid sequence according to any one of claims 1 to 26;
(b) cloning said nucleic acid sequence into a vector;
(c) constructing an antibody fragment library; and
(d) screening said library for clones expressing the antibody of interest.

**122.** The process according to claim 121, wherein said antibody (or fragment thereof) has binding affinity to a p53 protein or a portion thereof in vertebrates.

**123.** The process according to claim 121, wherein said nucleic acid sequence is obtained from an organ suffering from, or a collection point for expression of, the disease.

**124.** The process according to claim 123, wherein said organ is a lymph node.

**125.** The process according to any one of claims 121 to 124, wherein the vector is a phage display vector.

**126.** The process according to claim 125, wherein the vector is selected from the group consisting of MCO1, MCO3 and MCO6.

**127.** The process according to claim 125 or claim 126, wherein the vector is MCO1.

**128.** A method of locating a nucleotide sequence encoding a polypeptide of an antibody (or fragment thereof) having binding affinity to a p53 protein or portion thereof in vertebrates, using the nucleic acid sequence according to any one of claims 1 to 26, or an oligonucleotide fragment thereof.

**129.** The method according to claim 128, comprising:

(a) contacting a biological sample with a nucleic acid sequence according to any one of claims 1 to 26, or an oligonucleotide fragment thereof; and
(b) identifying nucleotide sequences in the biological sample which hybridise to said nucleic acid sequence or oligonucleotide fragment.

**130.** A method according to claim 129, wherein the oligonucleotide fragment is between about 10 to about 100 nucleotides in length.

**131.** A method according to claim 129 or claim 130, wherein the oligonucleotide fragment is between about 15 to about 30 nucleotides in length.

**Figure 1**

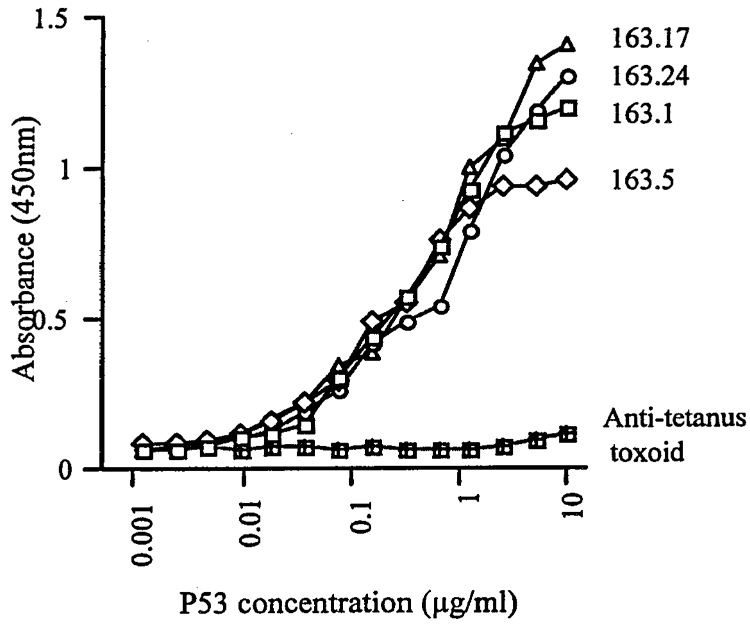
1.5
1
0.5
0
Absorbance (450nm)
0.001
0.01
0.1
1
10
P53 concentration (μg/ml)
163.17
163.24
163.1
163.5
Anti-tetanus toxoid

**Figure 2**

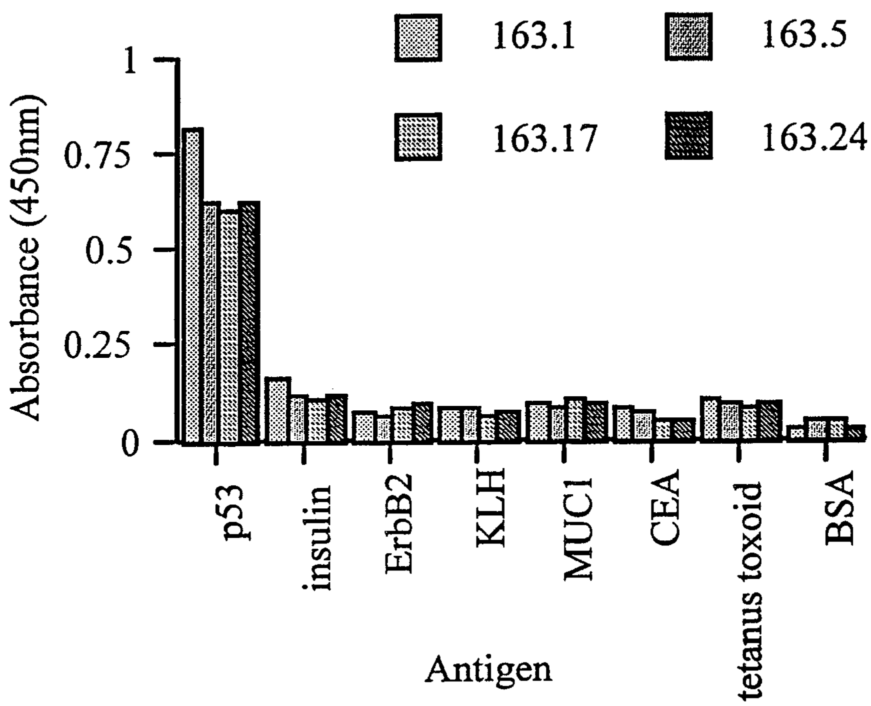
163.1
163.5
163.17
163.24
1
0.75
0.5
0.25
0
Absorbance (450nm)
p53
insulin
ErbB2
KLH
MUC1
CEA
tetanus toxoid
BSA
Antigen

Figure 3

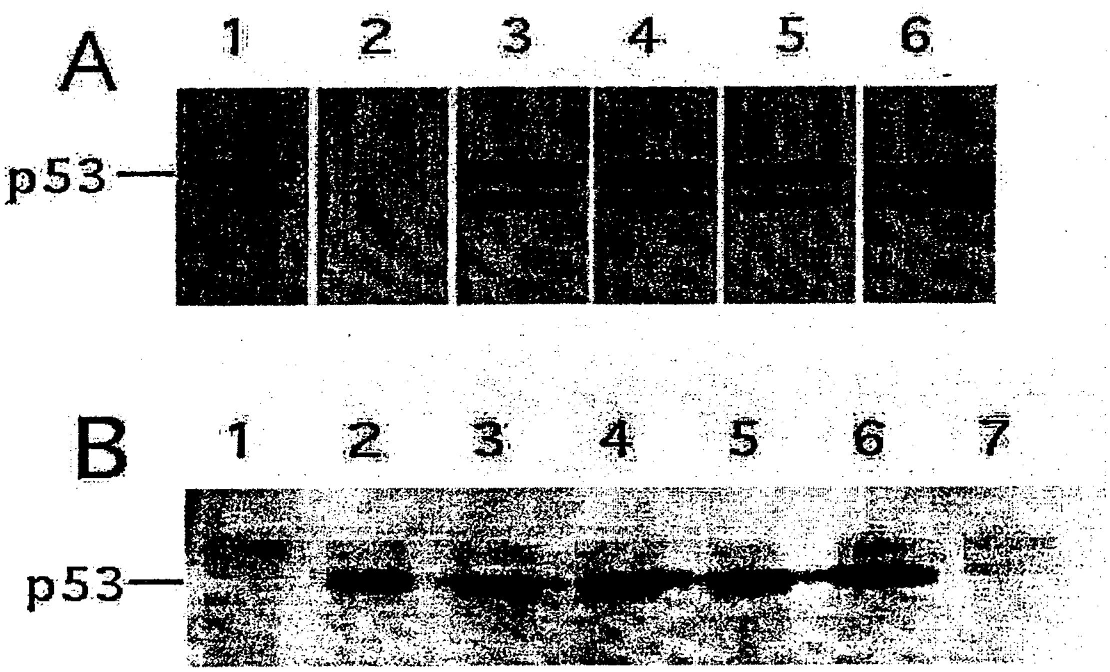

```
                              FR1                  CDR1          FR2                 CDR2                      FR3                      CDR3        FR4
DP-7    QVQLVQSGAEVKKPGASVKVSCKASGYTFT S--YYMH WVRQAPGQGLEWMG IINP--SGGSTSYAQKFQG RVTMTRDTSTSTVYMELSSLRSEDTAVYYC AR-YFDY WGQGTLVTVSS JH4b
163.22  VQL-E----eM-R---s-TI-cQa-RQ--S G--Q-I- -------------- V-n-----g-AN-aPS--- -LS-S--A--N-v--Kl---Tse------- LSQALK- -------A---
163.15  VQL-E---aeM-R---s-TI-cQa-RQ--S G--Q-I- -------------- V-n-----g-AN-aPS--- -LS-S--A--N-v--Kl---Tse------- LSQALK- -------A---
163.16  VQL-E----eM-R---s-TI-cQa-RQ--S G--Q-I- -------------- V-------g-AN-aPS--- -LS-S--A--N-v--Kl---Tse------- LSQALK- -------A---
163.23  VQL-E----eM-R---s-TI-cQa-RQ--S G--Q-I- -------------- V-------g-AN-aPS--- -LS-S--A--N-v--Kl---Tse------- LSQALK- -------A---
163.1   VQL-E----eM-R---s-TI-cQa-RQ--S G--Q-I- -------------- V-n-----g-AN-aPS--- -LS-S--A--N-v--Kl---Tse------- LSQALK- -------A---
163.9   VQL-E-------R---s-TI--Qa-RQD-S G--Q-Ih ---------F---- --n-----gsAN-aPk-K- -L--S--S--D-v--TlT--Tse---v--c LLQALKH -------A---
163.20  VQL-E--g----R---s-TI-cQa-RQD-S G--Q-Ih ---------F---- --n-----gsAN-aPk-K- -L--S--S--D-v--TlT--Tse---v--c LLQALKH -------A---
163.1   VQL-E-------R---s-TI--Qa-RQD-S G--Q-Ih ---------F---- --n-----gsAN-aPk-K- -L--S--S--D-v--TlT--Tse---v-yc LLQALKH -------A---
163.5   VQL-E--g----R---s-TI-cQa-RQD-S G--Q-Ih ---------F---- --n-----gsAN-aPk-K- -L--S--S--D-v--TlT--Tse---v--c LLQALKH -------A---
163.14  VQL-E-------R---s-TI--Qa-RQD-S G--Q-Ih ---------F---- --n-----gsAG-aPk-K- -L--S--S--D-v--TlT--Tse---v-yc LLQALKH -------A---
163.2   VQL-E--g----R---s-TI--Ra-RQD-S G--Q-Ih ---------F---- --n-----gsAN-aPk-K- -L--S--S--D-v--TlT--Tse---v-yc LLQALKH -------A---
163.6   VQL-E-------R---s-TI--Qa-RQD-S G--Q-Ih ---------F---- --n-----gsAN-aPk-K- -L--S--S--D-v--TlT--Tse---v-yc LLQALKH -------A---
163.24  VQL-E--g----R---s-TI--Qa-RQN-S G--Q-Ih ----------e--g --n-----gsAN-aPR-K- -LS-S--S--D-A-LTlT--Tse---v-F- LLQSLKH -------A---
163.17  VQL-E--g----R---s-TI--Qa-RQD-S G--Q-Ih ---------F---- --n-----gsAN-aPk-K- -L--S--S--E-v--TlT--Tse---v-yc LLQVLKH -------GP--
```

**Figure 4A**

```
                  FR1                      CDR1              FR2             CDR2                 FR3                       CDR3        FR4
DPk24    DIVMTQSPDSLAVSLGERATINCK SSQSVLYSSNNKNYLA WYQQKPGQPPKLLIY WASTRES GVPDRFSGSGSGTDFTLTISSLQAEDVAVYYC QQYYSTP
                                                                                                                 LT FGGGTKVEIK  JK4
163.15   AAEL-------------------- ---------------- --------------- ------- -------------------------------- --y-R---- ----------
163.17   AAEL-------------------- ---------------- --------------- ------- -------------------------------- ---F----- ----------
163.2    AAEL-------------------- ---------------- --------------- ------- -------------------------------- q--F----- ----------
163.6    AAEL----E--------------- ----v----------- --------------- ------- -------------------------------- q--F----- ----------
163.7    AAEL----E--------------- ----v----------- --------------- ------- -------------------------------- q--F--R-- ----------
                                                                                                                 YT FGQGTKLEIK  JK2
163.24   AAEL-------------------- ---------------- --------------- ------- -------------------------------- --------- ----------
163.23   AAEL-------------------- -N-----N--S----- ----------kl--- ------- -------------------------------- ---F----- ----------
163.16   AAEL-------------------- -N-----N--S----- ----------kl--- ------- -------------------------------- ---F----- ----------
163.1    AAEL-------------------- -N-----N--S----- ----------k---- -a----- ------------------T------------- ---F-S--- ----------
163.22   AAEL-------------------- -N-----N--S----- ----------k---- -a----- ------------------T------------- ---F-S--- ----------
163.14   AAEL-----------g-------- ---------------- --------------- -----Q- g-----R---s--------TN-----AaI--- ----G---- ----------
163.9    AAEL-----------g-------- ---------------- --------------- -----Q- g-----R---s--------TN-----AaI--- ----G---- ----------
163.20   AAEL----d----A---------- -s------L-----l- -----------l--H ------- -----------E------------e------- ----T---- ----------
163.5    AAEL----d----A---------- -s------L-----l- -----------l--H ------- -----------E------------e---GLF- ----T---- ----------
```

Figure 4B

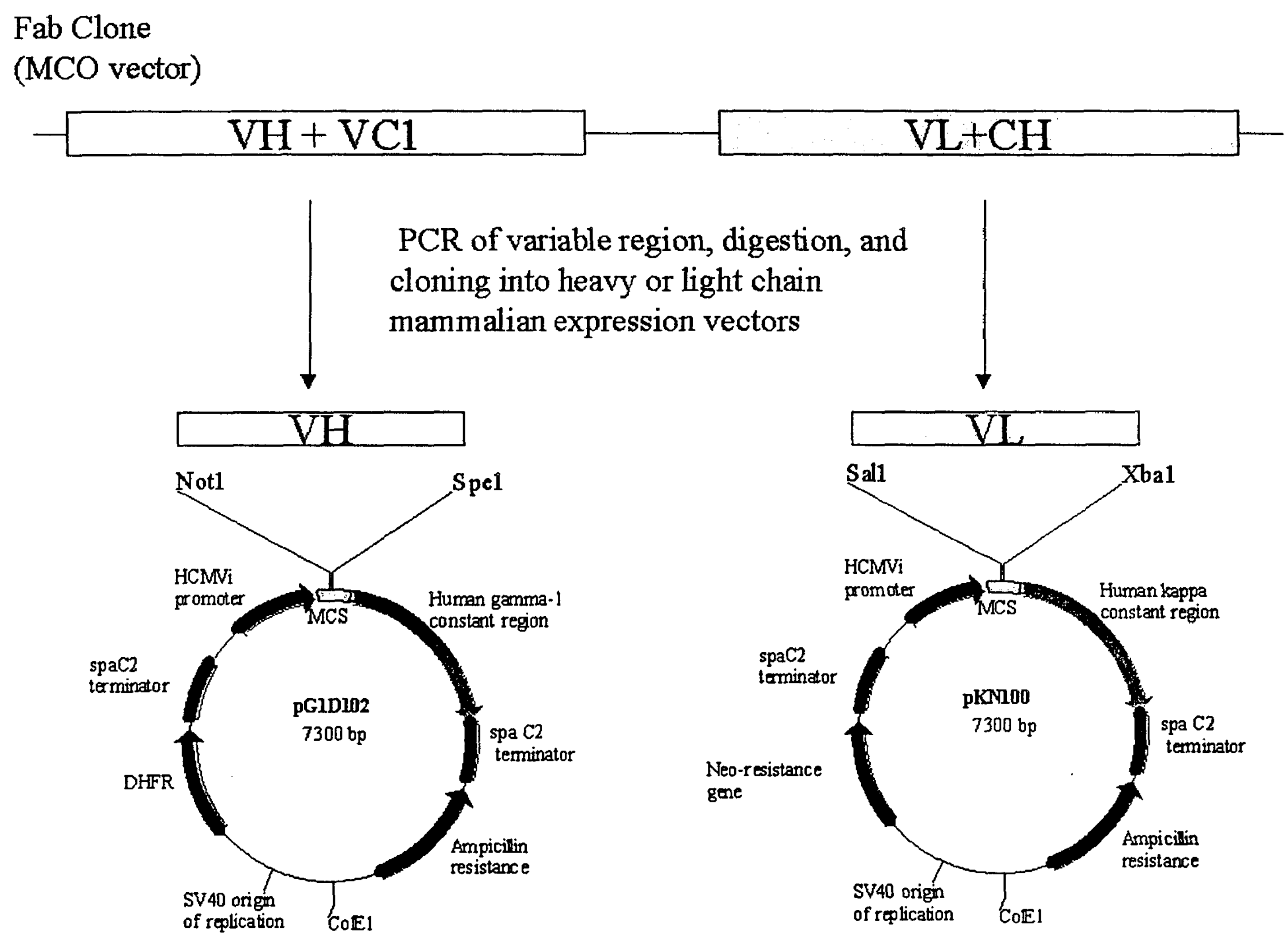
Fab Clone
(MCO vector)
VH + VC1
VL+CH
PCR of variable region, digestion, and cloning into heavy or light chain mammalian expression vectors
VH
VL
Not1
Spe1
Sal1
Xba1
HCMVi promoter
MCS
Human gamma-1 constant region
spaC2 terminator
pG1D102
7300 bp
spa C2 terminator
DHFR
Ampicillin resistance
SV40 origin of replication
CoE1
Human kappa constant region
pKN100
Neo-resistance gene

Figure 5

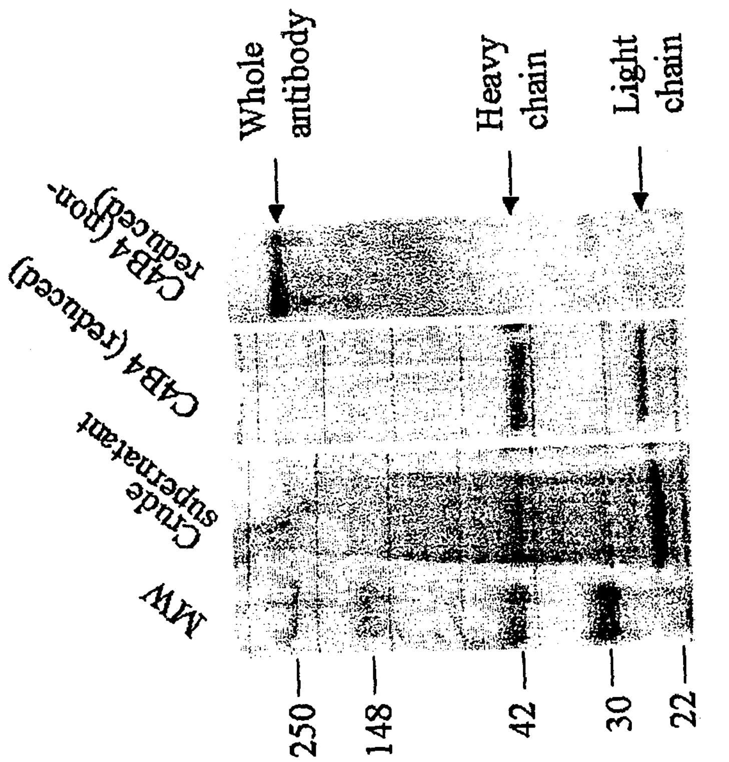
Whole antibody
Heavy chain
Light chain
CAB4 (non-reduced)
CAB4 (reduced)
Crude supernatant
MW
250
148
42
30
22

Figure 6

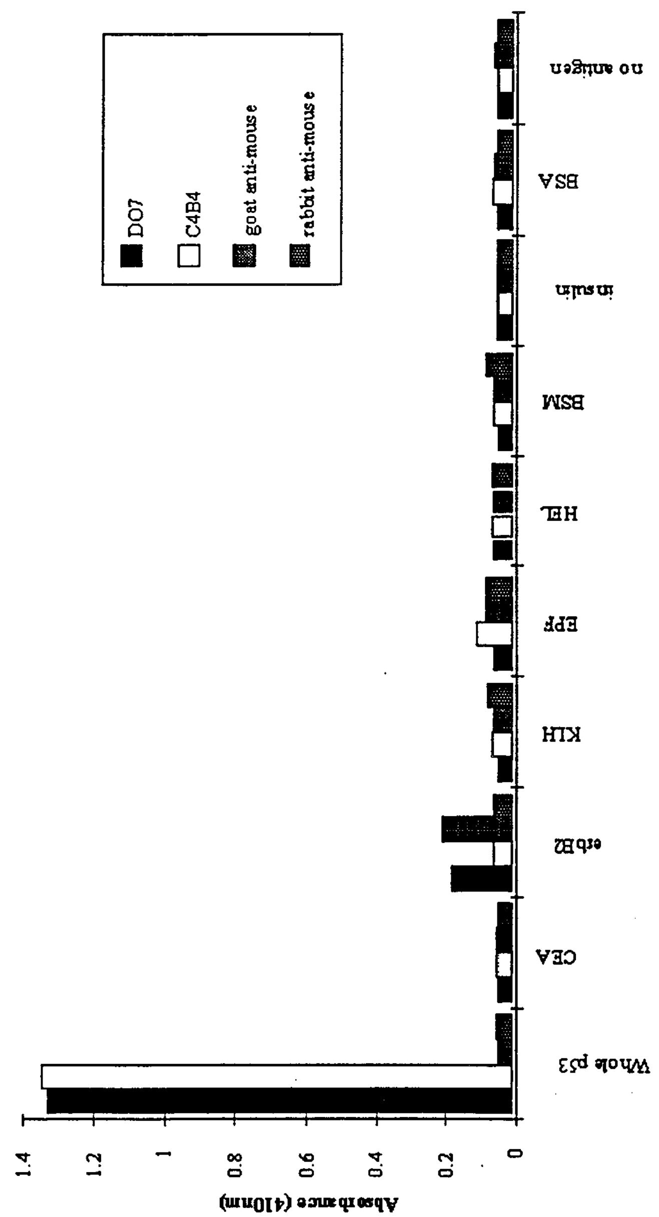
DO7
C4B4
goat anti-mouse
rabbit anti-mouse
Absorbance (410nm)
0
0.2
0.4
0.6
0.8
1
1.2
1.4
Whole p53
CEA
erbB2
KLH
EPF
HEL
BSM
insulin
BSA
no antigen

Figure 7

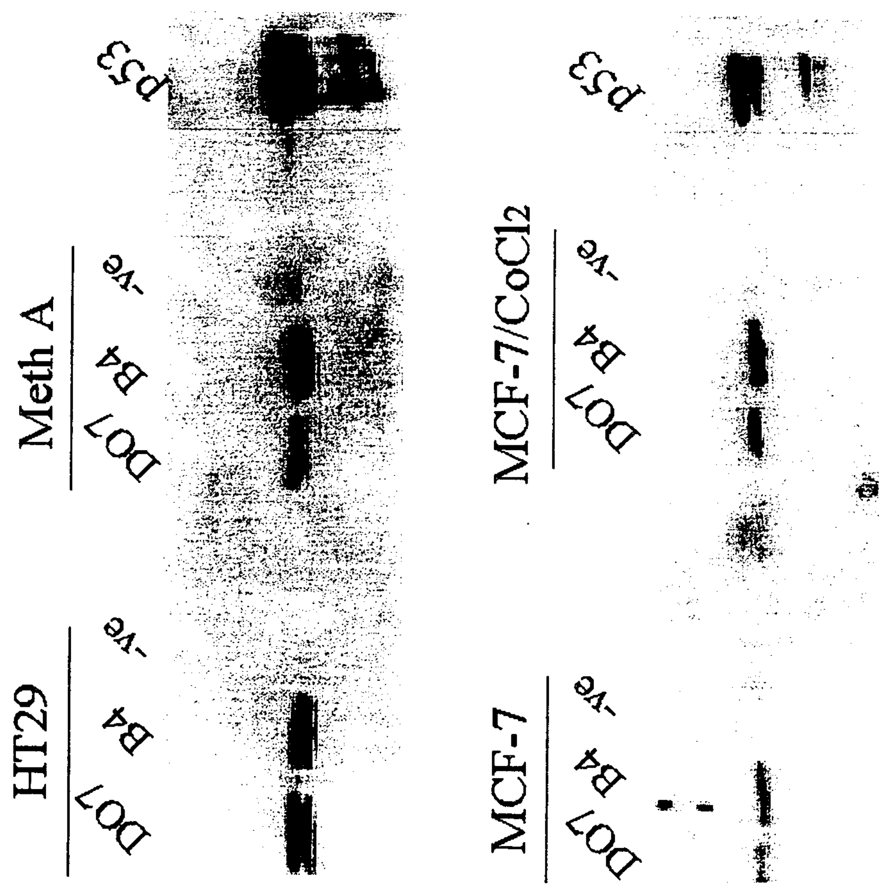
HT29
DO7
B4
-ve
Meth A
DO7
B4
-ve
p53
MCF-7
DO7
B4
-ve
MCF-7/CoCl2
DO7
B4
-ve
p53

Figure 8

```
p53      -SDLWKLLPENNVLSPLPSQAMDDLMLSPDDIEQWFTEDPGPDEAPRMPEA-
          20         30          40        50          60
Clone 1      WKLLPENNVLSPLPSQAMDDLMLSPDDIEQWF
Clone 7                     SQAMDDLMLSPDDIEQWF
Clone 34                        DDLMLSPDDIEQWF
```

# Figure 9

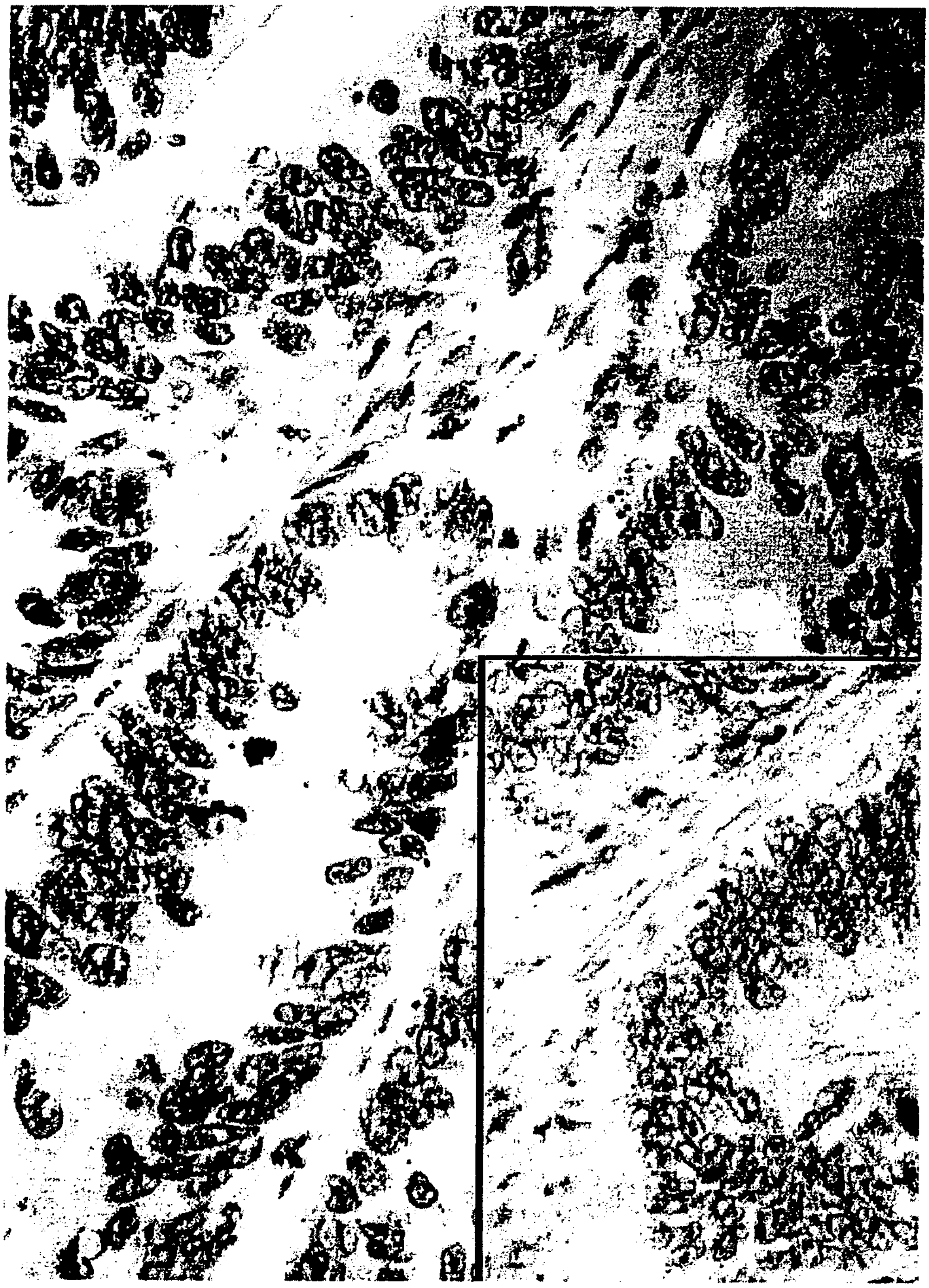

Figure 10

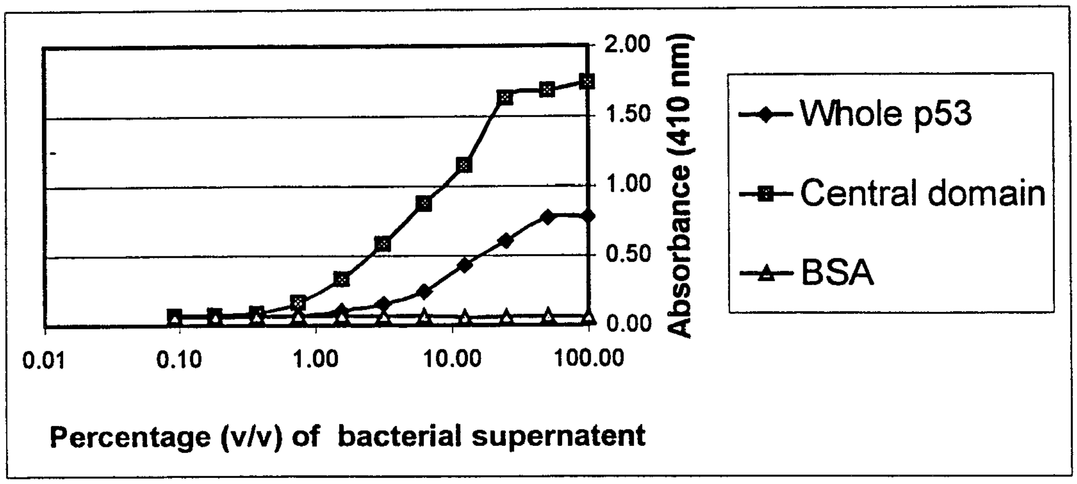
2.00
1.50
1.00
0.50
0.00
Absorbance (410 nm)
0.01
0.10
1.00
10.00
100.00
Percentage (v/v) of bacterial supernatent
Whole p53
Central domain
BSA

Figure 11

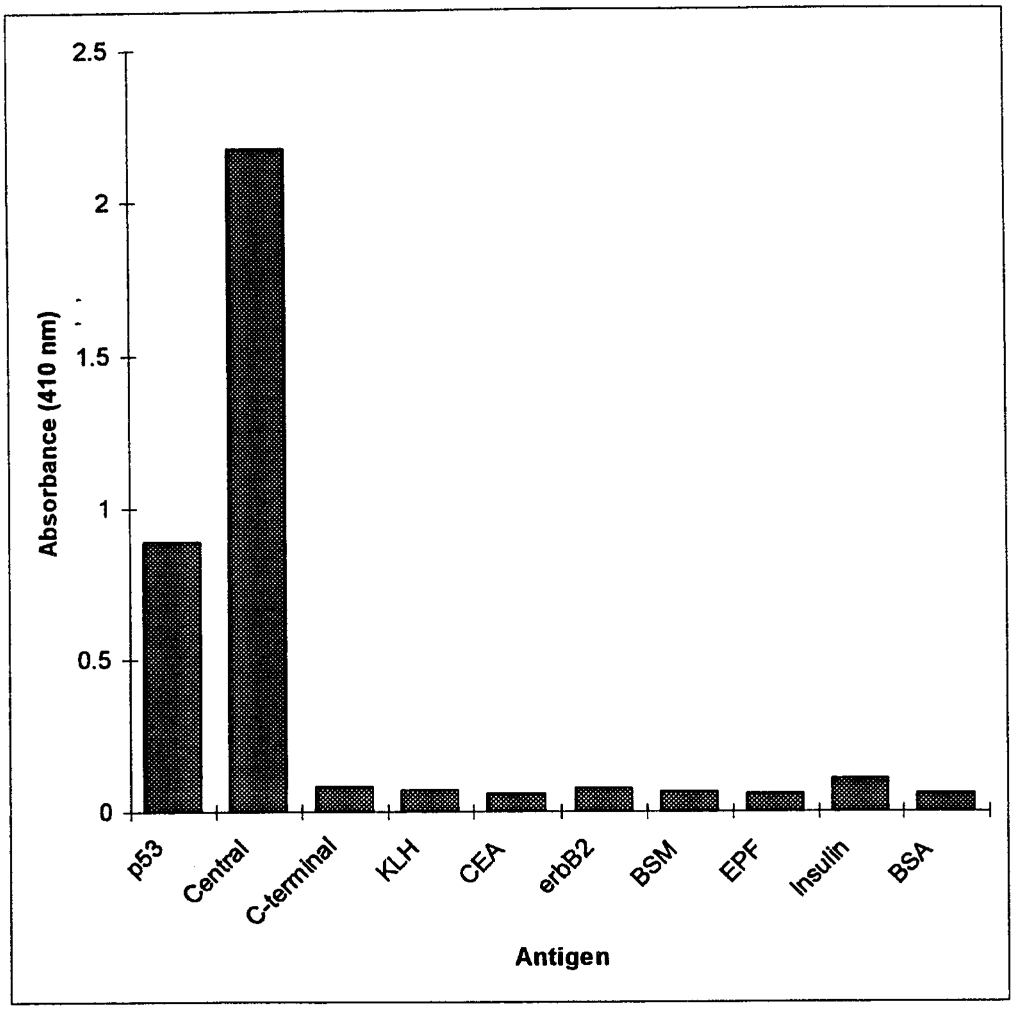
2.5
2
1.5
1
0.5
0
Absorbance (410 nm)
p53
Central
C-terminal
KLH
CEA
erbB2
BSM
EPF
Insulin
BSA
Antigen

Figure 12

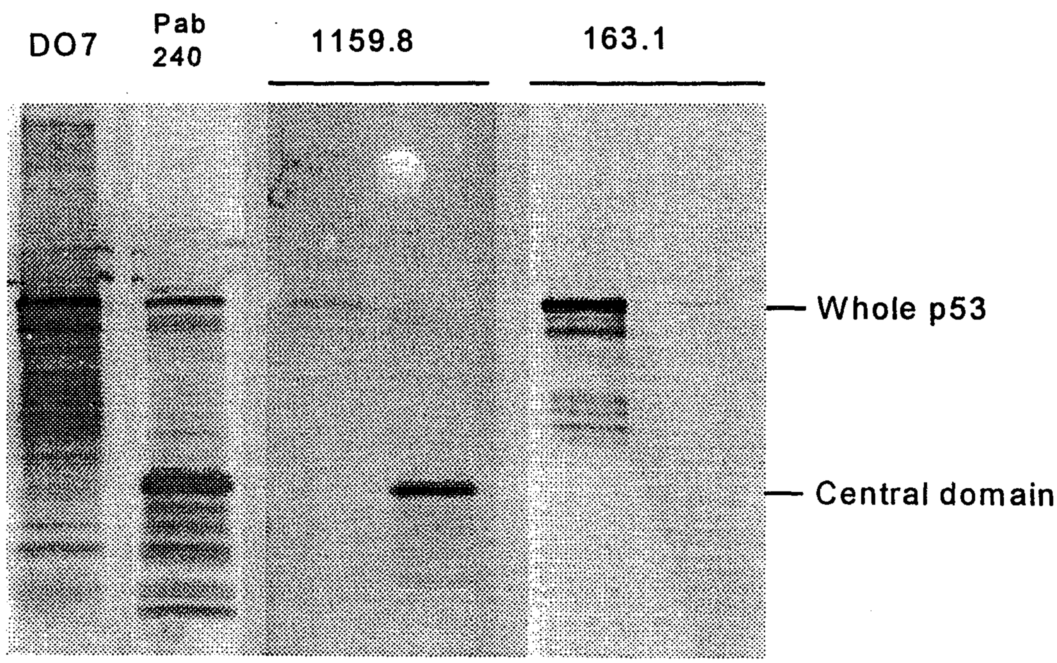


Figure 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Program Manual for the Wisconsin Package. *Genetics Computer Group, 575 Science Drive,* August 1996, 53711
- **NEEDLEMAN, S.B. ; WUNSCH, C.D.** *Journal of Molecular Biology,* 1970, vol. 48, 443-453
- **SAMBROOK et al.** Molecular Cloning, A Laboratory Manual. Cold Spring Harbour, 1989
- Current Protocols in Molecular Biology. John Wiley and Sons, 1995
- The Chemotherapy Source Book. Williams and Wilkins, 1996
- **SCATCHARD.** *Annals of the New York Academy of Sciences,* 1949, vol. 51, 660-672
- **MUNSON.** *Methods in Enzymology,* 1983, vol. 92, 543-577
- Remington's Pharmaceutical Science. Mack Publishing Company
- **HOLLSTEIN, M. ; D. SIDRANSKY ; B. VOGELSTEIN ; C. C. HARRIS.** p53 mutations in human cancers. *Science,* 1991, vol. 253, 49
- **PAVLETICH, N. P. ; K. A. CHAMBERS ; C. O. PABO.** The DNA-binding domain of p53 contains the four conserved regions and the major mutation hot spots. *Genes & Development,* 1993, vol. 7, 2556
- **VOGELSTEIN, B. ; K. W. KINZLER.** p53 function and dysfunction. *Cell,* 1992, vol. 70, 523
- **WINTER, S. F. ; J. D. MINNA ; B. E. JOHNSON ; T. TAKAHASHI ; A. F. GAZDAR ; D. P. CARBONE.** Development of antibodies against p53 in lung cancer patients appears to be dependent on the type of p53 mutation. *Cancer Research,* 1992, vol. 52, 4168
- **ABRAMS, P. G. ; J. L. ROSSIO ; H. C. STEVENSON ; K. A. FOON.** Optimal strategies for developing human-human monoclonal antibodies. *Methods in Enzymology,* 1986, vol. 121, 107
- **CLARK, M. A. ; N. J. HAWKINS ; A. PAPAIOANNOU ; R. J. FIDDES ; R. L. WARD.** Isolation of Human Anti-C-Erbb-2 Fabs From a Lymph Node-Derived Phage Display Library. *Clinical & Experimental Immunology,* 1997, vol. 109, 166
- **COOMBER, D. ; N. J. HAWKINS ; M. CLARK ; A. MEAGHER ; R. L. WARD.** Characterisation and Clinicopathological Correlates of Serum Anti-P53 Antibodies in Breast and Colon Cancer. *Journal of Cancer Research & Clinical Oncology,* 1996, vol. 122, 757
- **CHOMCZYNSKI, P. ; N. SACCHI.** Single-step method of RNA isolation by acid guanidinium thiocyanate-phenol-chloroform extraction. *Analytical Biochemistry,* 1987, vol. 162, 156
- **WARD, R. L. ; M. A. CLARK ; J. LEES ; N. J. HAWKINS.** Retrieval of Human Antibodies From Phage-Display Libraries Using Enzymatic Cleavage. *Journal of Immunological Methods,* 1996, vol. 189, 73
- **NISSIM, A. ; H. R. HOOGENBOOM ; I. M. TOMLINSON ; G. FLYNN ; C. MIDGLEY ; D. LANE ; G. WINTER.** Antibody fragments from a 'single pot' phage display library as immunochemical reagents. *Embo Journal,* 1994, vol. 13, 692
- **CHANG, B. ; P. CASALI.** The CDR1 sequences of a major proportion of human germline Ig VH genes are inherently susceptible to amino acid replacement. *Immunol Today,* 1994, vol. 15, 367
- **HENGEN, P. N.** Fidelity of DNA polymerases for PCR. *Trends in Biochemical Sciences,* 1995, vol. 20, 324
- **LUBIN, R. ; B. SCHLICHTHOLZ ; D. BENGOUFA ; G. ZALCMAN ; J. TREDANIEL ; A. HIRSCH ; C. C. DE FROMENTEL ; C. PREUDHOMME ; P. FENAUX ; G. FOURNIER et al.** Analysis of p53 antibodies in patients with various cancers define B-cell epitopes of human p53: distribution on primary structure and exposure on protein surface. *Cancer Research,* 1993, vol. 53, 5872
- **SOUSSI, T. ; P. MAY.** Structural aspects of the p53 protein in relation to gene evolution: a second look. *J Mol Biol.,* 1996, vol. 260, 623
- **KO, L. J. ; C. PRIVES.** p53: puzzle and paradigm. *Genes Dev.,* 1996, vol. 10, 1054
- **NAGESHA H. S. ; M. YU ; L. F. WANG.** Application of linker-ligation-PCR for construction of phage display epitope libraries. *Journal of Biological Methods,* 1996, vol. 60, 147-54
- **PETERSEN G ; D. SONG ; B. HUGLE-DORR ; I. OLDENBURG ; E. K. BAUTZ.** Mapping of linear epitopes recognized by monoclonal antibodies with gene-fragment phage display libraries. *Mol Gen Genet,* 1995, vol. 249, 425-31
- **SMITH G. P.** Filamentous fusion phage: novel expression vectors that display cloned antigens on the virion surface. *Science,* 1985, vol. 228, 1315-7

# SEQUENCE LISTING

<110> St Vincent's Hospital Sydney Limited

<120> Anti-p53 Antibodies

<130> 451541

<150> Australia PP9321
<151> 19 March 1999

<160> 60

<210> 1
<211> 339
<212> DNA
<213> Human

<400> 1

```
gcg gcc gag ctc acc cag tct cca gac tcc ctg gct gtg tct ctg  45

ggc gag agg gcc acc atc aac tgc aag tcc aac cag agt gtt tta  90

tac aac tcc aac agt aag aac tac tta gct tgg tac cag cag aaa  135

cca gga cag cct cct aaa ctg ctc att tac tgg gcg tct acc cgg  180

gaa tcc ggg gtc cct gac cga ttc agt ggc agc ggg tct ggg aca  225

gat ttc act ctc acc atc acc agc ctg cag gct gaa gat gtg gca  270

gtt tat tac tgt cag caa tat ttt agt tct ccc tac act ttt ggc  315

cag ggg acc aag ctg gaa atc aaa                              339
```

<130> 451541

<150> Australia PP9321
<151> 19 March 1999

<210> 2
<211> 342
<212> DNA
<213> Human

<400> 2

```
gtg cag ctg ctc gag cag tct ggg gct gaa atg aag agg cct ggg  45

gcc tcg gtg acg att tcc tgt cag gcc tct cga caa acc ttc agc  90

ggc cag tat ata cac tgg gtg cga cag gcc cct gga caa ggg ctt  135

gag tgg atg gga gtg atc aat cct agt ggt gga agc gca aac tac  180

gcg ccg agt ttc cag ggc aga ctc agc atg tcc agg gac gcg tcc  225

acg aac aca gtg tac atg aaa ttg agc agc ctg aca tcc gaa gac  270

acg gcc gtg tat tac tgt ctt tca cag gcc ctg aag tat tgg ggc  315

cag gga acc ctg gtc gcc gtc tcc tca  342
```

<130> 451541

<150> Australia PP9321
<151> 19 March 1999

<210> 3
<211> 339
<212> DNA
<213> Human

<400> 3

```
gcg gcc gag ctc acc cag tct cca gac tcc ctg gct gtg tct ctg  45

ggc gag agg gcc acc atc aac tgc aag tcc agc cag agt gtt tta  90

tac agc tcc aac aat aag aac tac tta gct tgg tac cag cag aaa  135

cca gga cag cct cct aag ctg ctc att tac tgg gca tct acc cgg  180

gaa tcc ggg gtc cct gac cga ttc agt ggc agc ggg tct ggg aca  225

gat ttc act ctc acc atc agc agc ctg cag gct gaa gat gtg gca  270

gtt tat tac tgt caa caa tat ttt agt act cca ctc act ttc ggc  315

gga ggg acc aag gtg gag atc aaa  339
```

<130> 451541

<150> Australia PP9321
<151> 19 March 1999

<210> 4
<211> 342
<212> DNA
<213> Human

<400> 4

```
cag ctg ctc gag cag tct gga gct gag gtg aag agg cct ggg gcc  45

tcg gtg aca att tcc tgc cgg gcc tct cga caa gat ttc agc ggc  90

cag tat att cat tgg gtg cga cag gcc cct gga caa ggg ttt gag  135

tgg atg gga ata atc aat cct agt ggt gga agt gca aac tac gcg  180

ccg aaa ttc aag ggc aga ctc acc atg tcc agg gac tcg tcc acg  225

gac aca gtt tac atg acc ttg acc agc ctg aca tcc gaa gac acg  270

gcc gtc tat tat tgc ctt tta cag gcc ctg aaa cat tgg ggc cag  315

gga acc ctg gtc gcc gtc tcc tca gcc  342
```

<130> 451541

<150> Australia PP9321
<151> 19 March 1999

<210> 5
<211> 339
<212> DNA
<213> Human

<400> 5

```
gcg gcc gag ctc acc cag tct cca gat tcc ctg gct gtg gct ctg  45

ggc gag agg gcc acc atc aac tgc aag tcc agt cag agt gtt tta  90

tac agc ctc aac aat aag aac tac ttg gct tgg tac cag cag aaa  135

cca gga cag cct cct aag cta ctc att cac tgg gca tct acc cgg  180

gaa tcc ggg gtc cct gac cga ttc agt ggc agc ggg tct gag aca  225

gat ttc act ctc acc atc agc agc ctg cag gct gag gat gtg gca  270

gtt tat tac tgt cag caa tat tat act act ccg tac act ttt ggc  315

cag ggg acc aag ctg gag atc aag  339
```

<130> 451541

<150> Australia PP9321
<151> 19 March 1999

<210> 6
<211> 360
<212> DNA
<213> Human

<400> 6

```
gtg cag ctg ctc gag cag tct gga gct gag gtg aag agg cct ggg  45

gcc tcg gtg aca att tcc tgt cag gcc tct cga caa gat ttc agc  90

ggc cag tat att cat tgg gtg cga cag gcc cct gga caa ggg ttt  135

gag tgg atg gga ata atc aat cct agt ggt gga agt gca aac tac  180

gcg ccg aaa ttc aag ggc aga ctc acc atg tcc agg gac tcg tcc  225

acg gac aca gtt tac atg acc ttg acc agc ctg aca tcc gaa gac  270

acg gcc gtc tat tac tgc ctt tta cag gcc ctg aaa cat tgg ggc  315

cag gga acc ctg gtc gcc gtc tcc tca  360
```

<130> 451541

<150> Australia PP9321
<151> 19 March 1999

<210> 7
<211> 339
<212> DNA
<213> Human

<400> 7

```
gcg gcc gag ctc acc cag tct cca gag tcc ctg gct gtg tct ctg  45

ggc gag agg gcc acc atc aac tgc aag tcc agc cag agt gtc tta  90

tac agc tcc aac aat aag aac tac tta gct tgg tac cag cag aaa  135

cca gga cag cct cct aag ctg ctc att tac tgg gca tct acc cgg  180

gaa tcc ggg gtc cct gac cga ttc agt ggc agc ggg tct ggg aca  225

gat ttc act ctc acc atc agc agc ctg cag gct gaa gat gtg gca  270

gtt tat tac tgt caa caa tat ttt agt act cca ctc act ttc ggc  315

gga ggg acc aag gtg gag atc aaa  339
```

<130> 451541

<150> Australia PP9321
<151> 19 March 1999

<210> 8
<211> 342
<212> DNA
<213> Human

<400> 8

```
gtg cag ctg ctc gag cag tct ggg gct gag gtg aag agg cct ggg  45

gcc tcg gtg aca att tcc tgc cag gcc tct cga caa gat ttc agc  90

ggc cag tat att cat tgg gtg cga cag gcc cct gga caa ggg ttt  135

gag tgg atg gga ata atc aat cct agt ggt gga agt gca aac tac  180

gcg ccg aaa ttc aag ggc aga ctc acc atg tcc agg gac tcg tcc  225

acg gac aca gtt tac atg acc ttg acc agc ctg aca tcc gaa gac  270

acg gcc gtc tat tat tgc ctt tta cag gcc ctg aaa cat tgg ggc  315

cag gga acc ctg gtc gcc gtc tcc tct  342
```

<130> 451541

<150> Australia PP9321
<151> 19 March 1999

<210> 9
<211> 339
<212> DNA
<213> Human

<400> 9

```
gcg gcc gag ctc acc cag tct cca gag tcc ctg gct gtg tct ctg  45

ggc gag agg gcc acc atc aac tgc aag tcc agc cag agt gtc tta  90

tac agc tcc aac aat aag aac tac tta gct tgg tac cag cag aaa  135

cca gga cag cct cct aag ctg ctc att tac tgg gca tct acc cgg  180

gaa tcc ggg gtc cct gac cga ttc agt ggc agc ggg tct ggg aca  225

gat ttc act ctc acc atc agc agc ctg cag gct gaa gat gtg gca  270

gtt tat tac tgt caa caa tat ttt agt act cca ctc act ttc ggc  315

gga ggg acc aag gtg gag atc aaa  339
```

<130> 451541

<150> Australia PP9321
<151> 19 March 1999

<210> 10
<211> 342
<212> DNA
<213> Human

<400> 10

```
gtg cag ctg ctc gag cag tct ggg gct gag gtg aag agg cct ggg  45

gcc tcg gtg aca att tcc tgc cag gcc tct cga caa gat ttc agc  90

ggc cag tat att cat tgg gtg cga cag gcc cct gga caa ggg ttt  135

gag tgg atg gga ata atc aat cct agt ggt gga agt gca aac tac  180

gcg ccg aaa ttc aag ggc aga ctc acc atg tcc agg gac tcg tcc  225

acg gac aca gtt tac atg acc ttg acc agc ctg aca tcc gaa gac  270

acg gcc gtc tat tat tgc ctt tta cag gcc ctg aaa cat tgg ggc  315

cag gga acc ctg gtc gcc gtc tcc tca  342
```

<130> 451541

<150> Australia PP9321
<151> 19 March 1999

<210> 11
<211> 339
<212> DNA
<213> Human

<400> 11

```
gcg gcc gag ctc acc cag tct cca gac tcc ctg gct gtg tct ctg  45

ggg gag agg gcc acc atc aac tgc aag tcc agc cag agt gtt tta  90

tac agc tcc aac aat aag aac tac tta gct tgg tac cag cag aaa  135

cca gga cag cct cct aag ctg ctc att tac tgg gca tct acc cgg  180

caa tcc ggt gtc cct gac cga ttc cgt ggc agc ggg tcc ggg aca  225

gat ttc act ctc acc atc acc aac ctg cag gct gaa gat gcg gcg  270

att tat tac tgt cag caa tat tat ggt act ccg tac act ttt ggc  315

cag ggg acc aaa tta gag atc aaa  339
```

<130> 451541

<150> Australia PP9321
<151> 19 March 1999

<210> 12
<211> 339
<212> DNA
<213> Human

<400> 12

```
gtg cag ctg ctc gag cag tct ggg gct gag gtg aag agg cct ggg  45

gcc tcg gtg aca att tcc tgc cag gcc tct cga caa gat ttc agc  90

ggc cag tat att cat tgg gtg cga cag gcc cct gga caa ggg ttt  135

gag tgg atg gga ata atc aat cct agt ggt gga agt gca aac tac  180

gcg ccg aaa ttc aag ggc aga ctc acc atg tcc agg gac tcg tcc  225

acg gac aca gtt tac atg acc ttg acc agc ctg aca tcc gaa gac  270

acg gcc gtc tat tac tgc ctt tta cag gcc ctg aaa cat tgg ggc  315

cag gga acc ctg gtc gcc gtc tcc tca  339
```

<130> 451541

<150> Australia PP9321
<151> 19 March 1999

<210> 13
<211> 339
<212> DNA
<213> Human

<400> 13

```
gcg gcc gag ctc acc cag tct cca gac tcc ctg gct gtg tct ctg  45

ggg gag agg gcc acc atc aac tgc aag tcc agc cag agt gtt tta  90

tac agc tcc aac aat aag aac tac tta gct tgg tac cag cag aaa  135

cca gga cag cct cct aag ctg ctc att tac tgg gca tct acc cgg  180

caa tcc ggt gtc cct gac cga ttc cgt ggc agc ggg tcc ggg aca  225

gat ttc act ctc acc atc acc aac ctg cag gct gaa gat gcg gcg  270

att tat tac tgt cag caa tat ttt agt tca ccc tac act ttt ggc  315

cag ggg acc aag ctg gag atc aaa  339
```

<130> 451541

<150> Australia PP9321
<151> 19 March 1999

<210> 14
<211> 342
<212> DNA
<213> Human

<400> 14

```
gtg cag ctg ctc gag cag tct ggg gct gag gtg aag agg cct ggg  45

gcc tcg gtg aca att tcc tgc cag gcc tct cga caa gat ttc agc  90

ggc cag tat att cat tgg gtg cga cag gcc cct gga caa ggg ttt  135

gag tgg atg gga ata atc aat cct agt ggt gga agt gcg ggc tac  180

gcg ccg aaa ttc aag ggc aga ctc acc atg tcc agg gac tcg tcc  225

acg gac aca gtt tac atg acc ttg acc agc ctg aca tcc gaa gac  270

acg gcc gtc tat tat tgc ctt tta cag gcc ctg aaa cat tgg ggc  315

cag gga acc ctg gtc gcc gtc tcc tca  342
```

<130> 451541

<150> Australia PP9321
<151> 19 March 1999

<210> 15
<211> 339
<212> DNA
<213> Human

<400> 15
gcg gcc gag ctc acc cag tct cca gac tcc ctg gct gtg tct ctg 45

ggc gag agg gcc acc atc aac tgc aag tcc agc cag agt gtt tta 90

tac agc tcc aac aat aag aac tac tta gct tgg tac cag cag aaa 135

cca gga cag cct cct aag ctg ctc att tac tgg gca tct acc cgg 180

gaa tcc ggg gtc cct gac cga ttc agt ggc agc ggg tct ggg aca 225

gat ttc act ctc acc atc agc agc ctg cag gct gaa gat gtg gca 270

gtt tat tac tgt cag caa tac tat agg act cct ctc act ttc ggc 315

gga ggg acc aag gtg gag atc aaa 339

<130> 451541

<150> Australia PP9321
<151> 19 March 1999

<210> 16
<211> 342
<212> DNA
<213> Human

<400> 16

```
gtg cag ctg ctc gag cag tct ggg gcg gaa atg aag agg cct ggg  45

gcc tcg gtg acg att tcc tgt cag gcc tct cga caa acc ttc agc  90

ggc cag tat ata cac tgg gtg cga cag gcc cct gga caa ggg ctt  135

gag tgg atg gga gtg atc aat cct agt ggt gga agc gca aac tac  180

gcg ccg agt ttc cag ggc aga ctc agc atg tcc agg gac gcg tcc  225

acg aac aca gtg tac atg aaa ttg agc agc ctg aca tcc gaa gac  270

acg gcc gtg tat tac tgt ctt tca cag gcc ctg aag tat tgg ggc  315

cag gga acc ctg gtc gcc gtc tcc tca  342
```

<130> 451541

<150> Australia PP9321
<151> 19 March 1999

<210> 17
<211> 339
<212> DNA
<213> Human

<400> 17

```
gcg gcc gag ctc acc cag tct cca gac tcc ctg gct gtg tct ctg  45

ggc gag agg gcc acc atc aac tgc aag tcc aac cag agt gtt tta  90

tac aat tcc aac agt aag aac tac tta gct tgg tac cag cag aaa  135

cca gga cag cct cct aaa ctt ctc att tac tgg gca tct acc cgg  180

gaa tcc ggg gtc cct gac cga ttc agt ggc agc ggg tct ggg aca  225

gat ttc act ctc acc atc agc agc ctg cag gct gaa gat gtg gca  270

gtt tat tac tgt cag caa tat ttt agt act ccc tac act ttt ggc  315

cag ggg acc aag ctg gag atc aaa  339
```

<130> 451541

<150> Australia PP9321
<151> 19 March 1999

<210> 18
<211> 342
<212> DNA
<213> Human

<400> 18

```
gtg cag ctg ctc gag cag tct ggg gct gaa atg aag agg cct ggg  45

gcc tcg gtg acg att tcc tgt cag gcc tct cga caa acc ttc agc  90

ggc cag tat ata cac tgg gtg cga cag gcc cct gga caa ggg ctt  135

gag tgg atg gga gtg atc aac cct agt ggt gga agc gca aac tac  180

gcg ccg agt ttc cag ggc aga ctc agc atg tcc agg gac gcg tcc  225

acg aac aca gtg tac atg aaa ttg agc agc ctg aca tcc gaa gac  270

acg gcc gtg tat tac tgt ctt tca cag gcc ctg aag tat tgg ggc  315

cag gga acc ctg gtc gcc gtc tcc tca  342
```

<130> 451541

<150> Australia PP9321
<151> 19 March 1999

<210> 19
<211> 339
<212> DNA
<213> Human

<400> 19

```
gcg gcc gag ctc acc cag tct cca gac tcc ctg gct gtg tct ctg  90

ggc gag agg gcc acc atc aac tgc aag tcc agc cag agt gtt tta

tac agc tcc aac aat aag aac tac tta gct tgg tac cag cag aaa  135

cca gga cag cct cct aag ctg ctc att tac tgg gca tct acc cgg  180

gaa tcc ggg gtc cct gac cga ttc agt ggc agc ggg tct ggg aca  225

gat ttc act ctc acc atc agc agc ctg cag gct gaa gat gtg gca  270

gtt tat tac tgt caa caa tat ttt agt act cca ctc act ttc ggc  315

gga ggg acc aag gtg gag atc aaa  339
```

<130> 451541

<150> Australia PP9321
<151> 19 March 1999

<210> 20
<211> 342
<212> DNA
<213> Human

<400> 20

```
gtg cag ctg ctc gag cag tct gga gct gag gtg aag agg cct ggg  45

gcc tcg gtg aca att tcc tgc cgg gcc tct cga caa gat ttc agc  90

ggc cag tat att cat tgg gtg cga cag gcc cct gga caa ggg ttt  135

gag tgg atg gga ata atc aat cct agt ggt gga agt gca aac tac  180

gcg ccg aaa ttc aag ggc aga ctc acc atg tcc agg gac tcg tcc  225

acg gac aca gtt tac atg acc ttg acc agc ctg aca tcc gaa gac  270

acg gcc gtc tat tat tgc ctt tta cag gcc ctg aaa cat tgg ggc  315

cag gga acc ctg gtc gga ccg tct tca  342
```

<130> 451541

<150> Australia PP9321
<151> 19 March 1999

<210> 21
<211> 339
<212> DNA
<213> Human

<400> 21

```
gcg gcc gag ctc acc cag tct cca gat tcc ctg gct gtg gct ctg 45

ggc gag agg gcc acc atc aac tgc aag tcc agt cag agt gtt tta  90

tac agc ctc aac aat aag aac tac ttg gct tgg tac cag cag aaa  135

cca gga cag cct cct aag cta ctc att cac tgg gca tct acc cgg  180

gaa tcc ggg gtc cct gac cga ttc agt ggc agc ggg tct gag aca  225

gat ttc act ctc acc atc agc agc ctg cag gct gag gat gtg gca  270

gtt tat tac tgt cag caa tat ttt agt tct ccc tac act ttt ggc  315

cag ggg acc aag ctg gaa atc aaa  339
```

<130> 451541

<150> Australia PP9321
<151> 19 March 1999

<210> 22
<211> 342
<212> DNA
<213> Human

<400> 22

```
gtg cag ctg ctc gag cag tct ggg gct gag gtg aag agg cct ggg  45

gcc tcg gtg aca att tcc tgc cag gcc tct cga caa gat ttc agc  90

ggc cag tat att cat tgg gtg cga cag gcc cct gga caa ggg ttt  135

gag tgg atg gga ata atc aat cct agt ggt gga agt gca aac tac  180

gcg ccg aaa ttc aag ggc aga ctc acc atg tcc agg gac tcg tcc  225

acg gac aca gtt tac atg acc ttg acc agc ctg aca tcc gaa gac  270

acg gcc gtc tat tac tgc ctt tta cag gcc ctg aaa cat tgg ggc  315

cag gga acc ctg gtc gcc gtc tcc tca  342
```

<130> 451541

<150> Australia PP9321
<151> 19 March 1999

<210> 23
<211> 339
<212> DNA
<213> Human

<400> 23

```
gcg gcc gag ctc acc cag tct cca gac tcc ctg gct gtg tct ctg  45

ggc gag agg gcc acc atc aac tgc aag tcc aac cag agt gtt tta  90

tac aac tcc aac agt aag aac tac tta gct tgg tac cag cag aaa  135

cca gga cag cct cct aaa ctg ctc att tac tgg gcg tct acc cgg  180

gaa tcc ggg gtc cct gac cga ttc agt ggc agc ggg tct ggg aca  225

gat ttc act ctc acc atc acc agc ctg cag gct gaa gat gtg gca  270

gtt tat tac tgt cag caa tat ttt agt tct ccc tac act ttt ggc  315

cag ggg acc aag ctg gaa atc aaa  339
```

<130> 451541

<150> Australia PP9321
<151> 19 March 1999

<210> 24
<211> 342
<212> DNA
<213> Human

<400> 24

```
gtg cag ctg ctc gag cag tct ggg gct gaa atg aag agg cct ggg  45

gcc tcg gtg acg att tcc tgt cag gcc tct cga caa acc ttc agc  90

ggc cag tat ata cac tgg gtg cga cag gcc cct gga caa ggg ctt  135

gag tgg atg gga gtg atc aat cct agt ggt gga agc gca aac tac  180

gcg ccg agt ttc cag ggc aga ctc agc atg tcc agg gac gcg tcc  225

acg aac aca gtg tac atg aaa ttg agc agc ctg aca tcc gaa gac  270

acg gcc gtg tat tac tgt ctt tca cag gcc ctg aag tat tgg ggc  315

cag gga acc ctg gtc gcc gtc tcc tca  342
```

<130> 451541

<150> Australia PP9321
<151> 19 March 1999

<210> 25
<211> 339
<212> DNA
<213> Human

<400> 25

```
gcg gcc gag ctc acc cag tct cca gac tcc ctg gct gtg tct ctg  45

ggc gag agg gcc acc atc aac tgc aag tcc aac cag agt gtt tta  90

tac aat tcc aac agt aag aac tac tta gct tgg tac cag cag aaa  135

cca gga cag cct cct aaa ctt ctc att tac tgg gca tct acc cgg  180

gaa tcc ggg gtc cct gac cga ttc agt ggc agc ggg tct ggg aca  225

gat ttc act ctc acc atc agc agc ctg cag gct gaa gat gtg gca  270

gtt tat tac tgt cag caa tat ttt agt act ccc tac act ttt ggc  315

cag ggg acc aag ctg gag atc aaa  339
```

<130> 451541

<150> Australia PP9321
<151> 19 March 1999

<210> 26
<211> 342
<212> DNA
<213> Human

<400> 26

```
gtg cag ctg ctc gag cag tct ggg gct gaa atg aag agg cct ggg

gcc tcg gtg acg att tcc tgt cag gcc tct cga caa acc ttc agc  90

ggc cag tat ata cac tgg gtg cga cag gcc cct gga caa ggg ctt  135

gag tgg atg gga gtg atc aac cct agt ggt gga agc gca aac tac  180

gcg ccg agt ttc cag ggc aga ctc agc atg tcc agg gac gcg tcc  225

acg aac aca gtg tac atg aaa ttg agc agc ctg aca tcc gaa gac  270

acg gcc gtg tat tac tgt ctt tca cag gcc ctg aag tat tgg ggc  315

cag gga acc ctg gtc gcc gtc tcc tca  342
```

<130> 451541

<150> Australia PP9321
<151> 19 March 1999

<210> 27
<211> 339
<212> DNA
<213> Human

<400> 27

```
gcg gcc gag ctc acc cag tct cca gac tcc ctg gct gtg tct ctg..45

ggc gag agg gcc acc atc aac tgc aag tcc agc cag agt gtt tta  90

tac agc tcc aac aat aag aac tac tta gct tgg tac cag cag aaa  135

cca gga cag cct cct aag ctg ctc att tac tgg gca tct acc cgg  180

gaa tcc ggg gtc cct gac cga ttc agt ggc agc ggg tct ggg aca  225

gat ttc act ctc acc atc agc agc ctg cag gct gaa gat gtg gca  270

gtt tat tac tgt cag caa tat tat agt act ccg tac act ttt ggc  315

cag ggg acc aag ctg gag atc aag  339
```

<130> 451541

<150> Australia PP9321
<151> 19 March 1999

<210> 28
<211> 342
<212> DNA
<213> Human

<400> 28

```
gtg cag ctg ctc gag cag tct gga gct gag gtg aag agg cct ggg  45

gcc tcg gtg aca att tcc tgc cag gcc tct cga caa aat ttc agc  90

ggc cag tat att cat tgg gtg cga cag gcc cct gga caa ggg ctt  135

gaa tgg atg ggc ata atc aat cct agt ggt gga agt gca aac tac  180

gcg ccg agg ttc aag ggc aga ctc tcc atg tcc agg gac tcg tcc  225

acg gac aca gct tac ttg aca ttg acc agc ctg aca tcc gaa gac  270

acg gcc gtc tat ttc tgt ctt tta cag tcc ctg aaa cat tgg ggc  315

cag gga acc ctg gtc gcc gtc tcc tca  342
```

<130> 451541

<150> Australia PP9321
<151> 19 March 1999

<210> 29
<211> 336
<212> DNA
<213> Human

<400> 29

```
gcg gcc gag ctc act cag tct cca ctc tcc ctg ccc gtc atc cct  45

gga gag ccg gcc tcc atc tcc tgc agg tct agt cag agc ctc ctg  90

cat agg aat gga tac aac tat ttg gat tgg tac ctg cag aag cca  135

ggg cag tct cca caa ctc ctg atc tat ttg ggt tct act cgg gcc  180

tcc ggg gtc cct gac aga ttc agt ggc agt gga tca ggc aca gat  225

ttt aca ctg aac atc aga aga gtg gag gct gag gat gtt ggg gtt  270

tat tat tgc atg caa ggt cta caa acg cca tac act ttc ggc gaa  315

ggg acc aag gtg gag atc aaa  336
```

<130> 451541

<150> Australia PP9321
<151> 19 March 1999

<210> 30
<211> 354
<212> DNA
<213> Human

<400> 30

```
gtg cag ctg ctc gag tct ggg gga ggc tta ata cag cca ggg cgg  45

tcc ctg aga ctc tca tgt aca gcc tct gga ttc ccc ttt ggt gat  90

tct gct atg acc tgg ttc cgc cag gct cca ggg aag ggg ctg gag  135

tgg gtg ggt ttc att aga agc aaa gct tat ggt gcg gca aca gca  180

tac gcc gcg tct atg aaa ggc aga gtt acc atc tca aga gat gat  225

gcc aaa agt atc gcc tat ctg cac atg agc aga ctg aag atc gag  270

gac aca gcc gtt tat ttc tgt agt aga gtg aaa gca ggg ggc cct  315

gac tac tgg ggc cag gga acc ctg gtc acc gtc tcc tca  354
```

<130> 451541

<150> Australia PP9321
<151> 19 March 1999

<210> 31
<211> 114
<212> Amino Acid
<213> Human

<400> 31

```
Ala Ala Glu Leu Thr Gln Ser Pro Asp Ser Leu Ala Val Ser Leu
 1               5                  10                  15

Gly Glu Arg Ala Thr Ile Asn Cys Lys Ser Asn Gln Ser Val Leu
                20                  25                  30

Tyr Asn Ser Asn Ser Lys Asn Tyr Leu Ala Trp Tyr Gln Gln Lys
                35                  40                  45

Pro Gly Gln Pro Pro Lys Leu Leu Ile Tyr Trp Ala Ser Thr Arg
                50                  55                  60

Glu Ser Gly Val Pro Asp Arg Phe Ser Gly Ser Gly Ser Gly Thr
                65                  70                  75

Asp Phe Thr Leu Thr Ile Thr Ser Leu Gln Ala Glu Asp Val Ala
                80                  85                  90

Val Tyr Tyr Cys Gln Gln Tyr Phe Ser Ser Pro Tyr Thr Phe Gly
                95                 100                 105

Gln Gly Thr Lys Leu Glu Ile Lys
           110
```

<130> 451541

<150> Australia PP9321
<151> 19 March 1999

<210> 32
<211> 114
<212> Amino Acid
<213> Human

<400> 32

```
Val Gln Leu Leu Glu Gln Ser Gly Ala Glu Met Lys Arg Pro Gly
  1               5                  10                  15

Ala Ser Val Thr Ile Ser Cys Gln Ala Ser Arg Gln Thr Phe Ser
                 20                  25                  30

Gly Gln Tyr Ile His Trp Val Arg Gln Ala Pro Gly Gln Gly Leu
                 35                  40                  45

Glu Trp Met Gly Val Ile Asn Pro Ser Gly Gly Ser Ala Asn Tyr
                 50                  55                  60

Ala Pro Ser Phe Gln Gly Arg Leu Ser Met Ser Arg Asp Ala Ser
                 65                  70                  75

Thr Asn Thr Val Tyr Met Lys Leu Ser Ser Leu Thr Ser Glu Asp
                 80                  85                  90

Thr Ala Val Tyr Tyr Cys Leu Ser Gln Ala Leu Lys Tyr Trp Gly
                 95                 100                 105

Gln Gly Thr Leu Val Ala Val Ser Ser
                 110
```

<130> 451541

<150> Australia PP9321
<151> 19 March 1999

<210> 33
<211> 113
<212> Amino Acid
<213> Human

<400> 33

```
Ala Ala Glu Leu Thr Gln Ser Pro Asp Ser Leu Ala Val Ser Leu
 1               5                  10                  15

Gly Glu Arg Ala Thr Ile Asn Cys Lys Ser Ser Gln Ser Val Leu
                20                  25                  30

Tyr Ser Ser Asn Asn Lys Asn Tyr Leu Ala Trp Tyr Gln Gln Lys
                35                  40                  45

Pro Gly Gln Pro Pro Lys Leu Leu Ile Tyr Trp Ala Ser Thr Arg
                50                  55                  60

Glu Ser Gly Val Pro Asp Arg Phe Ser Gly Ser Gly Ser Gly Thr
                65                  70                  75

Asp Phe Thr Leu Thr Ile Ser Ser Leu Gln Ala Glu Asp Val Ala
                80                  85                  90

Val Tyr Tyr Cys Gln Gln Tyr Phe Ser Thr Pro Leu Thr Phe Gly
                95                 100                 105

Gly Gly Thr Lys Val Glu Ile Lys
                110
```

<130> 451541

<150> Australia PP9321
<151> 19 March 1999

<210> 34
<211> 114
<212> Amino Acid
<213> Human

<400> 34

```
Gln Leu Leu Glu Gln Ser Gly Ala Glu Val Lys Arg Pro Gly Ala
 1               5                  10                  15

Ser Val Thr Ile Ser Cys Arg Ala Ser Arg Gln Asp Phe Ser Gly
                20                  25                  30

Gln Tyr Ile His Trp Val Arg Gln Ala Pro Gly Gln Gly Phe Glu
                35                  40                  45

Trp Met Gly Ile Ile Asn Pro Ser Gly Gly Ser Ala Asn Tyr Ala
                50                  55                  60

Pro Lys Phe Lys Gly Arg Leu Thr Met Ser Arg Asp Ser Ser Thr
                65                  70                  75

Asp Thr Val Tyr Met Thr Leu Thr Ser Leu Thr Ser Glu Asp Thr
                80                  85                  90

Ala Val Tyr Tyr Cys Leu Leu Gln Ala Leu Lys His Trp Gly Gln
                95                 100                 105

Gly Thr Leu Val Ala Val Ser Ser Ala
                110
```

<130> 451541

<150> Australia PP9321
<151> 19 March 1999

<210> 35
<211> 113
<212> Amino Acid
<213> Human

<400> 35

```
Ala Ala Glu Leu Thr Gln Ser Pro Asp Ser Leu Ala Val Ala Leu
 1               5                  10                  15

Gly Glu Arg Ala Thr Ile Asn Cys Lys Ser Ser Gln Ser Val Leu
                20                  25                  30

Tyr Ser Leu Asn Asn Lys Asn Tyr Leu Ala Trp Tyr Gln Gln Lys
                35                  40                  45

Pro Gly Gln Pro Pro Lys Leu Leu Ile His Trp Ala Ser Thr Arg
                50                  55                  60

Glu Ser Gly Val Pro Asp Arg Phe Ser Gly Ser Gly Ser Glu Thr
                65                  70                  75

Asp Phe Thr Leu Thr Ile Ser Ser Leu Gln Ala Glu Asp Val Ala
                80                  85                  90

Val Tyr Tyr Cys Gln Gln Tyr Tyr Thr Thr Pro Tyr Thr Phe Gly
                95                 100                 105

Gln Gly Thr Lys Leu Glu Ile Lys
               110
```

<130> 451541

<150> Australia PP9321
<151> 19 March 1999

<210> 36
<211> 114
<212> Amino Acid
<213> Human

<400> 36

```
Val Gln Leu Leu Glu Gln Ser Gly Ala Glu Val Lys Arg Pro Gly
 1               5                  10                  15

Ala Ser Val Thr Ile Ser Cys Gln Ala Ser Arg Gln Asp Phe Ser
                20                  25                  30

Gly Gln Tyr Ile His Trp Val Arg Gln Ala Pro Gly Gln Gly Phe
                35                  40                  45

Glu Trp Met Gly Ile Ile Asn Pro Ser Gly Gly Ser Ala Asn Tyr
                50                  55                  60

Ala Pro Lys Phe Lys Gly Arg Leu Thr Met Ser Arg Asp Ser Ser
                65                  70                  75

Thr Asp Thr Val Tyr Met Thr Leu Thr Ser Leu Thr Ser Glu Asp
                80                  85                  90

Thr Ala Val Tyr Tyr Cys Leu Leu Gln Ala Leu Lys His Trp Gly
                95                 100                 105

Gln Gly Thr Leu Val Ala Val Ser Ser
               110
```

<130> 451541

<150> Australia PP9321
<151> 19 March 1999

<210> 37
<211> 113
<212> Amino Acid
<213> Human

<400> 37

```
Ala Ala Glu Leu Thr Gln Ser Pro Glu Ser Leu Ala Val Ser Leu
 1               5                  10                  15

Gly Glu Arg Ala Thr Ile Asn Cys Lys Ser Ser Gln Ser Val Leu
                20                  25                  30

Tyr Ser Ser Asn Asn Lys Asn Tyr Leu Ala Trp Tyr Gln Gln Lys
                35                  40                  45

Pro Gly Gln Pro Pro Lys Leu Leu Ile Tyr Trp Ala Ser Thr Arg
                50                  55                  60

Glu Ser Gly Val Pro Asp Arg Phe Ser Gly Ser Gly Ser Gly Thr
                65                  70                  75

Asp Phe Thr Leu Thr Ile Ser Ser Leu Gln Ala Glu Asp Val Ala
                80                  85                  90

Val Tyr Tyr Cys Gln Gln Tyr Phe Ser Thr Pro Leu Thr Phe Gly
                95                 100                 105

Gly Gly Thr Lys Val Glu Ile Lys
               110
```

<130> 451541

<150> Australia PP9321
<151> 19 March 1999

<210> 38
<211> 114
<212> Amino Acid
<213> Human

<400> 38

```
Val Gln Leu Leu Glu Gln Ser Gly Ala Glu Val Lys Arg Pro Gly

Ala Ser Val Thr Ile Ser Cys Gln Ala Ser Arg Gln Asp Phe Ser
                20                  25                  30

Gly Gln Tyr Ile His Trp Val Arg Gln Ala Pro Gly Gln Gly Phe
                35                  40                  45

Glu Trp Met Gly Ile Ile Asn Pro Ser Gly Gly Ser Ala Asn Tyr
                50                  55                  60

Ala Pro Lys Phe Lys Gly Arg Leu Thr Met Ser Arg Asp Ser Ser
                65                  70                  75

Thr Asp Thr Val Tyr Met Thr Leu Thr Ser Leu Thr Ser Glu Asp
                80                  85                  90

Thr Ala Val Tyr Tyr Cys Leu Leu Gln Ala Leu Lys His Trp Gly
                95                 100                 105

Gln Gly Thr Leu Val Ala Val Ser Ser
                110
```

<130> 451541

<150> Australia PP9321
<151> 19 March 1999

<210> 39
<211> 113
<212> Amino Acid
<213> Human

<400> 39

```
Ala Ala Glu Leu Thr Gln Ser Pro Glu Ser Leu Ala Val Ser Leu
 1               5                  10                  15

Gly Glu Arg Ala Thr Ile Asn Cys Lys Ser Ser Gln Ser Val Leu
                20                  25                  30

Tyr Ser Ser Asn Asn Lys Asn Tyr Leu Ala Trp Tyr Gln Gln Lys
                35                  40                  45

Pro Gly Gln Pro Pro Lys Leu Leu Ile Tyr Trp Ala Ser Thr Arg
                50                  55
Glu Ser Gly Val Pro Asp Arg Phe Ser Gly Ser Gly Ser Gly Thr
                65                  70                  75

Asp Phe Thr Leu Thr Ile Ser Ser Leu Gln Ala Glu Asp Val Ala
                80                  85                  90

Val Tyr Tyr Cys Gln Gln Tyr Phe Ser Thr Arg Leu Thr Phe Gly
                95                 100                 105

Gly Gly Thr Lys Val Glu Ile Lys
               110
```

<130> 451541

<150> Australia PP9321
<151> 19 March 1999

<210> 40
<211> 114
<212> Amino Acid
<213> Human

<400> 40

```
Val Gln Leu Leu Glu Gln Ser Gly Ala Glu Val Lys Arg Pro Gly
 1               5                  10                  15

Ala Ser Val Thr Ile Ser Cys Gln Ala Ser Arg Gln Asp Phe Ser
                20                  25                  30

Gly Gln Tyr Ile His Trp Val Arg Gln Ala Pro Gly Gln Gly Phe
                35                  40                  45

Glu Trp Met Gly Ile Ile Asn Pro Ser Gly Gly Ser Ala Asn Tyr
                50                  55                  60

Ala Pro Lys Phe Lys Gly Arg Leu Thr Met Ser Arg Asp Ser Ser
                65                  70                  75

Thr Asp Thr Val Tyr Met Thr Leu Thr Ser Leu Thr Ser Glu Asp
                80                  85                  90

Thr Ala Val Tyr Tyr Cys Leu Leu Gln Ala Leu Lys His Trp Gly
                95                 100                 105

Gln Gly Thr Leu Val Ala Val Ser Ser
               110
```

<130> 451541

<150> Australia PP9321
<151> 19 March 1999

<210> 41
<211> 113
<212> Amino Acid
<213> Human

<400> 41

```
Ala Ala Glu Leu Thr Gln Ser Pro Asp Ser Leu Ala Val Ser Leu
 1               5                  10                  15

Gly Glu Arg Ala Thr Ile Asn Cys Lys Ser Ser Gln Ser Val Leu
                20                  25                  30

Tyr Ser Ser Asn Asn Lys Asn Tyr Leu Ala Trp Tyr Gln Gln Lys
                35                  40                  45

Pro Gly Gln Pro Pro Lys Leu Leu Ile Tyr Trp Ala Ser Thr Arg
                50                  55                  60

Gln Ser Gly Val Pro Asp Arg Phe Arg Gly Ser Gly Ser Gly Thr
                65                  70                  75

Asp Phe Thr Leu Thr Ile Thr Asn Leu Gln Ala Glu Asp Ala Ala
                80                  85                  90

Ile Tyr Tyr Cys Gln Gln Tyr Tyr Gly Thr Pro Tyr Thr Phe Gly
                95                 100                 105

Gln Gly Thr Lys Leu Glu Ile Lys
               110
```

<130> 451541

<150> Australia PP9321
<151> 19 March 1999

<210> 42
<211> 114
<212> Amino Acid
<213> Human

<400> 42

```
Val Gln Leu Leu Glu Gln Ser Gly Ala Glu Val Lys Arg Pro Gly
 1               5                  10                  15

Ala Ser Val Thr Ile Ser Cys Gln Ala Ser Arg Gln Asp Phe Ser
                20                  25                  30

Gly Gln Tyr Ile His Trp Val Arg Gln Ala Pro Gly Gln Gly Phe
                35                  40                  45

Glu Trp Met Gly Ile Ile Asn Pro Ser Gly Gly Ser Ala Asn Tyr
                50                  55                  60

Ala Pro Lys Phe Lys Gly Arg Leu Thr Met Ser Arg Asp Ser Ser
                65                  70                  75

Thr Asp Thr Val Tyr Met Thr Leu Thr Ser Leu Thr Ser Glu Asp
                80                  85                  90

Thr Ala Val Tyr Tyr Cys Leu Leu Gln Ala Leu Lys His Trp Gly
                95                 100                 105

Gln Gly Thr Leu Val Ala Val Ser Ser
               110
```

<130> 451541

<150> Australia PP9321
<151> 19 March 1999

<210> 43
<211> 113
<212> Amino Acid
<213> Human

<400> 43

```
Ala Ala Glu Leu Thr Gln Ser Pro Asp Ser Leu Ala Val Ser Leu
 1              5                   10                  15

Gly Glu Arg Ala Thr Ile Asn Cys Lys Ser Ser Gln Ser Val Leu
                20                  25                  30

Tyr Ser Ser Asn Asn Lys Asn Tyr Leu Ala Trp Tyr Gln Gln Lys
                35                  40                  45

Pro Gly Gln Pro Pro Lys Leu Leu Ile Tyr Trp Ala Ser Thr Arg
                50                  55                  60

Gln Ser Gly Val Pro Asp Arg Phe Arg Gly Ser Gly Ser Gly Thr
                65                  70                  75

Asp Phe Thr Leu Thr Ile Thr Asn Leu Gln Ala Glu Asp Ala Ala
                80                  85                  90

Ile Tyr Tyr Cys Gln Gln Tyr Phe Ser Ser Pro Tyr Thr Phe Gly
                95                 100                 105

Gln Gly Thr Lys Leu Glu Ile Lys
               110
```

<130> 451541

<150> Australia PP9321
<151> 19 March 1999

<210> 44
<211> 114
<212> Amino Acid
<213> Human

<400> 44

```
Val Gln Leu Leu Glu Gln Ser Gly Ala Glu Val Lys Arg Pro Gly
 1               5                  10                  15

Ala Ser Val Thr Ile Ser Cys Gln Ala Ser Arg Gln Asp Phe Ser
                20                  25                  30

Gly Gln Tyr Ile His Trp Val Arg Gln Ala Pro Gly Gln Gly Phe
                35                  40                  45

Glu Trp Met Gly Ile Ile Asn Pro Ser Gly Gly Ser Ala Gly Tyr
                50                  55                  60

Ala Pro Lys Phe Lys Gly Arg Leu Thr Met Ser Arg Asp Ser Ser
                65                  70                  75

Thr Asp Thr Val Tyr Met Thr Leu Thr Ser Leu Thr Ser Glu Asp
                80                  85                  90

Thr Ala Val Tyr Tyr Cys Leu Leu Gln Ala Leu Lys His Trp Gly
                95                 100                 105

Gln Gly Thr Leu Val Ala Val Ser Ser
               110
```

<130> 451541

<150> Australia PP9321
<151> 19 March 1999

<210> 45
<211> 113
<212> Amino Acid
<213> Human

<400> 45

```
Ala Ala Glu Leu Thr Gln Ser Pro Asp Ser Leu Ala Val Ser Leu
 1               5                  10                  15

Gly Glu Arg Ala Thr Ile Asn Cys Lys Ser Ser Gln Ser Val Leu
                20                  25                  30

Tyr Ser Ser Asn Asn Lys Asn Tyr Leu Ala Trp Tyr Gln Gln Lys
                35                  40                  45

Pro Gly Gln Pro Pro Lys Leu Leu Ile Tyr Trp Ala Ser Thr Arg
                50                  55                  60

Glu Ser Gly Val Pro Asp Arg Phe Ser Gly Ser Gly Ser Gly Thr
                65                  70                  75

Asp Phe Thr Leu Thr Ile Ser Ser Leu Gln Ala Glu Asp Val Ala
                80                  85                  90

Val Tyr Tyr Cys Gln Gln Tyr Tyr Arg Thr Pro Leu Thr Phe Gly
                95                 100                 105

Gly Gly Thr Lys Val Glu Ile Lys
               110
```

<130> 451541

<150> Australia PP9321
<151> 19 March 1999

<210> 46
<211> 114
<212> Amino Acid
<213> Human

<400> 46

```
Val Gln Leu Leu Glu Gln Ser Gly Ala Glu Met Lys Arg Pro Gly
 1               5                  10                  15

Ala Ser Val Thr Ile Ser Cys Gln Ala Ser Arg Gln Thr Phe Ser
                20                  25                  30

Gly Gln Tyr Ile His Trp Val Arg Gln Ala Pro Gly Gln Gly Leu
                35                  40                  45

Glu Trp Met Gly Val Ile Asn Pro Ser Gly Gly Ser Ala Asn Tyr
                50                  55                  60

Ala Pro Ser Phe Gln Gly Arg Leu Ser Met Ser Arg Asp Ala Ser
                65                  70                  75

Thr Asn Thr Val Tyr Met Lys Leu Ser Ser Leu Thr Ser Glu Asp
                80                  85                  90

Thr Ala Val Tyr Tyr Cys Leu Ser Gln Ala Leu Lys Tyr Trp Gly
                95                 100                 105

Gln Gly Thr Leu Val Ala Val Ser Ser
               110
```

<130> 451541

<150> Australia PP9321
<151> 19 March 1999

<210> 47
<211> 113
<212> Amino Acid
<213> Human

<400> 47

```
Ala Ala Glu Leu Thr Gln Ser Pro Asp Ser Leu Ala Val Ser Leu
 1               5                  10                  15

Gly Glu Arg Ala Thr Ile Asn Cys Lys Ser Asn Gln Ser Val Leu
                20                  25                  30

Tyr Asn Ser Asn Ser Lys Asn Tyr Leu Ala Trp Tyr Gln Gln Lys
                35                  40                  45

Pro Gly Gln Pro Pro Lys Leu Leu Ile Tyr Trp Ala Ser Thr Arg
                50                  55                  60

Glu Ser Gly Val Pro Asp Arg Phe Ser Gly Ser Gly Ser Gly Thr
                65                  70                  75

Asp Phe Thr Leu Thr Ile Ser Ser Leu Gln Ala Glu Asp Val Ala
                80                  85                  90

Val Tyr Tyr Cys Gln Gln Tyr Phe Ser Thr Pro Tyr Thr Phe Gly
                95                 100                 105

Gln Gly Thr Lys Leu Glu Ile Lys
               110
```

<130> 451541

<150> Australia PP9321
<151> 19 March 1999

<210> 48
<211> 114
<212> Amino Acid
<213> Human

<400> 48

```
Val Gln Leu Leu Glu Gln Ser Gly Ala Glu Met Lys Arg Pro Gly
 1               5                  10                  15

Ala Ser Val Thr Ile Ser Cys Gln Ala Ser Arg Gln Thr Phe Ser
                20                  25                  30

Gly Gln Tyr Ile His Trp Val Arg Gln Ala Pro Gly Gln Gly Leu
                35                  40                  45

Glu Trp Met Gly Val Ile Asn Pro Ser Gly Gly Ser Ala Asn Tyr
                50                  55                  60

Ala Pro Ser Phe Gln Gly Arg Leu Ser Met Ser Arg Asp Ala Ser
                65                  70                  75

Thr Asn Thr Val Tyr Met Lys Leu Ser Ser Leu Thr Ser Glu Asp
                80                  85                  90

Thr Ala Val Tyr Tyr Cys Leu Ser Gln Ala Leu Lys Tyr Trp Gly
                95                 100                 105

Gln Gly Thr Leu Val Ala Val Ser Ser
               110
```

<130> 451541

<150> Australia PP9321
<151> 19 March 1999

<210> 49
<211> 113
<212> Amino Acid
<213> Human

<400> 49

```
Ala Ala Glu Leu Thr Gln Ser Pro Asp Ser Leu Ala Val Ser Leu
 1               5                  10                  15

Gly Glu Arg Ala Thr Ile Asn Cys Lys Ser Ser Gln Ser Val Leu
                20                  25                  30

Tyr Ser Ser Asn Asn Lys Asn Tyr Leu Ala Trp Tyr Gln Gln Lys
                35                  40                  45

Pro Gly Gln Pro Pro Lys Leu Leu Ile Tyr Trp Ala Ser Thr Arg
                50                  55                  60

Glu Ser Gly Val Pro Asp Arg Phe Ser Gly Ser Gly Ser Gly Thr
                65                  70                  75

Asp Phe Thr Leu Thr Ile Ser Ser Leu Gln Ala Glu Asp Val Ala
                80                  85                  90

Val Tyr Tyr Cys Gln Gln Tyr Phe Ser Thr Pro Leu Thr Phe Gly
                95                 100                 105

Gly Gly Thr Lys Val Glu Ile Lys
               110
```

<130> 451541

<150> Australia PP9321
<151> 19 March 1999

<210> 50
<211> 114
<212> Amino Acid
<213> Human

<400> 50

```
Val Gln Leu Leu Glu Gln Ser Gly Ala Glu Val Lys Arg Pro Gly
 1               5                  10                  15

Ala Ser Val Thr Ile Ser Cys Arg Ala Ser Arg Gln Asp Phe Ser
                20                  25                  30

Gly Gln Tyr Ile His Trp Val Arg Gln Ala Pro Gly Gln Gly Phe
                35                  40                  45

Glu Trp Met Gly Ile Ile Asn Pro Ser Gly Gly Ser Ala Asn Tyr
                50                  55                  60

Ala Pro Lys Phe Lys Gly Arg Leu Thr Met Ser Arg Asp Ser Ser
                65                  70                  75

Thr Glu Thr Val Tyr Met Thr Leu Thr Ser Leu Thr Ser Glu Asp
                80                  85                  90

Thr Ala Val Tyr Tyr Cys Leu Leu Gln Ala Leu Lys His Trp Gly
                95                 100                 105

Gln Gly Thr Leu Val Gly Pro Ser Ser
               110
```

<130> 451541

<150> Australia PP9321
<151> 19 March 1999

<210> 51
<211> 114
<212> Amino Acid
<213> Human

<400> 51

```
Ala Ala Glu Leu Thr Gln Ser Pro Asp Ser Leu Ala Val Ala Leu
 1               5                  10                  15

Gly Glu Arg Ala Thr Ile Asn Cys Lys Ser Ser Gln Ser Val Leu
                20                  25                  30

Tyr Ser Leu Asn Asn Lys Asn Tyr Leu Ala Trp Tyr Gln Gln Lys
                35                  40                  45

Pro Gly Gln Pro Pro Lys Leu Leu Ile His Trp Ala Ser Thr Arg
                50                  55                  60

Glu Ser Gly Val Pro Asp Arg Phe Ser Gly Ser Gly Ser Glu Thr
                65                  70                  75

Asp Phe Thr Leu Thr Ile Ser Ser Leu Gln Ala Glu Asp Val Ala
                80                  85                  90

Val Tyr Tyr Cys Gln Gln Tyr Phe Ser Ser Pro Tyr Thr Phe Gly
                95                 100                 105

     Gln Gly Thr Lys Leu Glu Ile Lys
                110
```

<130> 451541

<150> Australia PP9321
<151> 19 March 1999

<210> 52
<211> 114
<212> Amino Acid
<213> Human

<400> 52

```
Val Gln Leu Leu Glu Gln Ser Gly Ala Glu Val Lys Arg Pro Gly
 1              5                   10                  15

Ala Ser Val Thr Ile Ser Cys Gln Ala Ser Arg Gln Asp Phe Ser
                20                  25                  30

Gly Gln Tyr Ile His Trp Val Arg Gln Ala Pro Gly Gln Gly Phe
                35                  40                  45

Glu Trp Met Gly Ile Ile Asn Pro Ser Gly Gly Ser Ala Asn Tyr
                50                  55                  60

Ala Pro Lys Phe Lys Gly Arg Leu Thr Met Ser Arg Asp Ser Ser
                65                  70                  75

Thr Asp Thr Val Tyr Met Thr Leu Thr Ser Leu Thr Ser Glu Asp>
                80                  85                  90

Thr Ala Val Tyr Tyr Cys Leu Leu Gln Ala Leu Lys His Trp Gly
                95                  100                 105

Gln Gly Thr Leu Val Ala Val Ser Ser
                110
```

<130> 451541

<150> Australia PP9321
<151> 19 March 1999

<210> 53
<211> 113
<212> Amino Acid
<213> Human

<400> 53

```
Ala Ala Glu Leu Thr Gln Ser Pro Asp Ser Leu Ala Val Ser Leu
 1               5                  10                  15

Gly Glu Arg Ala Thr Ile Asn Cys Lys Ser Asn Gln Ser Val Leu
                20                  25                  30

Tyr Asn Ser Asn Ser Lys Asn Tyr Leu Ala Trp Tyr Gln Gln Lys
                35                  40                  45

Pro Gly Gln Pro Pro Lys Leu Leu Ile Tyr Trp Ala Ser Thr Arg
                50                  55                  60

Glu Ser Gly Val Pro Asp Arg Phe Ser Gly Ser Gly Ser Gly Thr
                65                  70                  75

Asp Phe Thr Leu Thr Ile Thr Ser Leu Gln Ala Glu Asp Val Ala
                80                  85                  90

Val Tyr Tyr Cys Gln Gln Tyr Phe Ser Ser Pro Tyr Thr Phe Gly
                95                 100                 105

Gln Gly Thr Lys Leu Glu Ile Lys
               110
```

<130> 451541

<150> Australia PP9321
<151> 19 March 1999

<210> 54
<211> 114
<212> Amino Acid
<213> Human

<400> 54

```
Val Gln Leu Leu Glu Gln Ser Gly Ala Glu Met Lys Arg Pro Gly
 1               5                  10                  15

Ala Ser Val Thr Ile Ser Cys Gln Ala Ser Arg Gln Thr Phe Ser
                20                  25                  30

Gly Gln Tyr Ile His Trp Val Arg Gln Ala Pro Gly Gln Gly Leu
                35                  40                  45

Glu Trp Met Gly Val Ile Asn Pro Ser Gly Gly Ser Ala Asn Tyr
                50                  55                  60

Ala Pro Ser Phe Gln Gly Arg Leu Ser Met Ser Arg Asp Ala Ser
                65                  70                  75

Thr Asn Thr Val Tyr Met Lys Leu Ser Ser Leu Thr Ser Glu Asp
                80                  85                  90

Thr Ala Val Tyr Tyr Cys Leu Ser Gln Ala Leu Lys Tyr Trp Gly
                95                 100                 105

Gln Gly Thr Leu Val Ala Val Ser Ser
               110
```

<130> 451541

<150> Australia PP9321
<151> 19 March 1999

<210> 55
<211> 113
<212> Amino Acid
<213> Human

<400> 55

```
Ala Ala Glu Leu Thr Gln Ser Pro Asp Ser Leu Ala Val Ser Leu
 1               5                  10                  15

Gly Glu Arg Ala Thr Ile Asn Cys Lys Ser Asn Gln Ser Val Leu
                20                  25                  30

Tyr Asn Ser Asn Ser Lys Asn Tyr Leu Ala Trp Tyr Gln Gln Lys
                35                  40                  45

Pro Gly Gln Pro Pro Lys Leu Leu Ile Tyr Trp Ala Ser Thr Arg
                50                  55                  60

Glu Ser Gly Val Pro Asp Arg Phe Ser Gly Ser Gly Ser Gly Thr
                65                  70                  75

Asp Phe Thr Leu Thr Ile Ser Ser Leu Gln Ala Glu Asp Val Ala
                80                  85                  90

Val Tyr Tyr Cys Gln Gln Tyr Phe Ser Thr Pro Tyr Thr Phe Gly
                95                 100                 105

Gln Gly Thr Lys Leu Glu Ile Lys
               110
```

<130> 451541

<150> Australia PP9321
<151> 19 March 1999

<210> 56
<211> 114
<212> Amino Acid
<213> Human

<400> 56

```
Val Gln Leu Leu Glu Gln Ser Gly Ala Glu Met Lys Arg Pro Gly
 1               5                  10                  15

Ala Ser Val Thr Ile Ser Cys Gln Ala Ser Arg Gln Thr Phe Ser
                20                  25                  30

Gly Gln Tyr Ile His Trp Val Arg Gln Ala Pro Gly Gln Gly Leu
                35                  40                  45

Glu Trp Met Gly Val Ile Asn Pro Ser Gly Gly Ser Ala Asn Tyr
                50                  55                  60

Ala Pro Ser Phe Gln Gly Arg Leu Ser Met Ser Arg Asp Ala Ser
                65                  70                  75

Thr Asn Thr Val Tyr Met Lys Leu Ser Ser Leu Thr Ser Glu Asp
                80                  85                  90

Thr Ala Val Tyr Tyr Cys Leu Ser Gln Ala Leu Lys Tyr Trp Gly
                95                 100                 105

Gln Gly Thr Leu Val Ala Val Ser Ser
               110
```

<130> 451541

<150> Australia PP9321
<151> 19 March 1999

<210> 57
<211> 113
<212> Amino Acid
<213> Human

<400> 57

```
Ala Ala Glu Leu Thr Gln Ser Pro Asp Ser Leu Ala Val Ser Leu
 1               5                  10                  15

Gly Glu Arg Ala Thr Ile Asn Cys Lys Ser Ser Gln Ser Val Leu
                20                  25                  30

Tyr Ser Ser Asn Asn Lys Asn Tyr Leu Ala Trp Tyr Gln Gln Lys
                35                  40                  45

Pro Gly Gln Pro Pro Lys Leu Leu Ile Tyr Trp Ala Ser Thr Arg
                50                  55                  60

Glu Ser Gly Val Pro Asp Arg Phe Ser Gly Ser Gly Ser Gly Thr
                65                  70                  75

Asp Phe Thr Leu Thr Ile Ser Ser Leu Gln Ala Glu Asp Val Ala
                80                  85                  90

Val Tyr Tyr Cys Gln Gln Tyr Tyr Ser Thr Pro Tyr Thr Phe Gly
                95                 100                 105

Gln Gly Thr Lys Leu Glu Ile Lys
               110
```

<130> 451541

<150> Australia PP9321
<151> 19 March 1999

<210> 58
<211> 114
<212> Amino Acid
<213> Human

<400> 58

```
Val Gln Leu Leu Glu Gln Ser Gly Ala Glu Val Lys Arg Pro Gly
 1               5                  10                  15

Ala Ser Val Thr Ile Ser Cys Gln Ala Ser Arg Gln Asn Phe Ser
                20                  25                  30

Gly Gln Tyr Ile His Trp Val Arg Gln Ala Pro Gly Gln Gly Leu
                35                  40                  45

Glu Trp Met Gly Ile Ile Asn Pro Ser Gly Gly Ser Ala Asn Tyr
                50                  55                  60

Ala Pro Arg Phe Lys Gly Arg Leu Ser Met Ser Arg Asp Ser Ser
                65                  70                  75

Thr Asp Thr Ala Tyr Leu Thr Leu Thr Ser Leu Thr Ser Glu Asp
                80                  85                  90

Thr Ala Val Tyr Phe Cys Leu Leu Gln Ser Leu Lys His Trp Gly
                95                 100                 105

Gln Gly Thr Leu Val Ala Val Ser Ser
               110
```

<130> 451541

<150> Australia PP9321
<151> 19 March 1999

<210> 59
<211> 97
<212> Amino Acid
<213> Human

<400> 59

```
Ala Ala Glu Leu Thr Gln Ser Pro Leu Ser Leu Pro Val Ile Pro
1               5                   10                  15

Gly Glu Pro Ala Ser Ile Ser Cys Arg Ser Ser Gln Ser Leu Leu
                20                  25                  30

His Arg Asn Gly Tyr Asn Tyr Leu Asp Trp Tyr Leu Gln Lys Pro
                35                  40                  45

Gly Gln Ser Pro Gln Leu Leu Ile Tyr Leu Gly Ser Thr Arg Ala
                50                  55                  60

Ser Gly Val Pro Asp Arg Phe Ser Gly Ser Gly Ser Gly Thr Asp
                65                  70                  75

Phe Thr Leu Asn Ile Arg Arg Val Glu Ala Glu Asp Val Gly Val
                65                  70                  75

Tyr Tyr Cys Met Gln Gly Leu Gln Thr Pro Tyr Thr Phe Gly Glu
                80                  85                  90

Gly Thr Lys Val Glu Ile Lys
                95
```

<130> 451541

<150> Australia PP9321
<151> 19 March 1999

<210> 60
<211> 118
<212> Amino Acid
<213> Human

<400> 60

```
Val Gln Leu Leu Glu Ser Gly Gly Gly Leu Ile Gln Pro Gly Arg
1               5                   10                  15

Ser Leu Arg Leu Ser Cys Thr Ala Ser Gly Phe Pro Phe Gly Asp
                20                  25                  30

Ser Ala Met Thr Trp Phe Arg Gln Ala Pro Gly Lys Gly Leu Glu
                35                  40                  45

Trp Val Gly Phe Ile Arg Ser Lys Ala Tyr Gly Ala Ala Thr Ala
                50                  55                  60

Tyr Ala Ala Ser Met Lys Gly Arg Val Thr Ile Ser Arg Asp Asp
                65                  70                  75

Ala Lys Ser Ile Ala Tyr Leu His Met Ser Arg Leu Lys Ile Glu
                80                  85                  90

Asp Thr Ala Val Tyr Phe Cys Ser Arg Val Lys Ala Gly Gly Pro
                95                  100                 105

Asp Tyr Trp Gly Gln Gly Thr Leu Val Thr Val Ser Ser
                110                 115
```